# EUROPÄISCHE PATENTSCHRIFT

(11) **EP 1 280 770 B1**
(45) Veröffentlichungstag und Bekanntmachung des Hinweises auf die Patenterteilung: **23.06.2010**
(21) Anmeldenummer: 01917102.4
(22) Anmeldetag: 21.03.2001
(51) Int. Cl.: C07D 207/38, C07D 209/54, C07D 491/10, A01N 43/36, A01N 43/38, A01N 43/90, C07C 233/51, C07D 309/14, C07C 233/52, A01N 43/12, C07D 307/94, C07C 69/757, C07D 309/38, A01N 43/16, C07C 49/733, A01N 35/06

(54) **C2-PHENYLSUBSTITUIERTE CYCLISCHE KETOENOLE ALS SCHÄDLINGSBEKÄMPFUNGSMITTEL UND HERBIZIDE**
C 2? PHENYL-SUBSTITUTED CYCLIC KETO-ENOLS USED AS PESTICIDES AND HERBICIDES
CETOENOLS CYCLIQUES SUBSTITUES PAR PHENYLE C 2? UTILISES COMME AGENTS DE LUTTE CONTRE LES PARASITES ET COMME HERBICIDES

(30) Priorität: 03.04.2000 DE 10016544
(43) Veröffentlichungstag der Anmeldung: 05.02.2003
(73) Patentinhaber: Bayer Aktiengesellschaft, 51368 Leverkusen (DE)
(72) Erfinder: RUTHER, Michael, 40764 Langenfeld (DE); HAGEMANN, Hermann, 51375 Leverkusen (DE); SCHNEIDER, Udo, 51373 Leverkusen (DE); DOLLINGER, Markus, 51381 Leverkusen (DE); DAHMEN, Peter, 41470 Neuss (DE); WACHENDORFF-NEUMANN, Ulrike, 56566 Neuwied (DE); FISCHER, Reiner, 40789 Monheim (DE); GRAFF, Alan, 51375 Leverkusen (DE); BRETSCHNEIDER, Thomas, 53797 Lohmar (DE); ERDELEN, Christoph, 42799 Leichlingen (DE); DREWES, Mark, Wilhelm, 40764 Langenfeld (DE); FEUCHT, Dieter, 40789 Monheim (DE); LIEB, Folker, 51375 Leverkusen (DE)
(86) Internationale Anmeldenummer: PCT/EP2001/003215
(87) Internationale Veröffentlichungsnummer: WO 2001/074770

(56) Entgegenhaltungen:
- EP-A- 0 355 599
- WO-A-96/25395
- DE-A- 2 813 341
- US-A- 4 256 659
- US-A- 4 351 666
- US-A- 4 409 153
- US-A- 4 613 617

## Beschreibung

Die vorliegende Erfindung betrifft neue C₂-phenylsubstituierte cyclische Ketoenole, mehrere Verfahren zu ihrer Herstellung und ihre Verwendung als Schädlingsbekämpfungsmittel und Herbizide.

Von 3-Acyl-pyrrolidin-2,4-dionen sind phannazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599 und EP-A-415 211) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 94/01 997, WO 95/26 954, WO 95120 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/16748, WO 99/24437, WO 99/43649, WO 99/48869 und WO 99/55673).

Es ist bekannt, daß bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-di-hydrofuranon-Derivate mit herbiziden, akariziden und insektiziden Eigenschaften aus EP-A-528 156, EP-A-0 647 637, WO 95/26 345, WO 96/20 196, WO 96/25 395, WO 96/35 664, WO 97/01 535, WO 97/02 243, WO 97/36 868, WO 98/05638, WO 98/25928, WO 99/16748, WO 99/43649, WO 99/48869 und WO 99/55673 bekannt. Auch 3-Aryl-Δ³-dihydrothiphen-on-Derivate sind bekannt (WO 95/26 345, 96/25 395, WO 97/01 535, WO 97/02 243, WO 97/36 868, WO 98/05638, WO 98/25928, WO 99/16748, WO 99/43649, WO 99/48869, WO 99/55673).

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-A-588 137, WO 96/25 395, WO 96/35 664, WO 97/01 535, WO 97/02 243, WO 97/16 436, WO 97/19 941, WO 97/36 868, WO 98/05638, WO 99/43649, WO 99/48869 und WO 99/55673 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785, WO 96/02 539, WO 96/35 664, WO 97/01 535, WO 97/02 243, WO 97/02 243, WO 97/36 868, WO 99/05638, WO 99/43649, WO 99/48869 und WO 99/55673 beschrieben.

Es ist bekannt, daß bestimmte substituierte 2-Arylcyclopentandione herbizide und akarizide Eigenschaften besitzen (vgl. z.B. US-4 283 348; 4 338 122; 4 436 666; 4 526 723; 4 551 547; 4 632 698; WO 96/01 798; WO 96/03 366, WO 97/14 667 sowie WO 98/39281, WO 99/43649, WO 99/48869, WO 99/55673). Außerdem sind ähnlich substituierte Verbindungen bekannt; 3-Hydroxy-5,5-dimethyl-2-phenylcyclopent-2-en-1-on aus der Publikation Micklefield et al., Tetrahedron, (1992), 7519-26 sowie der Naturstoff Involutin (-)-cis-5-(3,4-dihydroxyphenyl)-3,4-dihydroxy-2-(4-hydroxyphenyl)-cyclopent-2-en-one aus der Publikation Edwards et al., J. Chem. Soc. S, (1967), 405-9. Eine insektizide oder akarizide Wirkung wird nicht beschrieben. Außerdem ist 2-(2,4,6-Trimethylphenyl)-1,3-indandion aus der Publikation J. Economic Entomology, 66, (1973), 584 und der Offenlegungsschrift DE-A 2 361 084 bekannt, mit Angabe von herbiziden und akariziden Wirkungen.

Es ist bekannt, daß bestimmte substituierte 2-Arylcyclohexandione herbizide und akarizide Eigenschaften besitzen (US-4 175 135, 4 209 432, 4 256 657, 4 256 658, 4 256 659, 4 257 858, 4 283 348, 4 303 669, 4 351 666, 4 409 153, 4 436 666, 4 526 723, 4 613 617, 4 659 372, DE-A 2 813 341, sowie Wheeler, T.N., J. Org. Chem. 44, 4906 (1979)), WO 99/43649, WO 99/48869, WO 99/55673).

Die Wirksamkeit und Wirkungsbreite dieser Verbindungen ist jedoch insbesondere bei niedrigen Aufwandmengen und Konzentrationen nicht immer voll zufriedenstellend. Weiterhin ist die Pflanzenverträglichkeit dieser Verbindungen nicht immer ausreichend.

Es wurden nun neue Verbindungen der Formel (I) gefunden 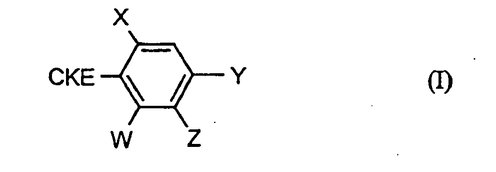 in welcher
- W: für Wasserstoff, Methyl, Ethyl, i-Propyl, Vinyl, oder Ethinyl steht,
- X: für Methyl, Ethyl, n-Propyl, iso-Propyl, Vinyl, oder Ethinyl steht,
- Y: für Wasserstoff, Methyl, Ethyl, i-Propyl, Vinyl oder Ethinyl steht,
- Z: für Wasserstoff, Methyl, Ethyl, n-Propyl, i-Butyl, Vinyl oder Ethinyl steht,
mit der Maßgabe, dass mindestens einer der Reste W, X, Y oder Z für eine Kette mit mindestens zwei Kohlenstoffatomen steht, wobei maximal nur einer der Reste W, X, Y oder Z für Vinyl oder Ethinyl stehen darf,
CKE für eine der Gruppen 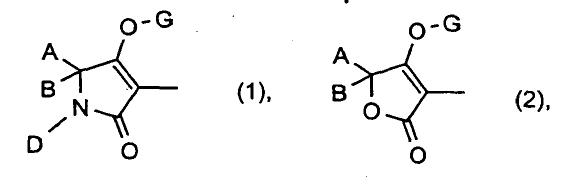 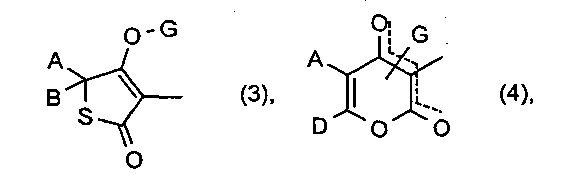 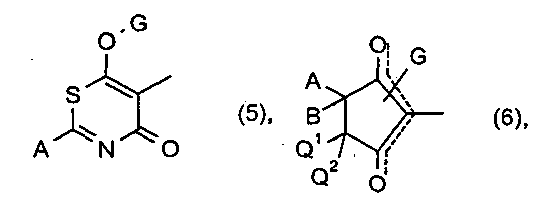 steht,
- A: für jeweils gegebenenfalls durch Fluor substituiertes C₁-C₄-Alkyl oder C₁-C₂-Alkoxy-C₁-C₂-alkyl, Cyclopropyl, Cyclopentyl oder Cyclohexyl und nur im Fall der Verbindungen der Formel (I-5) für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl steht,
- B: für Wasserstoff, Methyl oder Ethyl steht oder
- A, B: und das Kohlenstoffatom an das sie gebunden sind, für gesättigtes C₅-C₆-Cycloalkyl, in welchem gegebenenfalls ein Ringglied durch Sauerstoff oder Schwefel ersetzt ist und welches gegebenenfalls einfach durch Methyl, Ethyl, Propyl, Isopropyl, Trifluormethyl, Methoxy, Ethoxy, Propoxy oder Butoxy substituiert ist oder
- A, B: und das Kohlenstoffatom, an das sie gebunden sind, für C₆-Cycloalkyl, welches durch mit zwei nicht direkt benachbarten Sauerstoffatomen enthaltende Alkylendioxyl-Gruppe substituiert ist,
- A, B: und das Kohlenstoffatom, an das sie gebunden sind, für C₅-C₆-Cycloalkyl oder C₅-C₆-Cycloalkenyl, worin zwei Substituenten gemeinsam mit den Kohlenstoffatomen, an die sie gebunden sind, für C₂-C₄-Alkandiyl oder C₂-C₄-Alkendiyl oder Butadiendiyl stehen,
- D: für Wasserstoff, für jeweils gegebenenfalls durch Fluor substituiertes C₁-C₄-Allcyl, C₃-C₄-Alkenyl, C₁-C₄-Alkoxy-C₂-C₃-alkyl, Cyclopropyl, Cyclopentyl oder Cyclohexyl oder (jedoch nicht im Fall der Verbindungen der Formeln (I-1)) für gegebenenfalls einfach durch Fluor, Chlor, Methyl, Ethyl, n-Propyl, iso-Propyl, Methoxy, Ethoxy oder Tri-fluormethyl substituiertes Phenyl oder Pyridyl, steht,
- A und Q¹: gemeinsam für gegebenenfalls einfach oder zweifach durch Methyl oder Methoxy substituiertes C₃-C₄-Alkandiyl stehen oder
- Q¹: für Wasserstoff steht,
- Q²: für Wasserstoff steht,
- G: für Wasserstoff (a) oder für eine der Gruppen 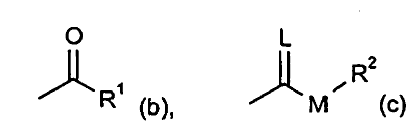 steht,
in welchen
L für Sauerstoff oder Schwefel steht und
M für Sauerstoff oder Schwefel steht.
- R¹: für C₁-C₆-Alkyl, C₂-C₆-Alkenyl, C₁-C₂-Alkoxy-C₁-alkyl, C₁-C₂-Alkylthio-C₁-alkyl oder gegebenenfalls einfach durch Fluor, Chlor, Methyl oder Methoxy substituiertes Cyclopropyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl steht,
- R²: für jeweils gegebenenfalls durch Fluor substituiertes C₁-C₈-Alkyl, C₂-C₆-Alkenyl oder C₁-C₄-Alkoxy-C₂-C₃-alkyl, Phenyl oder Benzyl steht.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1) bis (6) der Gruppe CKE ergeben sich folgende hauptsächliche Strukturen (I-1) bis (I-6): 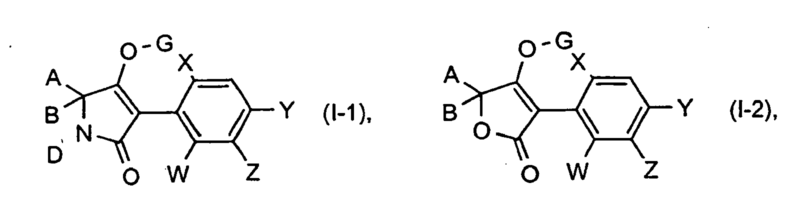 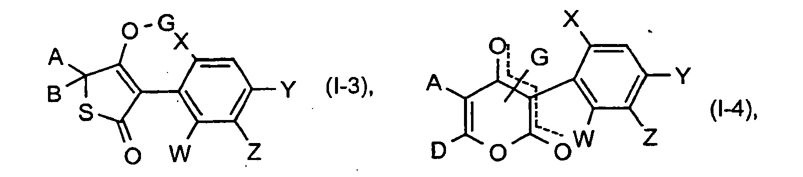 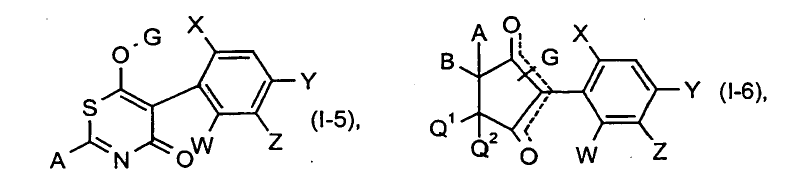 worin
A, B, D, G, Q¹, Q², W, X, Y und Z die oben angegebene Bedeutung haben.

Unter Einbeziehung der verschiedenen Bedeutungen (a), (b), (c), der Gruppe G ergeben sich folgende hauptsächliche Strukturen (I-1-a) bis (I-1-c), wenn CKE für die Gruppe (1) steht, 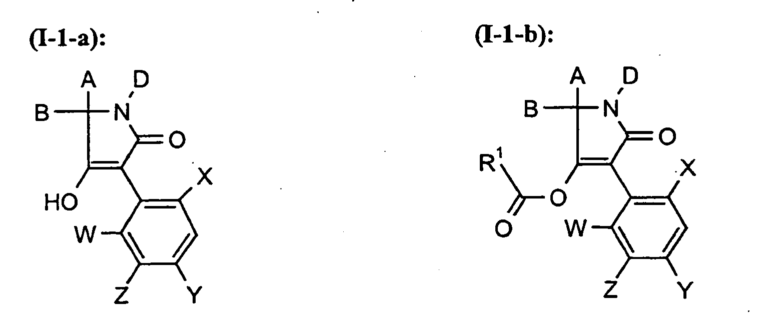 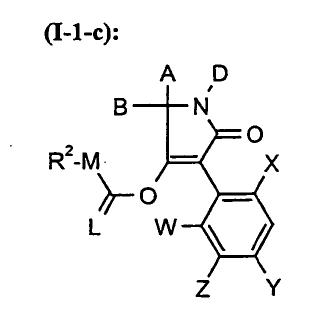 worin
A, B, D, L, M, W, X, Y, Z, R¹, R², die- oben angegebenen Bedeutungen besitzen.

Unter Einbeziehung der verschiedenen Bedeutungen (a), (b), (c), der Gruppe G ergeben sich folgende hauptsächliche Strukturen (I-2-a) bis (I-2-c), wenn CKE für die Gruppe (2) steht, 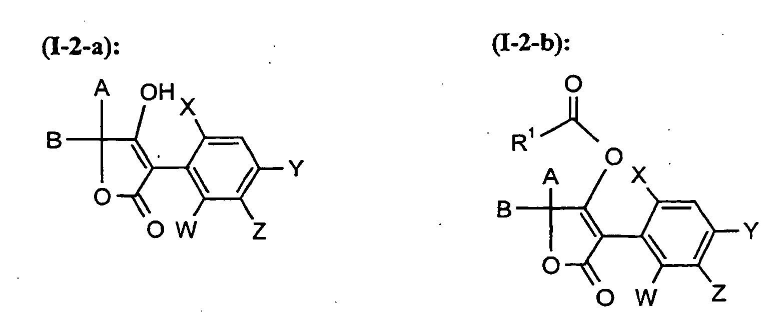 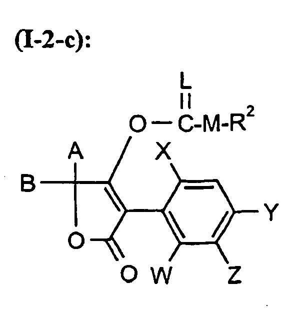 worin
A, B, L, M, W, X, Y, Z, R¹, R², die oben angegebene Bedeutung haben.

Unter Einbeziehung der verschiedenen Bedeutungen (a), (b), (c), der Gruppe G ergeben sich folgende hauptsächliche Strukturen (I-3-a) bis (I-3-c), wenn CKE für die Gruppe (3) steht, 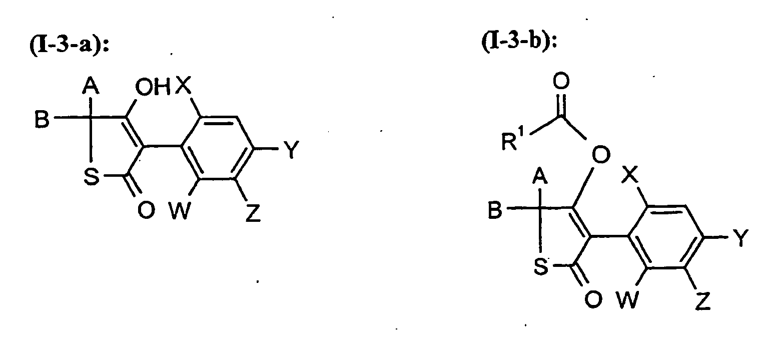 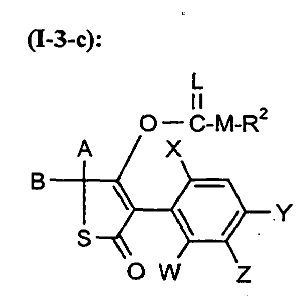 worin
A, B, L, M, W, X, Y, Z, R¹, R², die oben angegebenen Bedeutung besitzen.

Die Verbindungen der Formel (I-4) können in Abhängigkeit von der Stellung des Substituenten G in den zwei isomeren Formen der Formeln (I-4-A) und (I-4-B) vorliegen, 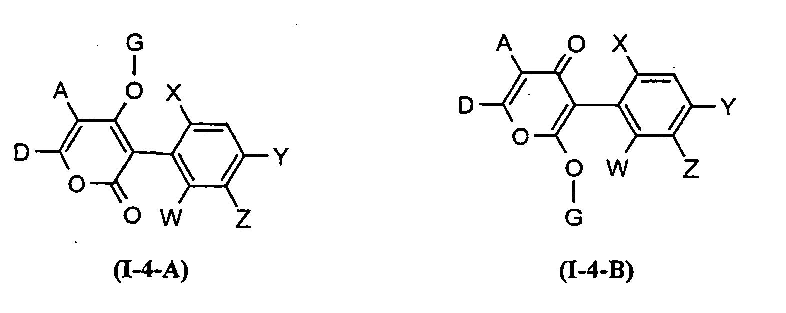 was durch die gestrichelte Linie in der Formel (I-4) zum Ausdruck gebracht werden soll.

Die Verbindungen der Formeln (I-4-A) und (I-4-B) können sowohl als Gemische als auch in Form ihrer reinen Isomeren vorliegen. Gemische der Verbindungen der Formeln (I-4-A) und (I-4-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, daß die Verbindungen gegebenenfalls in Form der Isomerengemische oder in der jeweils anderen isomeren Form vorliegen können.

Unter Einbeziehung der verschiedenen Bedeutungen (a), (b), (c), der Gruppe G ergeben sich folgende hauptsächliche Strukturen (I-4-a) bis (I-4-c), wenn CKE für die Gruppe (4) steht, 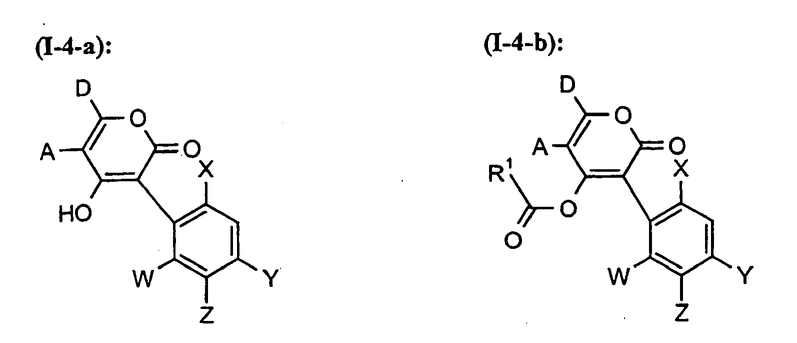 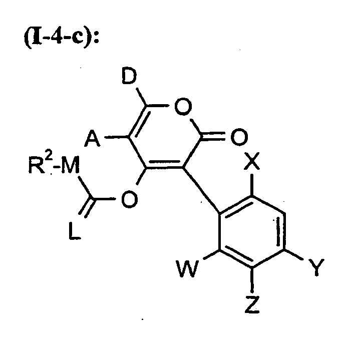 worin
A, D, M, W, X, Y, Z, R¹, R², die oben angegebenen Bedeutungen besitzen.

Unter Einbeziehung der verschiedenen Bedeutungen (a), (b), (c), der Gruppe G ergeben sich folgende hauptsächliche Strukturen (I-5-a) bis (I-5-c), wenn CKE für die Gruppe (5) steht, 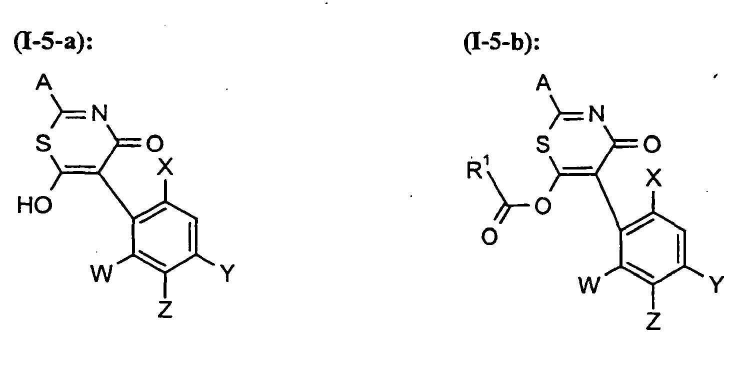 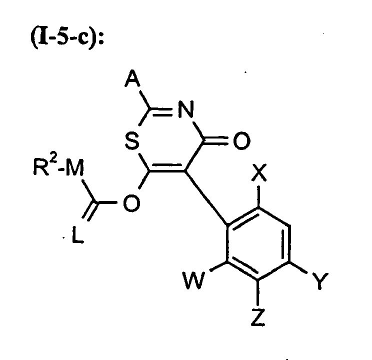 worin
A, L, M, W, X, Y, Z, R¹, R², die oben angegebenen Bedeutungen besitzen.

Die Verbindungen der Formel (I-6) können in Abhängigkeit von der Stellung des Substituenten G in den zwei isomeren Formen der Formeln (I-6-A) und (I-6-B) vorliegen, 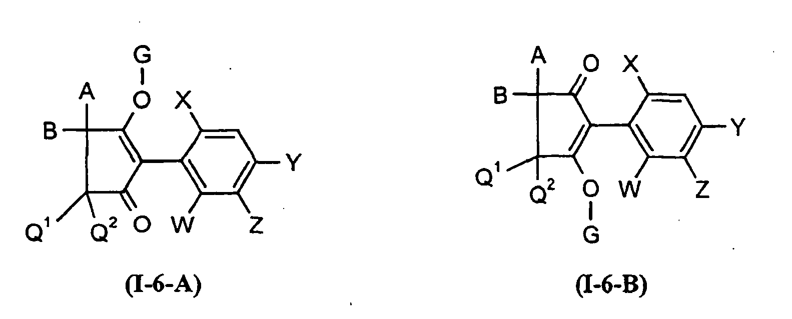 was durch die gestrichelte Linie in der Formel (I) zum Ausdruck gebracht werden soll.

Die Verbindungen der Formeln (I-6-A) und (I-6-B) können sowohl als Gemische als auch in Form ihrer reinen Isomeren vorliegen. Gemische der Verbindungen der Formeln (I-6-A) und (I-6-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, daß die Verbindungen gegebenenfalls in Form der Isomerengemische oder in der jeweils anderen isomeren Form vorliegen können.

Unter Einbeziehung der verschiedenen Bedeutungen (a), (b), (c), der Gruppe G ergeben sich folgende hauptsächlichen Strukturen (I-6-a) bis (I-6-c): 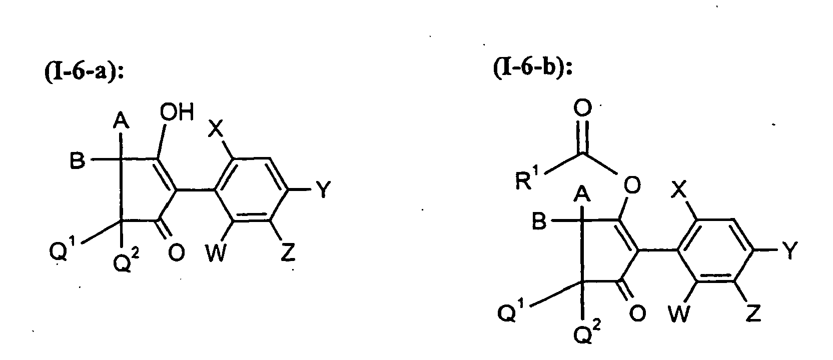 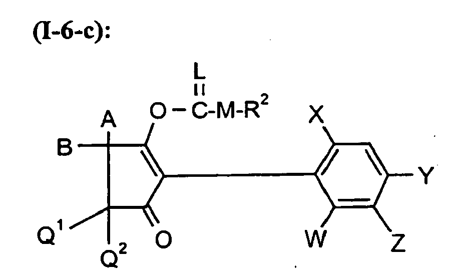 worin
A, B, Q¹, Q², L, M, W, X, Y, Z, R¹, R², die oben angegebenen Bedeutungen besitzen.

Weiterhin wurde gefunden, daß man die neuen Verbindungen der Formel (I) nach einem der im folgenden beschriebenen Verfahren erhält:
(A) Man erhält substituierte 3-Phenylpyrrolidin-2,4-dione bzw. deren Enole der Formel (I-1-a) 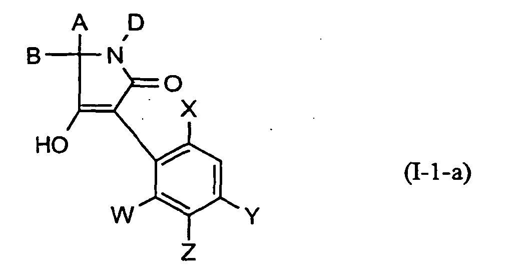 in welcher
   A, B, D, W, X, Y und Z die oben angegebenen Bedeutungen haben,
   wenn man
   N-Acylaminosäureester der Formel (II) 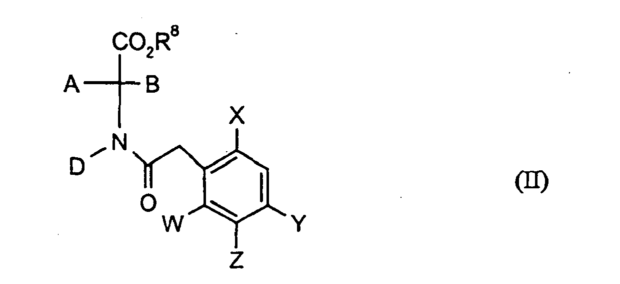 in welcher
   - A, B, D, W, X, Y und Z: die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, daß man substituierte 3-Phenyl-4-hydroxy-Δ³-di-hydrofuranon-Derivate der Formel (I-2-a) 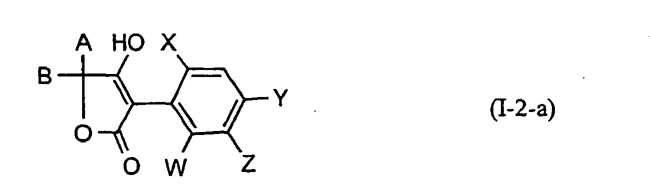 in welcher
   A, B, W, X, Y und Z die oben angegebenen Bedeutungen haben,
   erhält, wenn man
   Carbonsäureester der Formel (III) 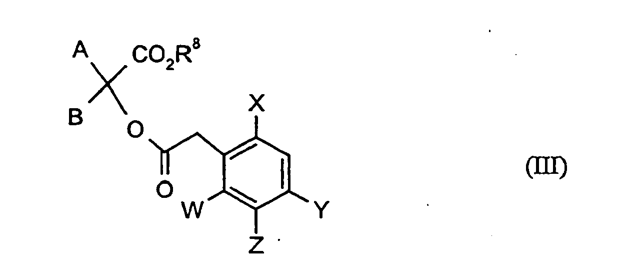 in welcher
   A, B, W, X, Y, Z und R⁸ die oben angegebenen Bedeutungen haben,
   in Gegenwart eines Verdünnungsmittels und in Gegenwart einer Base intramolekular kondensiert.
(C) Weiterhin wurde gefunden, daß man substituierte 3-Phenyl-4-hydroxy-Δ³-di-hydrothiophenon-Derivate der Formel (I-3-a) 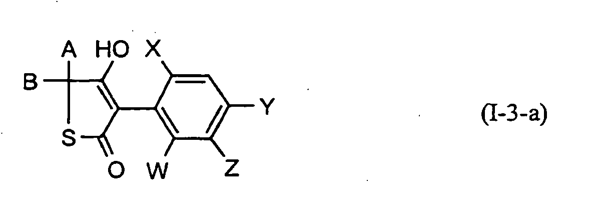 in welcher
   A, B, W, X, Y und Z die oben angegebenen Bedeutungen haben,
   erhält, wenn man
   ß-Ketocarbonsäureester der Formel (IV) 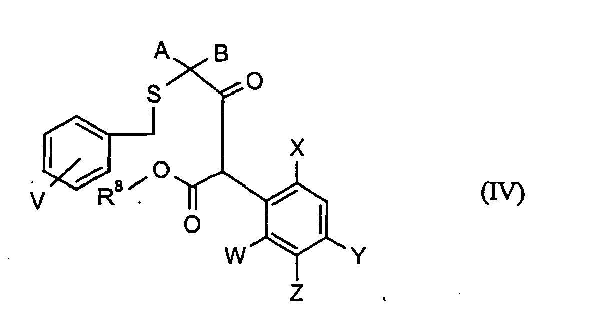 in welcher
   - A, B, W, X, Y, Z und R⁸: die oben angegebenen Bedeutungen haben und
   - V: für Wasserstoff, Halogen, Alkyl (bevorzugt C₁-C₆-Alkyl) oder Alkoxy (bevorzugt C₁-C₈-Alkoxy) steht,
   gegebenenfalls in Gegenwart eines Verdünnungsmittels und in Gegenwart einer Säure intramolekular cyclisiert.
(D) Weiterhin wurde gefunden, daß man die neuen substituierten 3-Phenylpyron-Derivate der Formel (I-4-a) 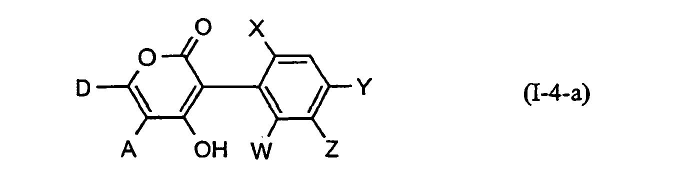 in welcher
   A, D, W, X, Y und Z die oben angegebenen Bedeutungen haben,
   erhält, wenn man
   Carbonylverbindungen der Formel (V) 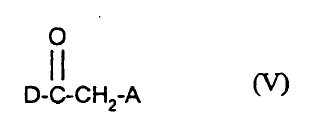 in welcher
   - A und D: die oben angegebenen Bedeutungen haben,
   oder deren Silylenolether der Formel (Va) 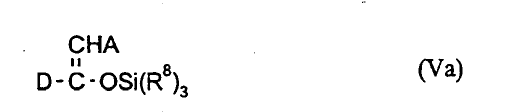 in welcher
   A, D und R⁸ die oben angegebene Bedeutung haben,
   mit Ketensäurehalogeniden der Formel (VI) 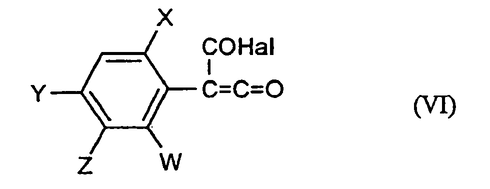 in welcher
   W, X, Y und Z die oben angegebenen Bedeutungen haben und
   Hal für Halogen (vorzugsweise für Chlor oder Brom) steht,
   gegebenenfalls in Gegenwart eines Verdünnungsmittels und gegebenenfalls in Gegenwart eines Säureakzeptors umsetzt.
   Weiterhin wurde gefunden,
(E) dass man die neuen substituierten Phenyl-1,3-thiazin-Derivate der Formel (I-5-a) 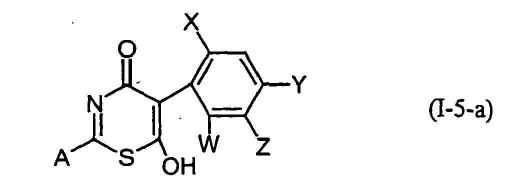 in welcher
   A, W, X, Y und Z die oben angegebene Bedeutung haben,
   erhält, wenn man Thioamide der Formel (VII) 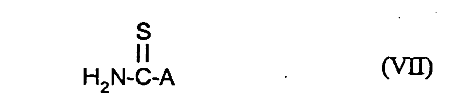 in welcher
   - A: die oben angegebene Bedeutung hat,
   mit Ketensäurehalogeniden der Formel (VI) 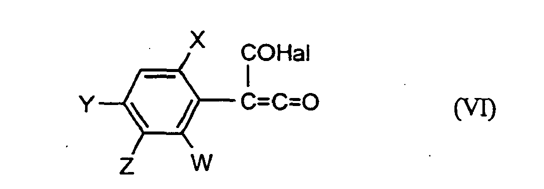 in welcher
   Hal, W, X, Y und Z die oben angegebenen Bedeutungen haben,
   gegebenenfalls in Gegenwart eines Verdünnungsmittels und gegebenenfalls in Gegenwart eines Säureakzeptors umsetzt.
   Weiterhin wurde gefunden,
(F) daß man Verbindungen der Formel (I-6-a) 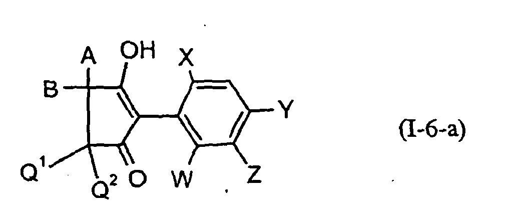 in welcher
   A, B,Q¹, Q², W, X, Y und Z die oben angegebene Bedeutung haben,
   erhält, wenn man
   Ketocarbonsäureester der Formel (VIII) 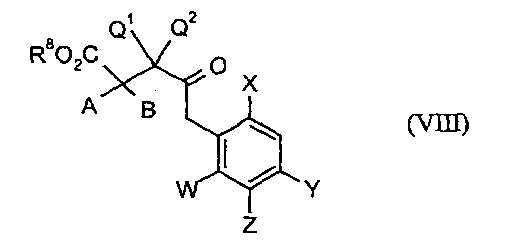 in welcher
   - A, B, Q¹, Q²,: W, X, Y und Z die oben angegebene Bedeutung haben, und
   - R⁸: für Alkyl (insbesondere C₁-C₈-Alkyl) steht,
   gegebenenfalls in Gegenwart eines Verdünnungsmittels und in Gegenwart einer Base intramolekular cyclisiert.
(H) daß man Verbindungen der oben gezeigten Formeln (I-1(a-c)) bis (I-6(a-c)), in welchen A, B, D, G, Q¹, Q², W, X, Y und Z die oben angegebene Bedeutung haben,
   wobei ein, maximal zwei Reste W, X, Y oder Z für R²²-C≡C- oder 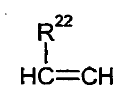 stehen, R²² steht für Wasserstoff oder C₁-C₄-Alkyl, bevorzugt für Wasserstoff oder C₁-C₂-Alkyl und besonders bevorzugt für Wasserstoff,
   erhält, wenn man Verbindungen der Formel (I-1'(a-c)) bis (I-6'(a-c)), 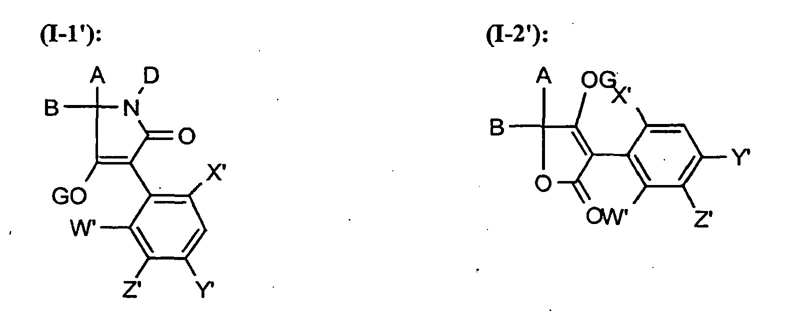 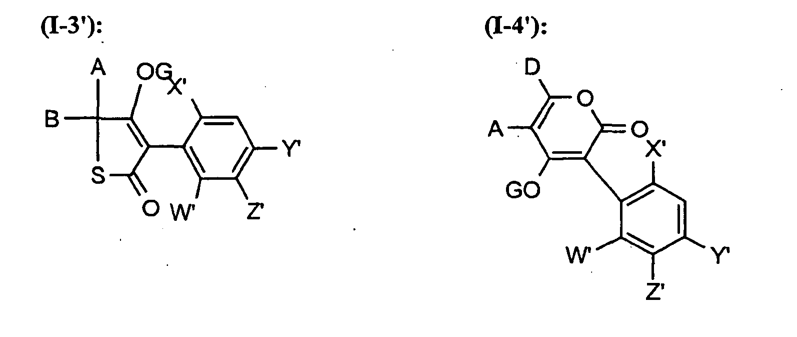 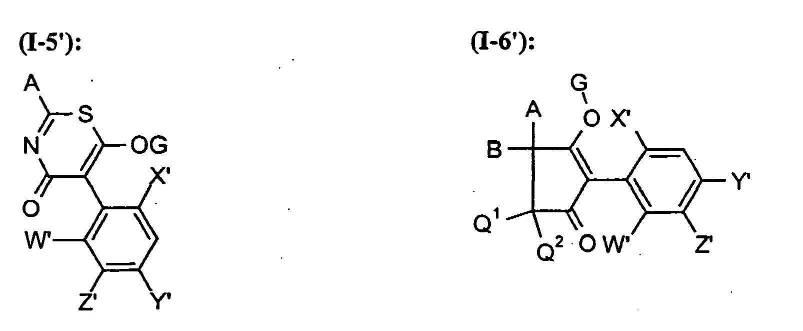 in welchen
   A, B, D, G, Q¹, Q², W, X', Y' und Z' die oben angegebene Bedeutung haben und wobei der Hochstrich ' bedeutet, dass ein, maximal zwei Reste W, X, Y und Z bei diesem Verfahren für Chlor, Brom, Jod, bevorzugt für Brom stehen, mit der Maßgabe, dass die anderen Reste W, X, Y oder Z nicht für Alkenyl oder Alkinyl stehen
   mit Silylacetylenen der Formel (X-a) oder Vinylstananen der Formel (X-b) 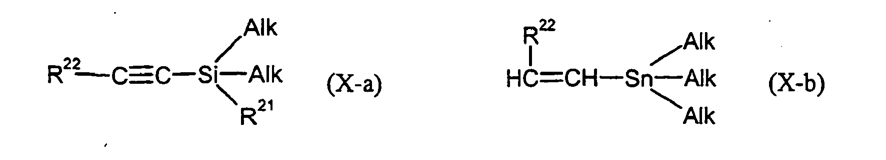 in welchen Alk bevorzugt für C₁-C₄-Alkyl und
   - R²¹: bevorzugt für C₁-C₄-Alkyl oder Phenyl steht,
   - R²²: bevorzugt für Wasserstoff oder C₁-C₄-Alkyl, besonders bevorzugt für Wasserstoff oder C₁-C₂-Alkyl und ganz besonders bevorzugt für Wasserstoff steht,
   in Gegenwart eines Lösungsmittels, gegebenenfalls in Gegenwart einer Base und eines Katalysators umsetzt, wobei als Katalysator insbesondere Palladiumkomplexe in Frage kommen.
   Außerdem wurde gefunden
(I) daß man die Verbindungen der oben gezeigten Formeln (I-1-b) bis (I-6-b), in welchen A, B, D, Q¹, Q², R¹, W, X, Y und Z die oben angebenen Bedeutungen haben, erhält, wenn man Verbindungen der oben gezeigten Formeln (I-1-a) bis (I-6-a), in welchen A, B, D, Q¹, Q², W, X, Y und Z die oben angegebenen Bedeutungen haben, jeweils
   (α) mit Säurehalogeniden der Formel (XI) 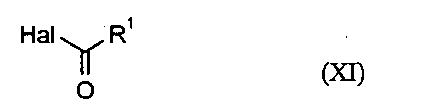 in welcher
      - R¹: die oben angegebene Bedeutung hat und
      - Hal: für Halogen (insbesondere Chlor oder Brom) steht
      oder
   (β) mit Carbonsäureanhydriden der Formel (XII)

      R¹-CO-O-CO-R¹ (XII)
   in welcher
   - R¹: die oben angegebene Bedeutung hat,
   gegebenenfalls in Gegenwart eines Verdünnungsmittels und gegebenenfalls in Gegenwart eines Säurebindemittels umsetzt;
(J) daß man die Verbindungen der oben gezeigten Formeln (I-1-c) bis (I-6-c), in welchen A, B, D, Q¹, Q², R², M, W, X, Y und Z die oben angegebenen Bedeutungen haben und L für Sauerstoff steht, erhält, wenn man Verbindungen der oben gezeigten Formeln (I-1-a) bis (I-6-a), in welchen A, B, D, Q¹, Q², W, X, Y und Z die oben angegebenen Bedeutungen haben, jeweils
   mit Chlorameisensäureestern oder Chlorameisensäurethioestem der Formel (XIII)

   R²-M-CO-Cl (XIII)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(K) daß man Verbindungen der oben gezeigten Formeln (I-1-c) bis (I-6-c), in welchen A, B, D, Q¹, Q², R², M, W, X, Y und Z die oben angegebenen Bedeutungen haben und L für Schwefel steht, erhält, wenn man Verbindungen der oben gezeigten Formeln (I-1-a) bis (I-6-a), in welchen A, B, D, Q¹, Q², W, X, Y und Z die oben angegebenen Bedeutungen haben, jeweils
   mit Chlormonothioameisensäureestern oder Chlordithioameisensäureestern der Formel (XIV) 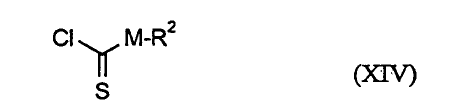 in welcher
   - M und R²: die oben angegebenen Bedeutungen haben,
   gegebenenfalls in Gegenwart eines Verdünnungsmittels und gegebenenfalls in Gegenwart eines Säurebindemittels umsetzt.

Weiterhin wurde gefunden, daß die neuen Verbindungen der Formel (I) eine sehr gute Wirksamkeit als Schädlingsbekämpfungsmittel, vorzugsweise als Insektizide, Akarizide und Herbizide aufweisen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- W: steht insbesondere bevorzugt für Wasserstoff, Methyl, Ethyl oder i-Propyl,
- X: steht insbesondere bevorzugt für Methyl, Ethyl, i-Propyl oder Vinyl,
- Y: steht insbesondere bevorzugt für Wasserstoff, Methyl, Ethyl, i-Propyl, Vinyl oder Ethinyl,
- Z: steht insbesondere bevorzugt für Wasserstoff, Methyl, Ethyl, n-Propyl oder i-Butyl,
mit der Maßgabe, dass mindestens einer der Reste W, X, Y oder Z für eine Kette mit mindestens zwei Kohlenstoffatomen steht, wobei maximal nur einer der Reste W, X, Y oder Z für Vinyl oder Ethinyl stehen darf,
CKE steht insbesondere bevorzugt für eine der Gruppen 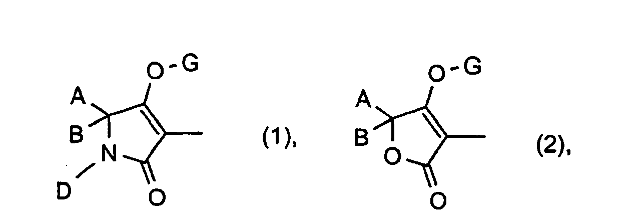 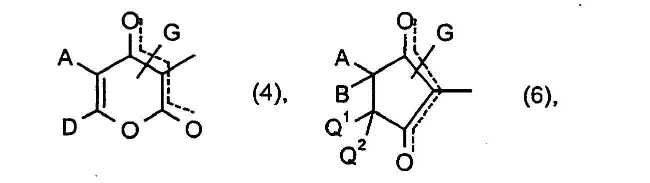
- A: steht insbesondere bevorzugt für Methyl,
- B: steht insbesondere bevorzugt für Methyl,
- A, B: und das Kohlenstoffatom, an das sie gebunden sind, stehen insbesondere bevorzugt für gesättigtes C₅-C₆-Cycloalkyl, in welchem gegebenenfalls ein Ringglied durch Sauerstoff ersetzt ist und welches gegebenenfalls einfach durch Methyl, Ethyl, Methoxy, Ethoxy substituiert ist.
- A und B: stehen insbesondere gemeinsam für 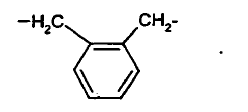
- D: steht insbesondere bevorzugt für Wasserstoff oder (jedoch nicht im Fall der Verbindung der Formel (I-1)) für durch Fluor substituiertes Phenyl,
- G: steht insbesondere bevorzugt für Wasserstoff (a) oder für eine der Gruppen 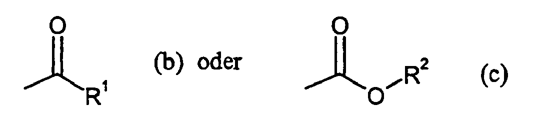
- R¹: steht insbesondere bevorzugt für C₁-C₆-Alkyl, C₁-C₂-Alkoxymethyl,
- R²: steht insbesondere bevorzugt für C₁-C₄-Alkyl.

Für CKE = (6) stehen
- A und Q¹: gemeinsam insbesondere bevorzugt für C₃-C₄-Alkyandiyl und
- B und Q²: jeweils insbesondere bevorzugt für Wasserstoff.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß insbesondere bevorzugt werden die Verbindungen der Formel (I), in welchen eine Kombination der vorstehend als insbesondere bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

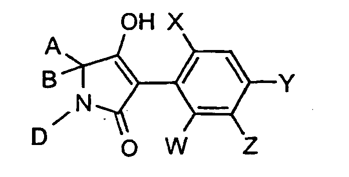

**Tabelle 1: W = CH₃, X = CH₃, Y = C≡CH, Z = H.**

| A | B | D |
|---|---|---|
| CH₃ | H | H |
| C₂H₅ | H | H |
| C₃H₇ | H | H |
| i-C₃H₇ | H | H |
| C₄H₉ | H | H |
| i-C₄H₉ | H | H |
| s-C₄H₉ | H | H |
| t-C₄H₉ | H | H |
| CH₃ | CH₃ | H |
| C₂H₅ | CH₃ | H |
| C₃H₇ | CH₃ | H |
| i-C₃H₇ | CH₃ | H |
| C₄H₉ | CH₃ | H |
| i-C₄H₉ | CH₃ | H |
| s-C₄H₉ | CH₃ | H |
| t-C₄H₉ | CH₃ | H |
| C₂H₅ | C₂H₅ | H |
| C₃H₇ | C₃H₇ | H |
| 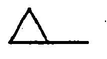 | CH₃ | H |
| 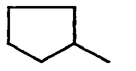 | CH₃ | H |
| 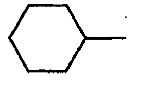 | CH₃ | H |
| -(CH₂)₂- | | H |
| -(C₂)₄- | | H |
| -(CH2)5- | | H |
| -(CH₂)₆- | | H |
| -(CH2)7- | | H |
| -(CH₂)₂-O-(CH₂)₂- | | H |
| -CH₂-O-(CH₂)₃- | | H |
| -(CH₂)₂-S-(CH₂)₂- | | H |
| -CH₂-CHCH₃-(CH₂)₃- | | H |
| -(CH₂)₂-CHCH₃-(CH₂)₂- | | H |
| -(CH₂)₂-CHC₂H₅-(CH₂)₂- | | H |
| -(CH₂)₂-CHC₃H₇-(CH₂)₂- | | H |
| -(CH₂)₂-CHi-C₃H₇-(CH₂)₂- | | H |
| -(CH₂)₂-CHOCH₃-(CH₂)₂- | | H |
| -(CH₂)₂-CHOC₂H₅-(CH₂)₂- | | H |
| -(CH₂)₂-CHOC₃H₇-(CH₂)₂- | | H |
| -(CH₂)₂-CHi-C₃H₇-(CH₂)₂- | | H |
| -(CH₂)₂-C(CH₃)₂-(CH₂)₂- | | H |
| -CH₂-(CHCH₃)₂-(CH₂)₂- | | H |
| 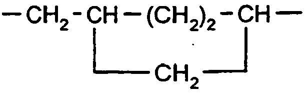 | | H |
| 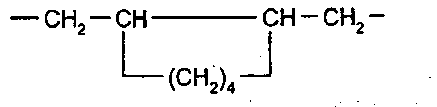 | | H |
| 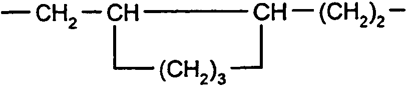 | | H |
| 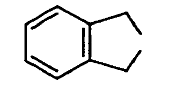 | | H |
| 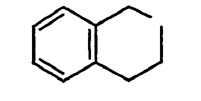 | | H |
| -(CH2)3- | | H |
| -(CH2)4- | | H |
| -CH₂-CHCH₃-CH₂- | | H |
| -CH₂-CH₂-CHCH₃- | | H |
| -CH₂-CHCH₃-CHCH₃- | | H |
| -CH₂-S-CH₂- | | H |
| -CH₂-S-(CH₂)₂- | | H |
| -(CH₂)₂-S-CH₂- | | H |
| 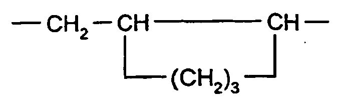 | | H |
| H | CH₃ | H |
| H | C₂H₅ | H |
| H | C₃H₇ | H |
| H | i-C₃H₇ | H |
| H | 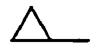 | H |
| H | 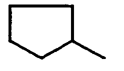 | H |
| H | 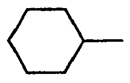 | H |
| CH₃ | CH₃ | H |
| CH₃ | C₂H₅ | H |
| CH₃ | C₃H₇ | H |
| CH₃ | i-C₃H₇ | H |
| CH₃ | 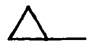 | H |
| CH₃ | 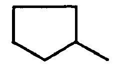 | H |
| CH₃ | 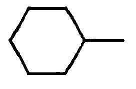 | H |
| C₂H₅ | CH₃ | H |
| C₂H₅ | C₂H₅ | H |

**Tabelle 2:** A, B und D wie in Tabelle 1 angegeben
   W = CH₃; X = CH₃; Y = CH=CH₂; Z = H.
**Tabelle 3:** A, B und D wie in Tabelle 1 angegeben
   W = CH₃; X = C₂H₅; Y = CH₃; Z = H.
**Tabelle 4:** A, B und D wie in Tabelle 1 angegeben
   W = CH₃; X = CH₃; Y = C₂H₅; Z=H.
**Tabelle 5:** A, B und D wie in Tabelle 1 angegeben
   W = C₂H₅; X = C₂H₅; Y = CH₃; Z = H.
**Tabelle 6:** A, B und D wie in Tabelle 1 angegeben
   W = C₂H₅; X = C₂Hₛ; Y = C₂H₅; Z = H.
**Tabelle 7:** A, B und D wie in Tabelle 1 angegeben
   W = CH₃; X = C≡CH; Y = CH₃; Z = H.
**Tabelle 8:** A, B und D wie in Tabelle 1 angegeben
   W = CH₃; X = CH=CH₂; Y = CH₃; Z = H.
**Tabelle 9:** A, B und D wie in Tabelle 1 angegeben
   W = CH₃; X = C₂H₅; Y = C₂H₅; Z = H.
**Tabelle 10:** A, B und D wie in Tabelle 1 angegeben
   W=H; X=CH₃; Y=C≡CH; Z=H.
**Tabelle 11:** A, B und D wie in Tabelle 1 angegeben
   W = H; X = CH₃; Y = CH=CH₂; Z = H.
**Tabelle 12:** A, B und D wie in Tabelle 1 angegeben
   W = H; X = C₂H₅; Y = CH₃; Z = H.
**Tabelle 13:** A, B und D wie in Tabelle 1 angegeben
   W = H; X = CH₃; Y = C₂H₅; Z = H.
**Tabelle 14:** A, B und D wie in Tabelle 1 angegeben
   W - H; X = CH₃; Y = H; Z = C₂H₅.
**Tabelle 15:** A, B und D wie in Tabelle 1 angegeben
   W = H; X = CH₃; Y = CH₃; Z = C₂H₅.

Im einzelnen seien außer den bei den Herstellungsbeispielen genannten Verbindungen die folgenden Verbindungen der Formel (I-2-a) genannt:

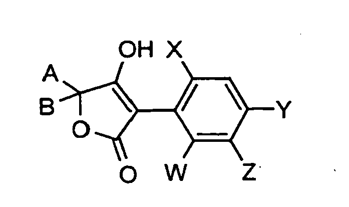

**Tabelle 16: W = CH₃, X = CH₃, Y = C≡CH, Z = H**

| A | B |
|---|---|
| CH₃ | H |
| C₂H₅ | H |
| C₃H₇ | H |
| i-C₃H₇ | H |
| C₄H₉ | H |
| i-C₄H₉ | H |
| s-C₄H₉ | H |
| t-C₄H₉ | H |
| CH₃ | CH₃ |
| C₂H₅ | CH₃ |
| C₃H₇ | CH₃ |
| i-C₃H₇ | CH₃ |
| C₄H₉ | CH₃ |
| i-C₄H₉ | CH₃ |
| s-C₄H₉ | CH₃ |
| t-C₄H₉ | CH₃ |
| C₂H₅ | C₂H₅ |
| C₃H₇ | C₃H₇ |
| 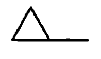 | CH₃ |
| 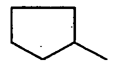 | CH₃ |
| 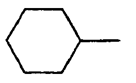 | CH₃ |
| -(H₂)₂- | |
| -(CH₂)₄- | |
| -(CH2)5- | |
| -(CH₂)₆- | |
| -(CH₂)₇- | |
| -(CH₂)₂-O-(CH₂)₂- | |
| -CH₂-O-(CH₂)₃- | |
| -(CH₂)₂-S-(CH₂)₂- | |
| -CH₂-CHCH₃-(CH₂)₃- | |
| -(CH₂)₂-CHCH₃-(CH₂)₂- | |
| (CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CHi-C₃H₇-(CH₂)₂- | |
| -(CH₂)₂-C(CH₃)₂-(CH₂)₂- | |
| -CH₂-(CHCH₃)₂-(CH2)₂- | |
| 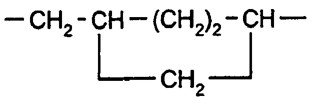 | |
| 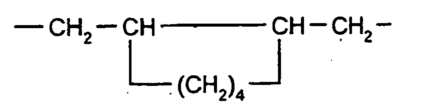 | |
| 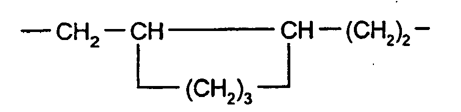 | |
| 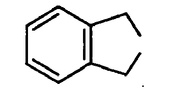 | |
| 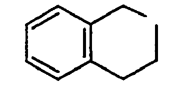 | |

**Tabelle 17:** A und B wie in Tabelle 16 angegeben
   W = CH₃; X = CH₃; Y = CH=CH₂; Z = H.
**Tabelle 18:** A und B wie in Tabelle 16 angegeben
   W = CH₃; X = C₂H₅; Y = CH₃; Z = H.
**Tabelle 19:** A und B wie in Tabelle 16 angegeben
   W = CH₃; X = CH₃; Y = C₂H₅; Z = H.
**Tabelle 20:** A und B wie in Tabelle 16 angegeben
   W = C₂H₅; X = C₂H₅; Y = CH₃; Z = H.
**Tabelle 21:** A und B wie in Tabelle 16 angegeben
   W=C₂H₅; X=C₂H₅; Y=C₂H₅ ;Z=H.
**Tabelle 22:** A und B wie in Tabelle 16 angegeben
   W = CH₃; X = C≡CH; Y = CH₃; Z = H.
**Tabelle 23:** A und B wie in Tabelle 16 angegeben
   W = CH₃; X = CH=CH₂; Y = CH₃; Z = H.
**Tabelle 24:** A und B wie in Tabelle 16 angegeben
   W = CH₃; X = C₂H₅; Y = C₂H₅; Z = H.
**Tabelle 25:** A und B wie in Tabelle 16 angegeben
   W = H; X = CH₃; Y = C≡CH; Z = H.
**Tabelle 26:** A und B wie in Tabelle 16 angegeben
   W = H; X = CH₃; Y = CH=CH₂; Z = H.
**Tabelle 27:** A und B wie in Tabelle 16 angegeben
   W = H; X = C₂H₅; Y = CH₃; Z = H.
**Tabelle 28:** A und B wie in Tabelle 16 angegeben
   W = H; X = CH₃; Y = C₂H₅; Z = H.
**Tabelle 29:** A und B wie in Tabelle 16 angegeben
   W = H; X = CH₃; Y = H; Z = C₂H₅.
**Tabelle 30:** A und B wie in Tabelle 16 angegeben
   W = H; X = CH₃; Y = CH₃; Z = C₂H₅.

Verwendet man gemäß Verfahren (A) N-(2-Methyl-4-ethinyl-phenylacetyl)-1-aminocyclohexan-carbonsäureethylester als Ausgangsstoff, so kann der Verlauf des erfindungsgemäßen Verfahrens durch folgendes Reaktionsschema wiedergegeben werden:

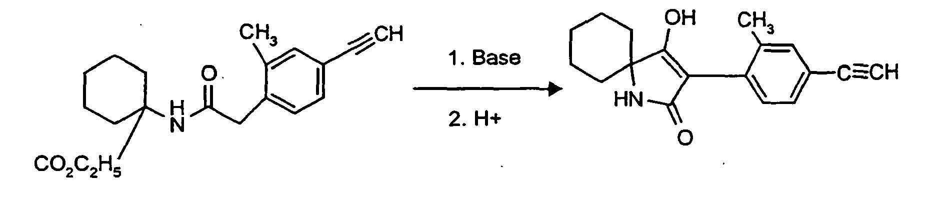

Verwendet man gemäß Verfahren (B) O-(2,4,6-Triethyl-phenylacetyl)-2-hydroxyisobuttersäureethylester, so kann der Verlauf des erfindungsgemäßen Verfahrens durch folgendes Reaktionsschema wiedergegeben werden:

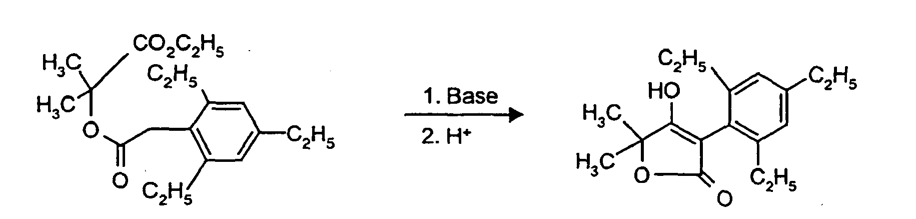

Verwendet man gemäß Verfahren (C) 2-(2,6-Dimethyl-4-ethyl-phenyl)-4-(4-methoxy)-benzylmercapto-4-methyl-3-oxo-valeriansäure-ethylester, so kann der Verlauf des erfindungsgemäßen Verfahrens durch folgendes Reaktionsschema wiedergegeben werden:

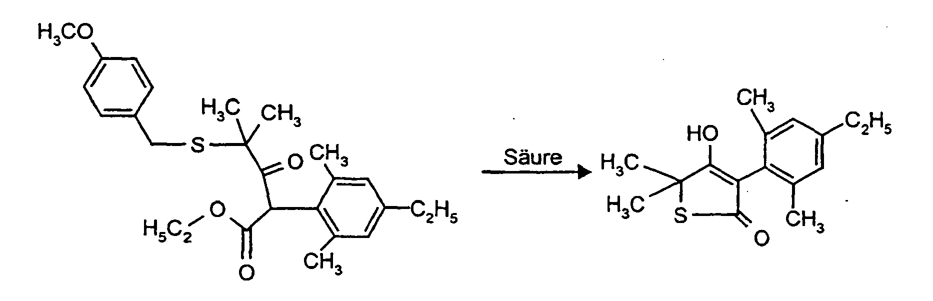

Verwendet man beispielsweise gemäß Verfahren (D) (Chlorcarbonyl)-2-(2-ethyl-4,6-dimethyl)-phenyl)-keten und Aceton als Ausgangsverbindungen, so kann der Verlauf des erfindungsgemäßen Verfahrens durch das folgende Reaktionsschema wiedergegeben werden:

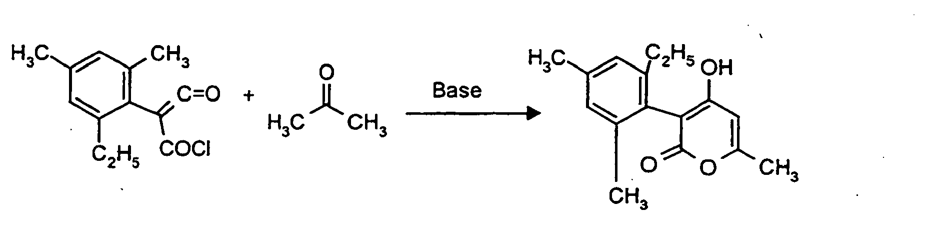

Verwendet man beispielsweise gemäß Verfahren (E) (Chlorcarbonyl)-2-(2,6-dimethyl-4-ethyl-phenyl)-keten und Thiobenzamid als Ausgangsverbindungen, so kann der Verlauf des erfindungsgemäßen Verfahrens durch das folgende Reaktionsschema wiedergegeben werden:

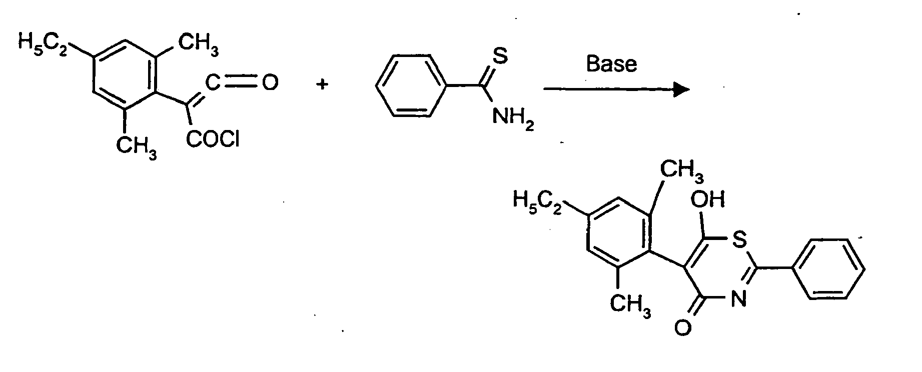

Verwendet man gemäß Verfahren (F) 5-(2,4-Diethyl-6-methyl-phenyl)-2,3-tetramethylen-4-oxo-valeriansäureethylester, so kann der Verlauf des erfindungsgemäßen Verfahrens durch folgendes Reaktionsschema wiedergegeben werden:

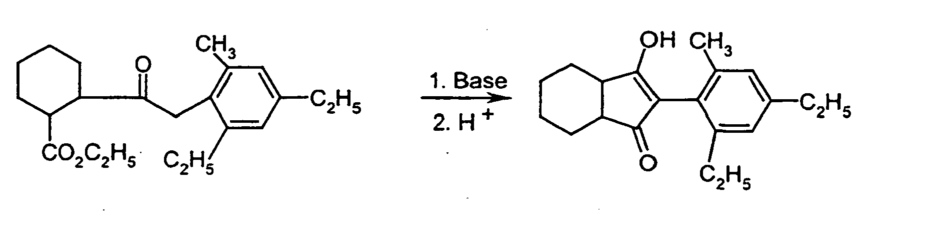

Verwendet man gemäß Verfahren (H) 3-[(2,6-Dimethyl-4-brom)-phenyl]-4,4-(pentamethylen)-pyrrolidin-2,4-dion als Ausgangsprodukt, so kann der Reaktionsverlauf durch folgendes Schema wiedergegeben werden:

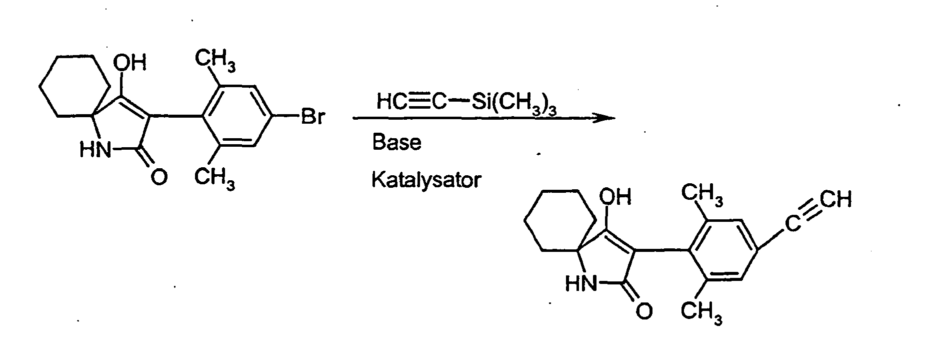

Verwendet man gemäß Verfahren (Iα) 3-(2,6-Dimethyl-4-ethinyl-phenyl)-5,5-dimethylpyrrolidin-2,4-dion und Pivaloylchlorid als Ausgangsstoffe, so kann der Verlauf des erfindungsgemäßen Verfahrens durch folgendes Reaktionsschema wiedergegeben werden:

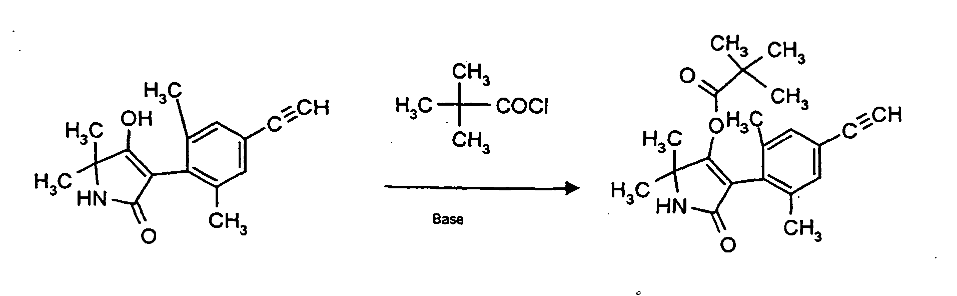

Verwendet man gemäß Verfahren (Iβ) 3-(2-Ethyl-4,6-dimethyl-phenyl)-4-hydroxy-5-phenyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

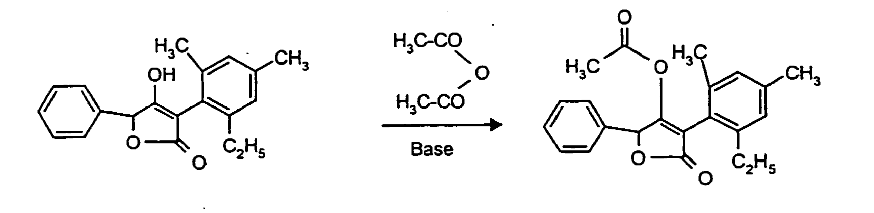

Verwendet man gemäß Verfahren (J) 8-[(2,6-Diethyl-4-methyl)-phenyl]-1-aza-bicyclo-(4,3,0^{1,6})-nonan-7,9-dion und Chlorameisensäureethoxyethylester als Ausgangsverbindungen, so kann der Verlauf des erfindungsgemäßen Verfahrens durch folgendes Reaktionsschema wiedergegeben werden:

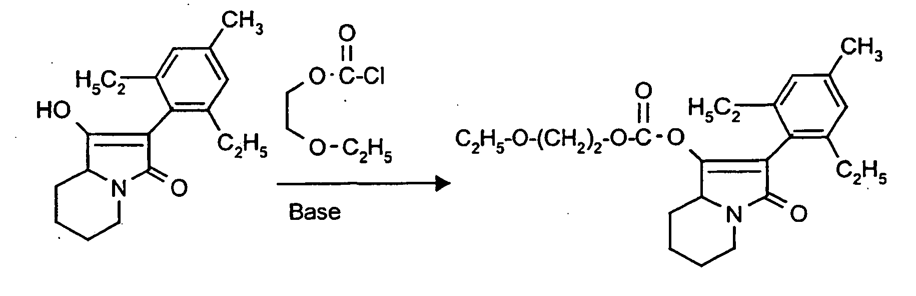

Verwendet man gemäß Verfahren (K), 3-(2-Ethyl-4,6-dimethyl -phenyl)-4-hydroxy-5-methyl-6-(3-pyridyl)-pyron und Chlormonothioameisensäuremethylester als Ausgangsprodukte, so kann der Reaktionsverlauf folgendermaßen wiedergegeben werden:

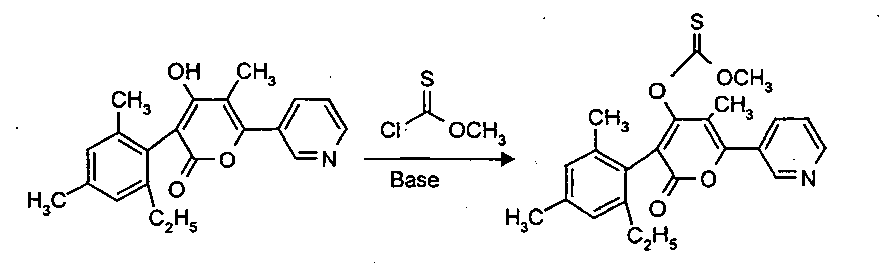

Die beim erfindungsgemäßen Verfahren (a) als Ausgangsstoffe benötigten Verbindungen der Formel (II) 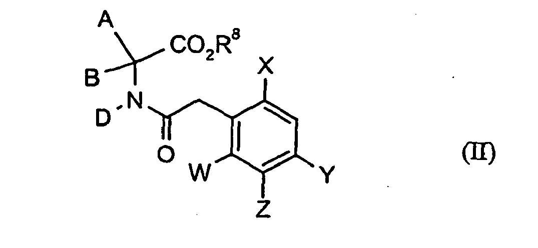 in welcher
A, B, D, W, X, Y, Z und R⁸ die oben angegebenen Bedeutungen haben,
sind neu.

Man erhält die Acylaminosäureester der Formel (II) beispielsweise, wenn man Aminosäurederivate der Formel (XXI) 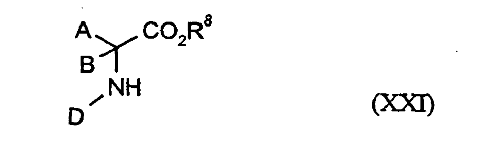 in welcher
- A, B, R⁸ und D: die oben angegebenen Bedeutungen haben,
mit substituierten Phenylessigsäurehalogeniden der Formel (XXII) 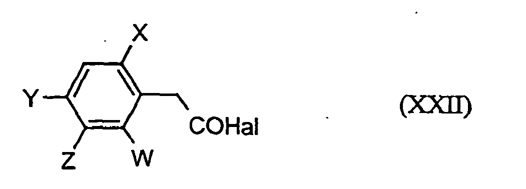 in welcher
- W, X, Y und Z: die oben angegebenen Bedeutungen haben und
- Hal: für Chlor oder Brom steht,
acyliert (Chem. Reviews 52, 237-416 (1953); Bhattacharya, Indian J. Chem. 6, 341-5, 1968)
oder wenn man Acylaminosäuren der Formel (XXIII) 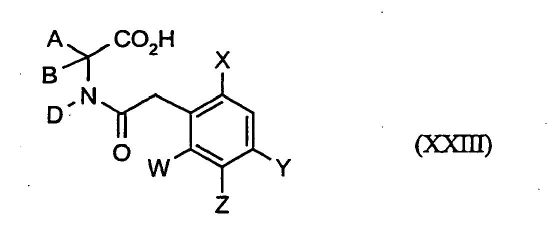 in welcher
- A, B, D, W, X, Y und Z: die oben angegebenen Bedeutungen haben,
verestert (Chem. Ind. (London) 1568 (1968)).

Die Verbindungen der Formel (XXIII) 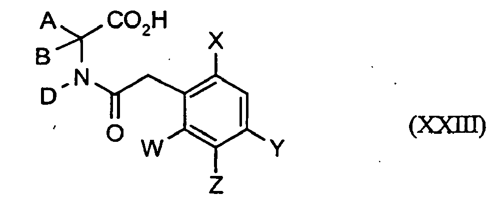 in welcher
- A, B, D, W, X, Y und Z: die oben angegebenen Bedeutungen haben,
sind neu.

Man erhält die Verbindungen der Formel (XXIII), wenn man Aminosäuren der Formel (XXIV) 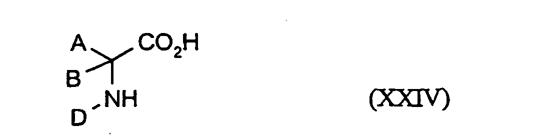 in welcher
- A, B und D: die oben angegebenen Bedeutungen haben,
mit substituierten Phenylessigsäurehalogeniden der Formel (XXII) 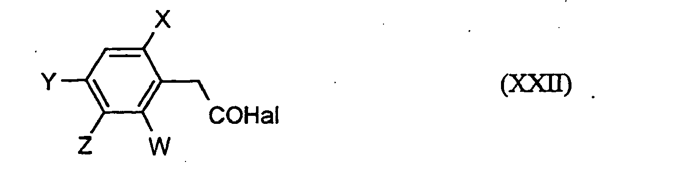 in welcher
- W, X, Y und Z: die oben angegebenen Bedeutungen haben und
- Hal: für Chlor oder Brom steht,
beispielsweise nach Schotten-Baumann acyliert (Organikum, VEB Deutscher Verlag der Wissenschaften, Berlin 1977, S. 505).

Die Verbindungen der Formel (XXII) sind neu. Sie lassen sich nach im Prinzip bekannten Verfahren darstellen (s. z.B. H. Henecka, Houben-Weyl, Methoden der Organischen Chemie, Bd. 8, S. 467-469 (1952)).

Man erhält die Verbindungen der Formel (XXII) beispielsweise, indem man substituierte Phenylessigsäuren der Formel (XXV) 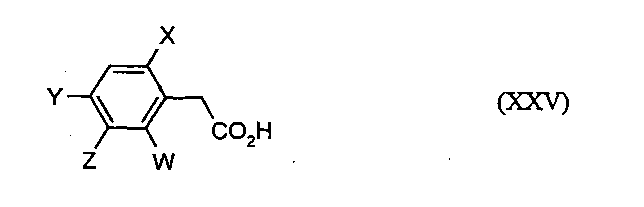 in welcher
- W, X, Y und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XXI) und (XXIV) sind teilweise bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XXIV), in der A und B einen Ring bilden, sind im allgemeinen nach der Bucherer-Bergs-Synthese oder nach der Strecker-Synthese erhältlich und fallen dabei jeweils in unterschiedlichen Isomerenformen an. So erhält man unter den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen.

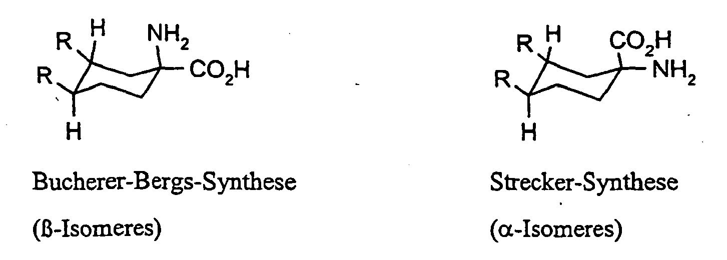

(L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 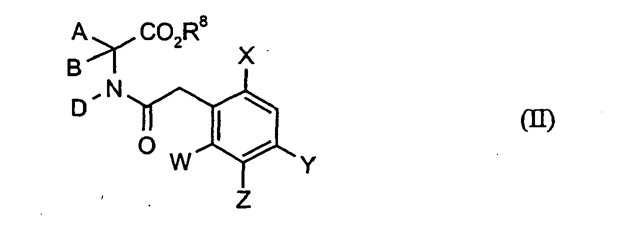 in welcher
A, B, D, W, X, Y, Z und R⁸ die oben angegebenen Bedeutungen haben,
herstellen, wenn man Aminonitrile der Formel (XXVI) 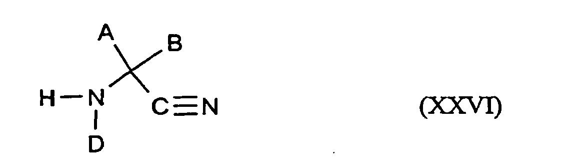 in welcher
- A, B und D: die oben angegebenen Bedeutungen haben,
mit substituierten Phenylessigsäurehalogeniden der Formel (XXII) 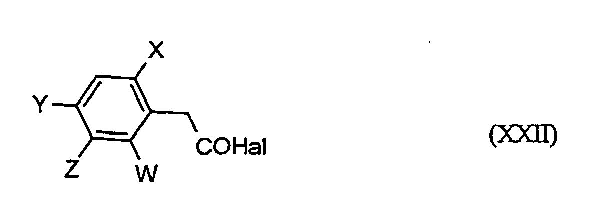 in welcher
W, X, Y, Z und Hal die oben angegebenen Bedeutungen haben,
zu Verbindungen der Formel (XXVII) 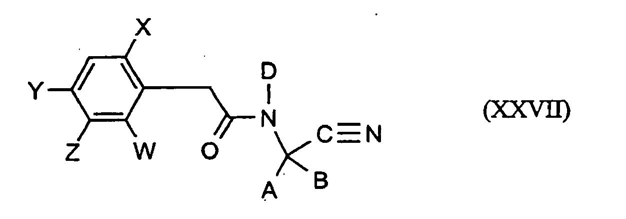 in welcher
- A, B, D, W, X, Y und Z: die oben angegebenen Bedeutungen haben,
umsetzt,
und diese anschließend einer sauren Alkoholyse unterwirft.

Die Verbindungen der Formel (XXVII) sind ebenfalls neu.

Die bei dem erfindungsgemäßen Verfahren (B) als Ausgangstoffe benötigten Verbindungen der Formel (III) 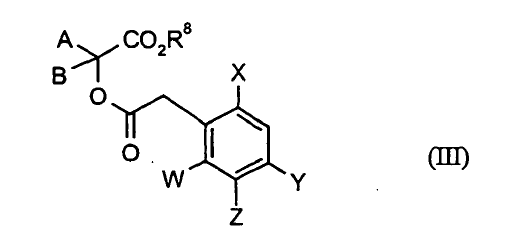 in welcher
- A, B, W, X, Y, Z und R⁸: die oben angegebenen Bedeutungen haben,
sind neu.

Sie lassen sich nach im Prinzip bekannten Methoden herstellen.

So erhält man die Verbindungen der Formel (III) beispielsweise, wenn man 2-Hydroxycarbonsäureester der Formel (XXVIII) 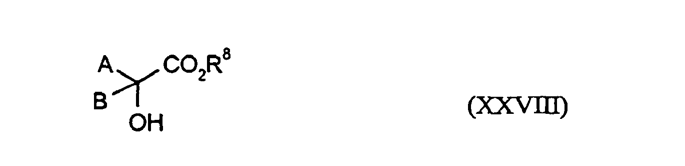 in welcher
- A, B und R⁸: die oben angegebenen Bedeutungen haben,
mit substituierten Phenylessigsäurehalogeniden der Formel (XXII) 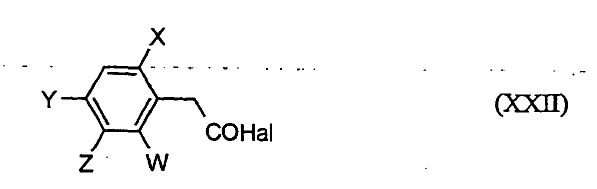 in welcher
- W, X, Y, Z und Hal: die oben angegebenen Bedeutungen haben,
acyliert (Chem. Reviews 52, 237-416 (1953)).

Weiterhin erhält man Verbindungen der Formel (III), wenn man substituierte Phenylessigsäuren der Formel (XXV) 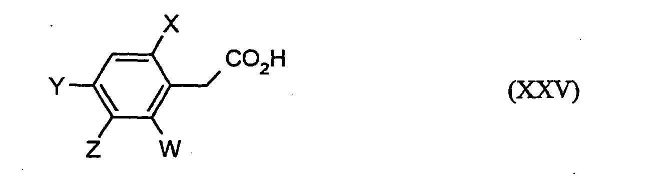 in welcher
- W, X, Y und Z: die oben angegebenen Bedeutungen haben,
mit α-Halogencarbonsäureestern der Formel (XXIX) 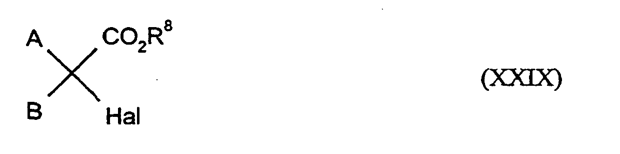 in welcher
- A, B und R⁸: die oben angegebenen Bedeutungen haben und
- Hal: für Chlor oder Brom steht,
alkyliert.

Die Verbindungen der Formel (XXV) sind neu.

Die Verbindungen der Formel (XXIX) sind käuflich.

Beispielsweise erhält man die Verbindungen der Formel (XXV), 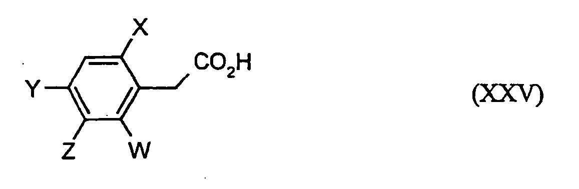 in welcher
- W, X, Y und Z: die oben angegebenen Bedeutungen haben,
- und Y: außerdem für -C≡C-Si(CH₃)₃ stehen kann,
wenn man Phenylessigsäureester der Formel (XXX) 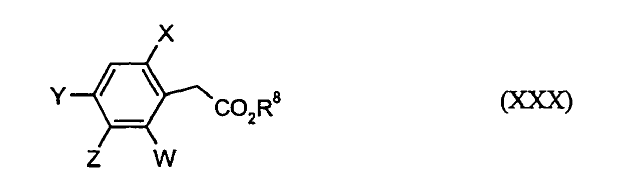 in welcher
- W, X, Y, Z und R⁸: die oben angegebene Bedeutung haben,
in Gegenwart von Säuren oder Basen, in Gegenwart eines Lösungsmittels unter allgemein bekannten Standardbedingungen verseift.

Die Verbindungen der Formel (XXX) sind neu.

Die Verbindungen der Formel (XXX) 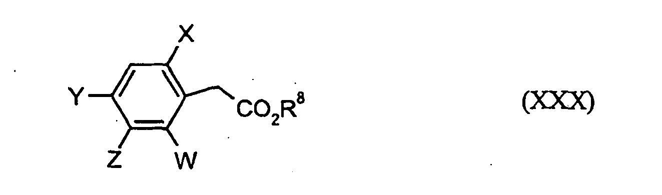 in welcher
- W, X. Y, Z und R⁸: die oben angegebene Bedeutung haben,
- und Y: außerdem für -C≡C-Si(CH₃)₃ stehen kann,
erhält man beispielsweise,
wenn man Phenylessigsäureester der Formel (XXX-a) 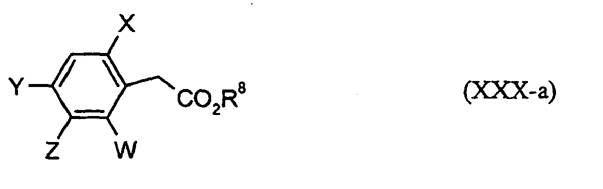 in welcher
- R⁸, W, X, Y und Z: die oben angegebene Bedeutung haben,
und ein oder zwei der Reste insbesondere ein Rest W, X, Y oder Z für Chlor, Brom oder Jod, insbesondere für Brom steht, mit der Maßgabe, dass die anderen Reste W, X, Y oder Z nicht für Alkenyl oder Alkinyl stehen,
mit Silylacetylen der Formel (X-a) oder Vinylstananen der Formel (X-b) 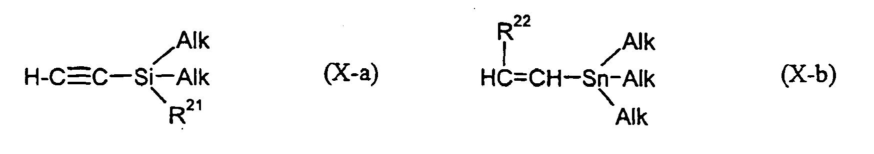 in welchen Alk, bevorzugt für C₁-C₄-Alkyl,
- R²¹: bevorzugt für C₁-C₄-Alkyl oder Phenyl steht und
- R²²: die oben angegebene Bedeutung hat,
in Gegenwart eines Lösungsmittels, gegebenenfalls in Gegenwart einer Base und eines Katalysators (bevorzugt eines der oben genannten Palladiumkomplexe) umsetzt.

Die Phenylessigsäureester der Formel (XXX-a) sind teilweise aus den Anmeldungen WO 96/35 664, WO 97/02 243, WO 97/01535, WO 97/36868 oder WO 98/05638 bekannt oder lassen sich nach den dort beschriebenen Verfahren herstellen.

Weiterhin erhält man Phenylessigsäureester der Formel (XXX) nach den weiter hinten beschriebenen Verfahren (P) und (Q).

Die bei dem obigen Verfahren (C) als Ausgangsstoffe benötigten Verbindungen der Formel (IV) 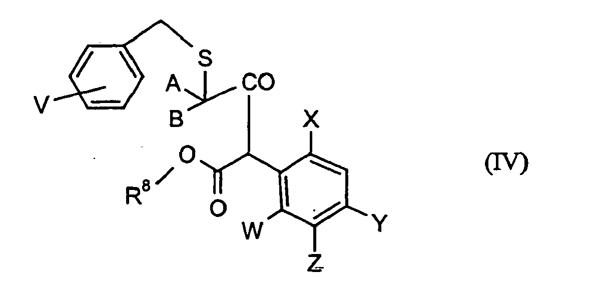 in welcher
A, B, V, W, X, Y, Z und R⁸ die oben angegebenen Bedeutungen haben,
sind neu.

Sie lassen sich nach im Prinzip bekannten Methoden herstellen.

Man erhält die Verbindungen der Formel (IV) beispielsweise, wenn man substituierte Phenylessigsäureester der Formel (XXX) 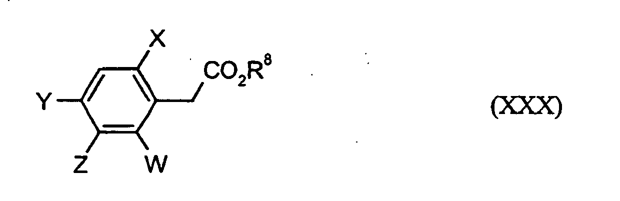 in welcher
- W, X, Y, R⁸ und Z: die oben angegebenen Bedeutungen haben,
mit 2-Benzylthio-carbonsäurehalogeniden der Formel (XXXI) 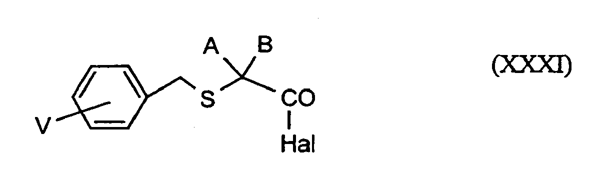 in welcher
- A, B und V: die oben angegebenen Bedeutungen haben und
- Hal: für Halogen (insbesondere Chlor oder Brom) steht,
in Gegenwart von starken Basen acyliert (siehe z.B. M.S. Chambers, E.J. Thomas, D.J. Williams, J. Chem. Soc. Chem. Commun., (1987), 1228).

Die Benzylthio-carbonsäurehalogenide der Formel (XXXI) sind teilweise bekannt und/oder lassen sich nach bekannten Verfahren herstellen (J. Antibiotics (1983), 26, 1589).

Die bei den obigen Verfahren (D) und (E) als Ausgangsstoffe benötigten Halogencarbonylketene der Formel (VI) sind neu. Sie lassen sich nach im Prinzip bekannten Methoden in einfacher Weise herstellen (vgl. beispielsweise Org. Prep. Proced. Int., 7, (4), 155-158, 1975 und DE 1 945 703). So erhält man z.B. die Verbindungen der Formel (VI) 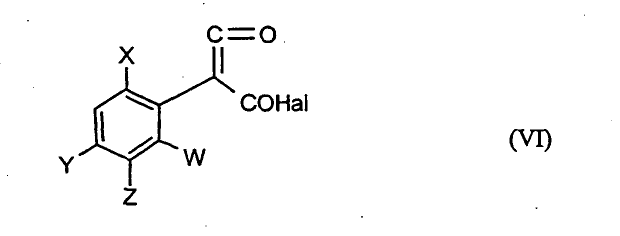 in welcher
- W, X, Y und Z: die oben angegebenen Bedeutungen haben und
- Hal: für Chlor oder Brom steht,
wenn man
substituierte Phenylmalonsäuren der Formel (XXXII) 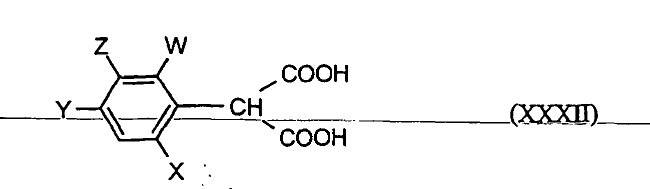 in welcher
- W, X, Y und Z: die oben angegebenen Bedeutungen haben,
mit Säurehalogeniden, wie beispielsweise Thionylchlorid, Phosphor(V)chlorid, Phosphor(III)chlorid, Oxalylchlorid, Phosgen oder Thionylbromid gegebenenfalls in Gegenwart von Katalysatoren, wie beispielsweise Diethylformamid, Methyl-Sterylformamid oder Triphenylphosphin und gegebenenfalls in Gegenwart von Basen wie z.B. Pyridin oder Triethylamin, umsetzt.

Die substituierten Phenylmalonsäuren der Formel (XXXII) sind neu. Sie lassen sich in einfacher Weise nach bekannten Verfahren herstellen (vgl. z.B. Organikum, VEB Deutscher Verlag der Wissenschaften, Berlin 1977, S. 517 ff, EP-A-528 156, WO 96/35 664, WO 97/02 243, WO 97/01535, WO 97/36868 und WO 98/05638).

So erhält man Phenylmalonsäuren der Formel (XXXII) 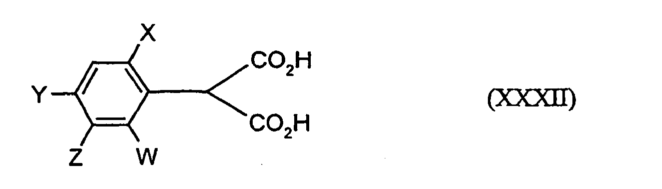 in welcher
- W, X, Y und Z: die oben angegebenen Bedeutungen haben,
wenn man Phenylmalonsäureester der Formel (XXXIII) 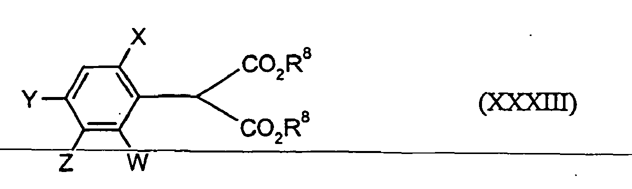 in welcher
- W, X, Y, Z und R⁸: die oben angegebene Bedeutung haben,
zunächst in Gegenwart einer Base und einem Lösungsmittel verseift und anschließend vorsichtig ansäuert (EP-528 156, WO 96/35 664, WO 97/02 243).

Die Malonsäureester der Formel (XXXIII) 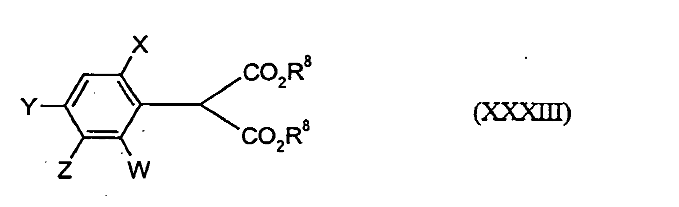 in welcher
- W, X, Y, Z und R⁸: die oben angegebene Bedeutung haben,
sind teilweise bekannt.

Sie lassen sich nach allgemein bekannten Methoden der Organischen Chemie darstellen (vgl. z.B. Tetrahedron Lett. 27, 2763 (1986), Organikum VEB Deutscher Verlag der Wissenschaften, Berlin 1977, S. 587 ff., WO 96/35664, WO 97/02243, WO 97/01535, WO 97/36868, WO 98/05638 und WO 99/47525).

Die für das erfindungsgemäße Verfahren (D) als Ausgangsstoffe benötigten Carbonylverbindungen der Formel (V) 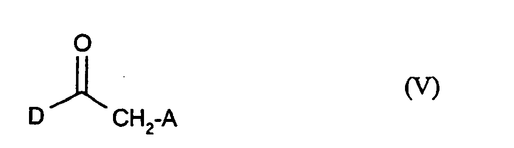 in welcher
- A und D: die oben angegebenen Bedeutungen haben,
oder deren Silylenolether der Formel (Va) 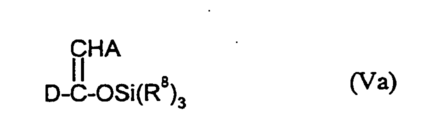 in welcher
- A, D und R⁸: die oben angegebenen Bedeutungen haben,
sind käufliche, allgemeine bekannte oder nach bekannten Verfahren zugängliche Verbindungen.

Die Herstellung der zur Durchführung des erfindungsgemäßen Verfahrens (E) als Ausgangsstoffe benötigten Ketensäurechloride der Formel (VI) wurden bereits oben beschrieben. Die zur Durchführung des erfindungsgemäßen Verfahrens (E) benötigten Thioamide der Formel (VII) 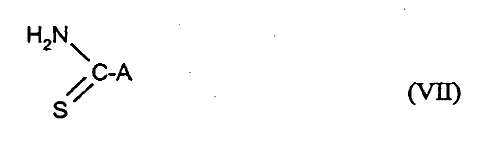 in welcher
- A: die oben angegebene Bedeutung hat,
sind allgemein in der Organischen Chemie bekannte Verbindungen.

Die bei dem obigen Verfahren (F) als Ausgangsstoffe benötigten Verbindungen der Formel (VIII) 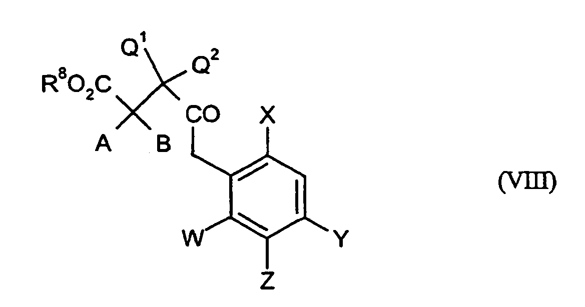 in welcher
- A, B, Q¹, Q², W, X, Y, Z und R⁸: die oben angegebene Bedeutung haben,
sind neu.

Sie lassen sich nach im Prinzip bekannten Methoden herstellen.

Man erhält die 5-Aryl-4-ketocarbonsäureester der Formel (VIII) beispielsweise, wenn man 5-Aryl-4-ketocarbonsäuren der Formel (XXXIV) 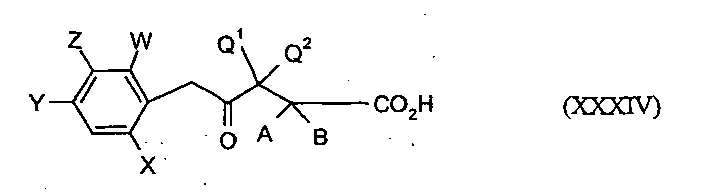 in welcher
W, X, Y, Z, A, B, Q¹ und Q² die oben angegebene Bedeutung haben,
verestert (vgl. z.B. Organikum, 15. Auflage, Berlin, 1977, Seite 499) oder alkyliert (Siene Herstellungsbeispiel).

Die 5-Aryl-4-ketocarbonsäuren der Formel (XXXIV) 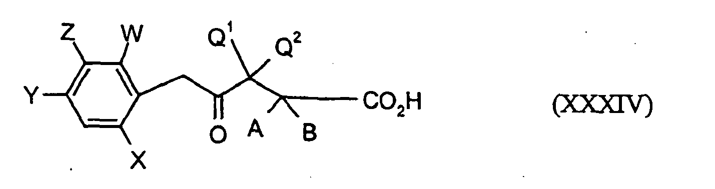 in welcher
A, B, Q¹, Q², W, X, Y und Z die oben angegebene Bedeutung haben,
sind neu, lassen sich aber nach im Prinzip bekannten Methoden herstellen (siehe Herstellungsbeispiel).

Man erhält die 5-Aryl-4-ketocarbonsäuren der Formel (XXXTV) beispielsweise, wenn man 2-Phenyl-3-oxo-adipinsäureester der Formel (XXXV) 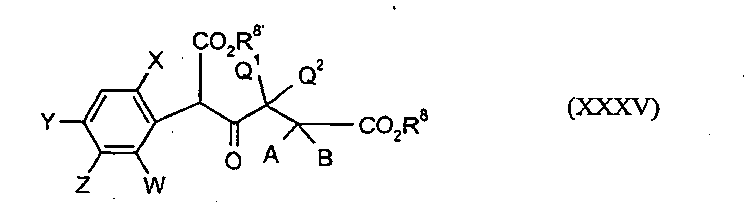 in welcher
- , B, D¹, D², W, X, Y und Z: A die oben angegebene Bedeutung haben und
- R⁸ und R⁸': für Alkyl (insbesondere C₁-C₈-Alkyl) stehen und
bei Einsatz der Verbindung der Formel (XXXVII) R⁸ für Wasserstoff steht
gegebenenfalls in Gegenwart eines Verdünnungsmittels und gegebenenfalls in Gegenwart einer Base oder Säure decarboxyliert (vgl. z.B. Organikum, 15. Auflage, Berlin, 1977, Seite 519 bis 521).

Die Verbindungen der Formel (XXXV) 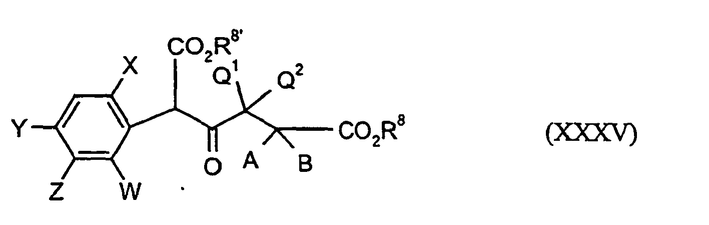 in welcher
A, B, Q¹, Q², W, X, Y, Z, R⁸, R^{8'} die oben angegebene Bedeutung haben und bei Einsatz der Verbindung der Formel (XXXVII) R⁸ für Wasserstoff steht sind neu.

Man erhält die Verbindungen der Formel (XXXV) beispielsweise,
wenn man Dicarbonsäurehalbesterchloride der Formel (XXXVI), 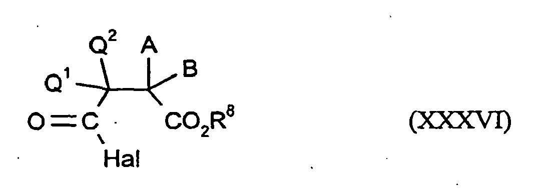 in welcher
- A, B, Q¹, Q² und R⁸: die oben angegebene Bedeutung haben und
- Hal: für Chlor oder Brom steht,
oder Carbonsäureanhydride der Formel (XXXVII) 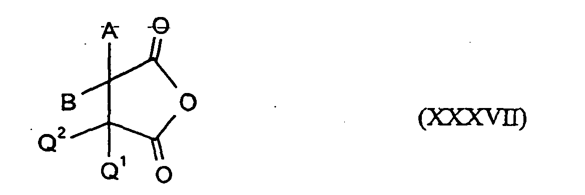 in welcher
A, B, Q¹ und Q² die oben angegebene Bedeutung haben,
mit einem Phenylessigsäureester der Formel (XXX) 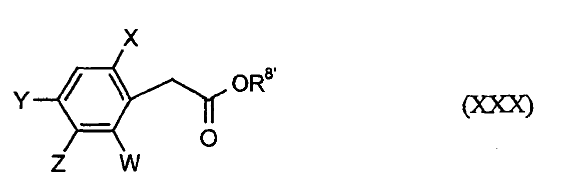 in welcher
- W, X, Y, Z und R^{8'}: die oben angegebene Bedeutung haben,
in Gegenwart eines Verdünnungsmittels und in Gegenwart einer Base acyliert (vgl. z.B. M.S. Chambers, E. J. Thomas, D.J. Williams, J. Chem. Soc. Chem. Commun., (1987), 1228, vgl. auch die Herstellungsbeispiele).

Die Verbindungen der Formeln (XXXVI) und (XXXVII) sind teilweise bekannte. Verbindungen der Organischen Chemie und/oder lassen sich nach im Prinzip bekannten Methoden in einfacher Weise herstellen.

Die Verbindungen der Formel (XXX) wurden bereits bei den Vorstufen für das Verfahren (B) beschrieben oder sind als Beispiele in den nachfolgenden Verfahren (P) und (Q) explizit beschrieben.
(P) So erhält man weiterhin Verbindungen der Formel (XXX), 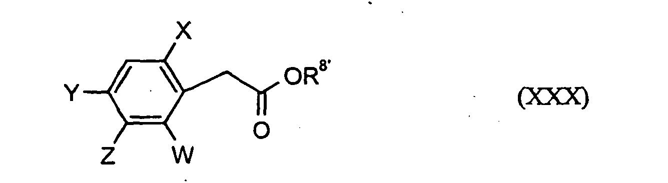 in welcher
   W, X, Y, Z und R^{8'} die oben angegebene Bedeutung haben,
   wenn man Acylphenylphenylessigsäureester der Formel (XLII) 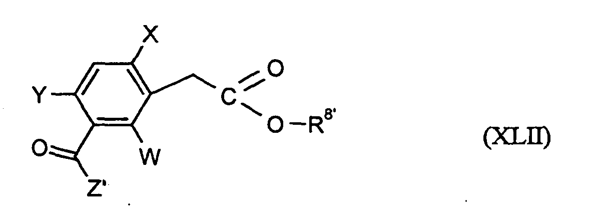 in welcher
   W, X, Y und R^{8'} die oben angegebene Bedeutung haben und
   Z' für Alkyl steht,
   gegebenenfalls in Gegenwart eines Lösungsmittels mit geeigneten Reaknossisitteln (wie z.B. Zn/HCl, Wasserstoff/Katalysator, Hydrazin/Base) reduziert.

Die Verbindungen der Formel (XLII) sind neu.

Man erhält Verbindungen der Formel (XLII) 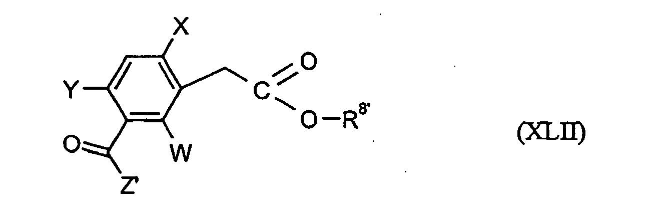 in welcher
W, X, Y, Z' und R^{8'} die oben angegebene Bedeutung haben,
wenn man Phenylessigsäureester der Formel (XXX-b) 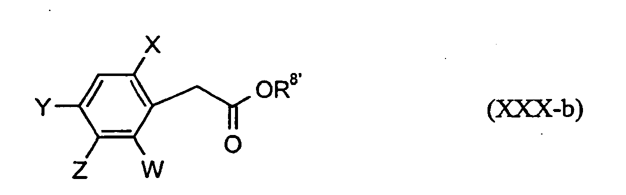 in welcher
W, X, Y und R^{8'} die oben angegebene Bedeutung haben und
Z für Wasserstoff steht,
gegebenenfalls in Gegenwart eines Lösungsmittels mit einem Carbonsäurechlorid oder Carbonsäureanhydrid in Gegenwart einer Säure oder einer Lewis-Säure (z.B. Aluminiumchlorid, Eisen(III)-bromid) nach Friedel-Crafts acyliert.

Die Verbindungen (XXX-b) sind bekannt oder nach den in der eingangs zitierten Literatur beschriebenen Verfahren herstellbar.
(Q) Weiterhin erhält man Phenylessigsäureester der Formel (XXX), 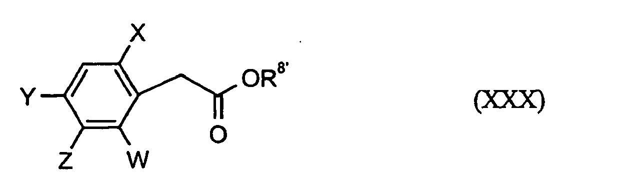 in welcher
   - X: für Alkyl,
   - W, Y und Z: für Wasserstoff oder Alkyl und
   - R^{8'}: für Alkyl steht,
   wenn man Phenylessigsäureester der Formel (XXX-c), 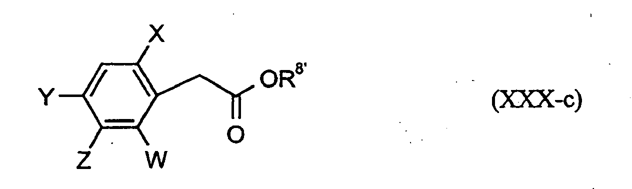 in welcher
   - X: für Alkyl,
   - R^{8'}: für Alkyl und
   - W, Y und Z: neben Wasserstoff und Alkyl auch für Chlor oder Brom stehen können,
   in Gegenwart eines Lösungsmittels und in Gegenwart eines Reduktionsmittels (z.B. Wasserstoff in Gegenwart eines Edelmetallkatalysators wie beispielsweise Palladium oder Platin) enthalogeniert.

Die Verbindungen der Formel (XXX-c) sind aus den eingangs zitierten Patentanmeldungen bekannt oder lassen sich nach den dort beschriebenen Verfahren herstellen.

Die bei dem obigen Verfahren (H) als Ausgangsstoffe benötigten Verbindungen der Formeln (I-1'a) bis (I-8'-a), in welchen A, B, D, Q¹, Q², Q³, Q⁴, Q⁵, Q⁶, W, X', Y' und Z' die oben angegebene Bedeutung haben, sind teilweise bekannt (WO 96/35 664, WO 97/02 243, WO 97/01535, WO 97/36868, WO 98/05638) oder lassen sich gemäß den dort beschriebenen Verfahren herstellen.

Die Kupplungsreagenzien der Formel (X-a) und (X-b) 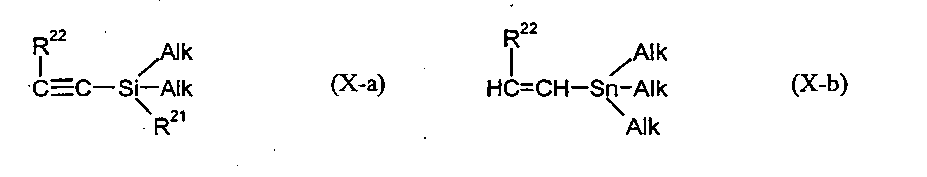 in welcher
Alk, R²¹ und R²² die oben angegebene Bedeutung haben,
sind teilweise käuflich oder lassen sich nach allgemein bekannten Verfahren in einfacher Weise herstellen.

Die zur Durchführung der erfindungsgemäßen Verfahren (I), (J), (K), außerdem als Ausgangsstoffe benötigten Säurehalogenide der Formel (XI), Carbonsäureanhydride der Formel (XII), Chlorameisensäureester oder Chlorameisensäurethioester der Formel (XIII), Chlormonothioameisensäureester oder Chlordithioameisensäureester der Formel (XIV), sind allgemein bekannte Verbindungen der Organischen bzw. Anorganischen Chemie.

Die Verbindungen der Formeln (V), (VII), (XI) bis (XX), (XXI), (XXIV), (XXVI), (XXVITI), (XXIX), (XXXI), (XXXVI), (XXXVII) und (XLI) sind darüber hinaus aus den eingangs zitierten Patentanmeldungen bekannt und/oder lassen sich nach den dort angegebenen Methoden herstellen.

Das Verfahren (A) ist dadurch gekennzeichnet, daß man Verbindungen der Formel (II), in welcher A, B, D, W, X, Y, Z und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumokid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgerührt.

Bei der Durchführung des erfindungsgemäßen Verfahrens (A) setzt man die Reaktionskomponenten der Formel (II) und die deprotonierenden Basen im allgemeinen in etwa doppeltäquimolaren Mengen ein. Es ist jedoch auch möglich, die eine oder andere Komponente in einem größeren Überschuß (bis zu 3 Mol) zu verwenden.

Das Verfahren (B) ist dadurch gekennzeichnet, daß Verbindungen der Formel (III), in welcher A, B, W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (B) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natrium-ethylat und Kalium-tert.-butylat einsetzbar.

Die Reaktionstemperaturen können bei der Durchführung des erfindungsgemäßen Verfahrens (B) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (III) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (C) ist dadurch gekennzeichnet, daß man Verbindungen der Formel (IV) in welcher A, B, V, W, X, Y, Z und R⁸ die oben angegebene Bedeutung haben, in Gegenwart einer Säure und gegebenenfalls in Gegenwart eines Verdünnungsmittels intramolekular cyclisiert.

Als Verdünnungsmittel können bei dem erfindungsgemäßen Verfahren (C) alle inerten organischen Solventien eingesetzt werden. Vorzugsweise verwendbar sind Kohlenwasserstoffe, wie Toluol und Xylol, ferner halogenierte Kohlenwasserstoffe wie Dichlormethan, Chloroform, Ethylenchlorid, Chlorbenzol, Dichlorbenzol, außerdem polare Lösungsmittel, wie Dimethylsulfoxid Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Gegebenenfalls kann auch die eingesetzte Säure als Verdünnungsmittel dienen.

Als Säure können bei dem erfindungsgemäßen Verfahren (C) alle üblichen anorganischen und organischen Säuren eingesetzt werden wie z.B. Halogenwasserstoffsäuren, Schwefelsäure, Alkyl-, Aryl- und Haloalkylsulfonsäuren, insbesondere halogenierte Alkylcarbonsäuren wie z.B. Trifluoressigsäure.

Die Reaktionstemperaturen können bei der Durchführung des erfindungsgemäßen Verfahrens (C) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (C) wird im allgemeinen unter Normaldruck durchgeführt.

Bei der Durchführung des erfindungsgemäßen Verfahrens (C) setzt man die Reaktionskomponenten der Formeln (IV) und die Säure z.B. in äquimolaren Mengen ein. Es ist jedoch gegebenenfalls auch möglich, die Säure als Lösungsmittel oder als Katalysator zu verwenden.

Das erfindungsgemäße Verfahren (D) ist dadurch gekennzeichnet, daß man Carbonylverbindungen der Formel (V) oder deren Enolether der Formel (V-a) mit Ketensäurehalogeniden der Formel (VI) in Gegenwart eines Verdünnungsmittels und gegebenenfalls in Gegenwart eines Säureakzeptors umsetzt.

Als Verdünnungsmittel können bei dem erfindungsgemäßen Verfahren (D) alle inerten organischen Solventien eingesetzt werden. Vorzugsweise verwendbar sind Kohlenwasserstoffe, wie Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid oder N-Methyl-pyrrolidon.

Als Säureakzeptoren können bei der Durchführung der erfindungsgemäßen Verfahrensvariante (D) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung der erfindungsgemäßen Verfahrensvariante (D) innerhalb eines größeren Bereiches variiert werden. Zweckmäßigerweise arbeitet man bei Temperaturen zwischen 0°C und 250°C, vorzugsweise zwischen 50°C und 220°C.

Das erfindungsgemäße Verfahren (D) wird zweckmäßigerweise unter Normaldruck durchgeführt.

Bei der Durchführung des erfindungsgemäßen Verfahrens (D) setzt man die Reaktionskomponenten der Formeln (V) und (VI), in welchen A, D, W, X, Y und Z die oben angegebenen Bedeutungen haben und Hal für Halogen steht, und gegebenenfalls die Säureakzeptoren im allgemeinen in etwa äquimolaren Mengen ein. Es ist jedoch auch möglich, die eine oder andere Komponente in einem größeren Überschuß (bis zu 5 Mol) zu verwenden.

Das erfindungsgemäße Verfahren (E) ist dadurch gekennzeichnet, daß man Thioamide der Formel (VII) mit Ketensäurehalogeniden der Formel (VI) in Gegenwart eines Verdünnungsmittels und gegebenenfalls in Gegenwart eines Säureakzeptors umsetzt.

Als Verdünnungsmittel können bei der erfindungsgemäßen Verfahrensvariante (E) alle inerten organischen Solventien eingesetzt werden. Vorzugsweise verwendbar sind Kohlenwasserstoffe, wie Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid und N-Methyl-pyrrolidon.

Als Säureakzeptoren können bei der Durchführung des erfindungsgemäßen Verfahrens (E) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung des erfindungsgemäßen Verfahrens (E) innerhalb eines größeren Bereiches variiert werden. Zweckmäßigerweise arbeitet man bei Temperaturen zwischen 0°C und 250°C, vorzugsweise zwischen 20°C und 220°C.

Das erfindungsgemäße Verfahren (E) wird zweckmäßigerweise unter Normaldruck durchgeführt.

Bei der Durchführung des erfindungsgemäßen Verfahrens (E) setzt man die Reaktionskomponenten der Formeln (VII) und (VI), in welchen A, W, X, Y und Z die oben angegebenen Bedeutungen haben und Hal für Halogen steht und gegebenenfalls die Säureakzeptoren im allgemeinen in etwa äquimolaren Mengen ein. Es ist jedoch auch möglich, die eine oder andere Komponente in einem größeren Überschuß (bis zu 5 Mol) zu verwenden.

Das Verfahren (F) ist dadurch gekennzeichnet, daß man Verbindungen der Formel (VIII), in welcher A, B, Q¹, Q², W, X, Y, Z und R⁸ die oben angegebene Bedeutung haben, in Gegenwart einer Base einer intramolekularen Kondensation unterwirft.

Als Verdünnungsmittel können bei dem erfindungsgemäßen Verfahren (F) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (F) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natriummethylat, Natrium-ethylat und Kalium-tert.-butylat einsetzbar.

Die Reaktionstemperaturen können bei der -Durchführung des erfindungsgemäßen Verfahrens (F) innerhalb eines größeren Bereiches variiert werden. Im allgemeinen arbeitet man bei Temperaturen zwischen -75°C und 250°C, vorzugsweise zwischen -50°C und 150°C.

Das erfindungsgemäße Verfahren (F) wird im allgemeinen unter Normaldruck durchgeführt.

Bei der Durchführung des erfindungsgemäßen Verfahrens (F) setzt man die Reaktionskomponenten der Formel (VIII) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Zur Durchführung des erfindungsgemäßen Verfahrens (H) sind Palladium(0)-Komplexe als Katalysator geeignet. Bevorzugt wird beispielsweise Tetrakis-(triphenylphosphin)palladium oder Bis-(triphenylphosphin)-palladium-dichlorid/Triphenylphosphin.

Als Säureakzeptoren zur Durchführung des erfindungsgemäßen Verfahrens (H) kommen anorganische oder organische Basen in Frage. Hierzu gehören vorzugsweise Erdalkalimetall- oder Alkalimetallhydroxide, -acetate, -carbonate oder -hydrogencarbonate, wie beispielsweise Natrium-, Kalium-, Barium- oder Ammoniumhydroxid, Natrium-, Kalium-, Calcium- oder Ammoniumacetat, Natrium-, Kalium- oder Ammoniumcarbonat, Natriumhydrogen- oder Kaliumhydrogencarbonat, Alkalifluoride, wie beispielsweise Cäsiumfluorid, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens (H) kommen Wasser, organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Dicalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Düsopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol; Alkohole, wie Methanol, Ethanol, n- oder i-Propanol, n-, iso-, sek.- oder tert.-Butanol, Ethandiol, Propan-1,2-diol, Ethoxyethanol, Methoxyethanol, Diethylenglykolmonomethylether, Diethylenglykohnonomethylether; Wasser.

Die Reaktionstemperatur kann bei dem erfindungsgemäßen Verfahren (H) innerhalb eines größeren Bereiches variiert werden. Im allgemeinen arbeitet man bei Temperaturen zwischen 0°C und +140°C, bevorzugt zwischen 50°C und +100°C.

Bei der Durchführung des erfindungsgemäßen Verfahrens (H) werden die Kupplungsreagenzien der Formel (X-a) oder (X-b) und Verbindungen der Formeln (I-1'-a) bis (I-6'-a) im molaren Verhältnis 1:1 bis 5:1, vorzugsweise 1:1 bis 2:1 eingesetzt. Vom Katalysator setzt man im allgemeinen 0,005 bis 0,5 Mol, vorzugsweise 0,01 Mol bis 0,1 Mol pro Mol der Verbindungen der Formeln (I-1'-a) bis (I-6'-a) ein. Die Base setzt man im allgemeinen in einem Überschuß ein.

Das Verfahren (I-α) ist dadurch gekennzeichnet, daß man Verbindungen der Formeln (I-1-a) bis (I-6-a) jeweils mit Carbonsäurehalogeniden der Formel (XI) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (I-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (I-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (I-α) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des ernndungsgemäßen Verfahrens (I-α) werden die Ausgangsstoffe der Formeln (I-1-a) bis (I-6-a) und das Carbonsäurehalogenid der Formel (XI) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (I-ß) ist dadurch gekennzeichnet, daß man Verbindungen der Formeln (I-1-a) bis (I-6-a) mit Carbonsäureanhydriden der Formel (XII) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (I-ß) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (I-ß) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (I-ß) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (I-ß) werden die Ausgangsstoffe der Formeln (I-1-a) bis (I-6-a) und das Carbonsäureanhydrid der Formel (XII) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (J) ist dadurch gekennzeichnet, daß man Verbindungen der Formeln (I-1-a) bis (I-6-a) jeweils mit Chlorameisensäureestern oder Chlorameisensäurethiolestern der Formel (XIII) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (J) alle üblichen Säureakzeptoren in Betracht. Vorzugsweise verwendbar sind tertiäre Amine, wie Triethylamin, Pyridin, DABCO, DBU, DBA, Hünig-Bäse und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (J) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (J) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (J) wird im allgemeinen unter Normaldruck durchgeführt.

Bei der Durchführung des erfindungsgemäßen Verfahrens (J) werden die Ausgangsstoffe der Formeln (I-1-a) bis (I-6-a) und der entsprechende Chlorameisensäureester bzw. Chlorameisensäurethiolester der Formel (XIII) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das erfindungsgemäße Verfahren (K) ist dadurch gekennzeichnet, daß man Verbindungen der Formeln (I-1-a) bis (I-6-a) jeweils mit Verbindungen der Formel (XIV) in Gegenwart eines Verdünnungsmittels und gegebenenfalls in Gegenwart eines Säurebindemittels umsetzt.

Beim Herstellungsverfahren (K) setzt man pro Mol Ausgangsverbindung der Formeln (I-1-a) bis (I-6-a) ca. 1 Mol Chlormonothioameisensäureester bzw. Chlordithioameisensäureester der Formel (XIV) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-6-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp..

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica.

Aus der Ordnung der Dermaptera z.B. Forficula auricularia.

Aus der Ordnung der Isoptera z.B. Reticulitermes spp..

Aus der Ordnung der Phthiraptera z.B. Pediculus humanus corporis, Haematopinus spp., Linognathus spp., Trichodectes spp., Damalinia spp..

Aus der Ordnung der Thysanoptera z.B. Hercinothrips femoralis, Thrips tabaci, Thrips palmi, Frankliniella occidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio horlulanus, Oscinella frit, Phorbia spp., Pegomyia hyoscyami, Ceratitis capitata, Dacus oleae, Tipula paludosa, Hylemyia spp., Liriomyza spp..

Aus der Ordnung der Siphonaptera z.B. Xenopsylla cheopis, Ceratophyllus spp..

Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfindunsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zur den Insektiziden zählen beispielsweise Phosphorsäureester, Carbamate, Carbonsäureester, chlorierte Kohlenwasserstoffe, Phenylhamstoffe, durch Mikroorganismen hergestellte Stoffe u.a.

Besonders günstige Mischpartner sind z.B. die folgenden:

### Fungizide

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat, Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthena.t, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb; Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol;
α-(5-Methyl-1,3-dioxan-5-yl)β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-l-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethmyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrmidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrcl-2,5-dion
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin, 8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-trazol-1-yl)-cycloheptanol,
cis-4-[3-[4-(1,1-Dimethylpropyl)-phenyl-2-methylpropyl]-2,6-dimethyl-morpholin-hydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alani.nat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-fiuranyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N-methoxy-methanimidamid,
N-Formyl-N-hydroxy-DL-alanin -Natrium
O,O-Diethyl-[2-(dipropylamino)-2-oxoethyl]-ethylphosphoramidothioat,
O-Methyl-S-phenyl-phenylpropylphosphorainidothioat,
S-Methyl-1,2,3-benzothiadiazol-7-carbothioat,
spiro[2H]-1-Benzopyran-2,1'(3'H)-isobenzofuran]-3'-on,

### Bakterizide

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,
Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos, Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene,
Imidacloprid, Isazofos, Isofenphos, Isoxathion, Ivermectin,
Kernpolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon,
Triflumuron, Trimethacarb,
Vamidothion, Vaniliprole, Verticillium lecanii
YI 5302
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat 1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol 2-(Acetlyoxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid 3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3(2H)-pyridazinon
4-Chlor-5-[(6-chlor-3-pyridinyl)methoxy]-2-(3,4-dichlorphenyl)-3(2H)-pyridazinon Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N'-nitro-guanidin
N-Methyl-N-(1 -methyl-2-propenyl)-1,2-hydrazindicarbothioamid
N-Methyl-N'-2-propenyl-1,2-hydrazindicarbothioamid
O,O-Diethyl-[2-(dipropylamino)-2-oxoethyl]-ethylphosphoramidothioat

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpudems sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden. Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel-(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthzin, Cypermethrin, Deltamethrin, Permethrin, Imidacloprid, NI-25, Flufenoxuron, Hexaflumuron, Transfluthrin, Thiacloprid, Methoxyphenoxid und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflußkrebse) zusammengefaßt werden, besondere Bedeutung zu.

Es wurde nun überraschenderweise gefunden, daß die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis-(trialkylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-n-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-tert.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[b]thiophencarbonsäurecyclohexylamid-S,S-dioxid, Dichlofluanid, Fluorfolpet, 3-Iod-2-propinyl-butylcarbamat, Tolylfluanid und Azole wie Azaconazole, Cyproconazol, Epoxyconazole, Hexaconazole, Metconazole, Propiconazole und Tebuconazole;
   Molluskizide wie
   Fentinacetat, Metaldehyd, Methiocarb, Niclosamid, Thiodicarb und Trimethacarb;
oder herkömmliche Antifouling-Wirkstoffe wie 4,5-Dichlor-2-octyl-4-isothiazolin-3-on, Diiodmethylparatrylsulfon, 2-(N,N-Di-methylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wäßrigen System, Vinylchlorid/Vinylacetat-Copolymersysteme in Form wäßriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium,

Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.

Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Wie bereits oben erwähnt, können erfindungsgemäße alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIAA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der Formel (I) behandelt werden. Die bei den Wirkstoffen oben angegebenen Vorzugsbereiche gelten auch für die Behandlung dieser Pflanzen. Besonders hervorgehoben sei die Pflanzenbehandlung mit den im vorliegenden Text speziell aufgeführten Mischungen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmitteln als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise

Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, BAS-662H, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesseningsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele

### Beispiel I-1-a-1

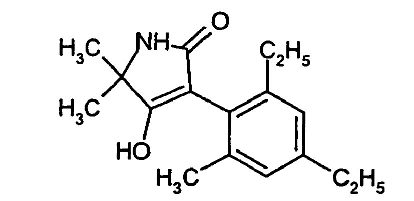

Zu 6,5 g Kalium-tert.-butylat in 25 ml wasserfreiem Dimethylformamid (DMF) tropft man bei 60°C 7,4 g der Verbindung gemäß Beispiel n-1 gelöst in 1 ml wasserfreiem DMF und rührt unter dünnschichtchromatographischer Kontrolle weiter. Nach Beendigung der Reaktion gibt man 170 ml Eiswasser hinzu, säuert mit konz. Salzsäure bei 0°C bis 10°C auf pH 2 an, saugt ab und wäscht mit Eiswasser nach. Der Rückstand wird an Kieselgel mit Methylenchlorid/Methanol 9:1 als Laufmittel chromatographisch gereinigt.
Ausbeute: 3,90 g (≙ 58.00 % d. Theorie), Fp. 199°C

In Analogie zu Beispiel (I-1-a-1) und gemäß den allgemeinen Angaben zur Herstellung wurden folgende Verbindungen der Formel (I-a-1) erhalten

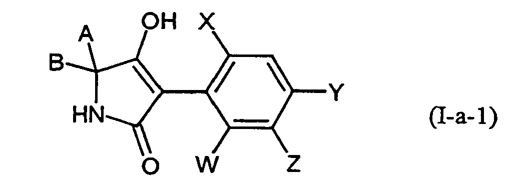

| Bsp.-Nr. | W | X | Y | Z | A | B | Fp.°C | Isomer |
|---|---|---|---|---|---|---|---|---|
| I-1-a-2 | CH₃ | C₂H₅ | C₂H₅ | H | -(CH₂)₂-CHCH₃-(CH₂)₂- | | 154 | β |
| I-1-a-3 | CH₃ | C₂H₅ | C₂H₅ | H | -(CH₂)₂-CHOCH₃-(CH₂)₂- | | 225 | β |
| I-1-a-4 | C₂H₅ | C₂H₅ | C₂H₅ | H | CH₃ | C_{H3} | 200 | - |
| I-1-a-5 | C₂H₅ | C₂H₅ | C₂H₅ | H | -(CH₂)₂-CHCH₃-(CH₂)₂- | | 115 | β |
| I-1-a-6 | C₂H₅ | C₂H₅ | C₂H₅ | H | -(CH₂)₂-CHOCH₃-(CH₂)₂- | | 222 | β |
| I-1-a-7 | i-C₃H₇ | i-C₃H₇ | i-C₃H₇ | H | -(CH₂)₂-CHOCH₃-(CH₂)₂- | | >230 | β |
| I-1-a-8 | CH₃ | C₂H₅ | CH₃ | H | CH₃ | CH₃ | >220 | |
| I-1-a-9 | CH₃ | C₂H₅ | CH₃ | H | -(CH₂)₂-CHCH₃-(CH₂)₂- | | 114 | β |
| I-1-a-10 | C₂H₅ | C₂H₅ | H | H | -(CH₂)₂-CHCH₃-(CH₂)₂- | | >220 | β |
| I-1-a-11 | C₂H₅ | C₂H₅ | H | H | -(CH₂)₂-O-(CH₂)₂- | | >220 | - |
| I-1-a-12 | CH₃ | C₂H₅ | H | H | CH₃ | CH₃ | 109 | - |
| I-1-a-13 | CH₃ | C₂H₅ | H | H | -(CH₂)₂-CHCH₃-(CH₂)₂- | | 216 | β |
| I-1-a-14 | H | CH₃ | H | i-C₄H₉ | CH₃ | CH₃ | 160 | |
| I-1-a-15 | H | CH₃ | H | C₃H₇ | -(CH₂)₂-CHOCH₃-(CH₂)₂- | | 197 | β |
| I-1-a-16 | H | CH₃ | H | C₃H₇ | -(CH₂)₂-CHOC₂H₅-(CH₂)₂- | | 193 | β |
| I-1-a-17 | H | CH₃ | H | i-C₄H₉ | -(CH₂)₂-CHOCH₃-(CH₂)₂- | | 233 | β |
| I-1-a-18 | H | CH₃ | H | i-C₄H₉ | -(CH₂)₂-CHOC₂H₅-(CH₂)₂- | | 184 | β |
| I-1-a-19 | H | CH₃ | CH₃ | C₂H₅ | -(CH₂)₂-CHOCH₃-(CH₂)₂- | | 156 | β |
| I-1-a-20 | H | CH₃ | CH₃ | C₂H₅ | -(CH₂)₂-CHOC₂H₅-(CH₂)₂- | | 168 | β |
| I-1-a-21 | CH₃ | CH₃ | -C≡CH | H | -(CH₂)₂-CHOCH₃-(CH₂)₂- | | 127 | β |
| I-1-a-22 | H | CH₃ | C₂H₅ | H | -(CH₂)₂-CHCH₃-(CH₂)₂- | | 211 | β |
| I-1-a-23 | H | C₂H₅ | CH₃ | H | -(CH₂)₂-CHCH₃-(CH₂)₂- | | 210 | β |
| I-1-a-24 | H | C₂H₅ | CH₃ | H | -(CH₂)₂-O-(CH₂)₂- | | >220 | - |
| I-1-a-25 | H | C₂H₅ | CH₃ | H | CH₃ | CH₃ | 192 | - |
| I-1a-26 | H | CH₃ | H | C₂H₅ | -(CH₂)₂-CHOC₂H₅-(CH₂)₂- | | 181 | β |
| I-1a-27 | H | CH₃ | H | C₂H₅ | -(CH₂)₂-CHOCH₃-(CH₂)₂- | | 222 | β |
| I-1a-28 | CH₃ | CH₃ | CH=CH₂ | H | -(CH₂)₂-CHOCH₃-(CH₂)₂- | | 278 | β |
| I-1a-29 | CH₃ | CH₃ | C₂H₅ | H | -(CH₂)₂-CHOCH₃-(CH₂)₂- | | 268 | β |
| I-1a-30 | CH₃ | CH₃ | C₂H₅ | H | -(CH₂)₂-CHOCH₃-(CH₂)₂- | | 248 | α |

### Beispiel I-1-b-1

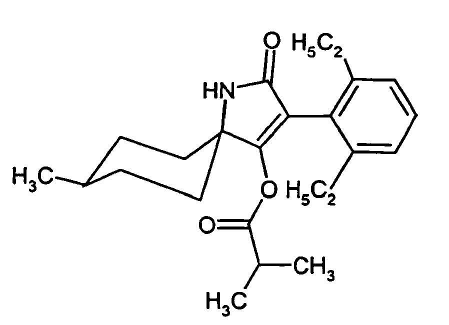

2,5 g der Verbindung I-1-a-10 in 50 ml wasserfreiem Essigsäureethylester werden mit 1,7 ml Triethylamin unter Rückfluß erhitzt und 1,3 ml Isobuttersäurechlorid in 5 ml wasserfreiem Essigsäureethylester wird hinzugetropft und unter dünnschichtchromatographischer Kontrolle wird unter Rückfluß gerührt. Das Lösungsmittel wird abdestilliert und der Rückstand in Methylenchlorid aufgenommen, mit 50 ml 0,5 N NaOH gewaschen, getrocknet und einrotiert. Der Rückstand wird anschließend aus Methyl-tert.-butylether (MTB-Ether)/n-Hexan umkristallisiert.
Ausbeute: 2 g (≙ 65 % der Theorie), Fp. 209°C

In Analogie zu Beispiel (I-1-b-1) und gemäß den allgemeinen Angaben zur Herstellung erhält man folgende Verbindungen der Formel (I-1-b)

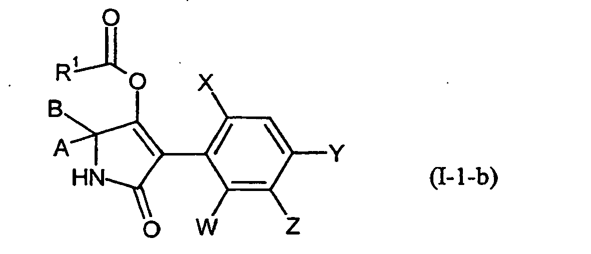

| Bsp.-Nr. | W | X | Y | Z | A | B | R¹ | Fp.°C | Isomer |
|---|---|---|---|---|---|---|---|---|---|
| I-1-b-2 | CH₃ | C₂H₅ | H | H | CH₃ | CH₃ | i-C₃H₇ | 145 | - |
| I-1-b-3 | CH₃ | C₂H₅ | H | H | -(CH₂)₂-CHCH₃-(CH₂)₂- | | H₅C₂-0-CH₂- | 135 | β |
| I-1-b-4 | CH₃ | C₂H₅ | H | H | CH₃ | CH₃ | H₅C₂-O-CH₂- | 76 | - |
| I-1-b-5 | H | CH₃ | C₂H₅ | H | -(CH₂)₂-CHCH₃-(CH₂)₂- | | i-C₃H₇ | 201 | β |
| I-1-b-6 | H | CH₃ | C₂H₅ | H | -(CH₂)₂-CHCH₃-(CH₂)₂- | | i-C₄H₉ | 215 | β |
| I-1-b-7 | H | C₂H₅ | CH₃ | H | -(CH₂)₂-O-(CH₂)₂- | | i-C₃H₇ | 190 | - |

### Beispiel I-1-c-1

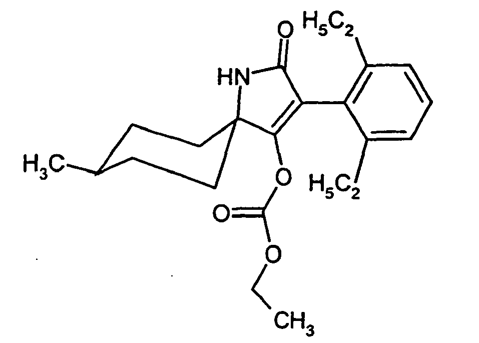

2,51 g der Verbindung der Formel I-1-a-10 und 1,2 ml Triethylamin werden vorgelegt. Bei 0 bis 10°C werden 0,8 ml Chlorameisensäureethylester in 5 ml wasserfreiem Dichlormethan zugetropft und man rührt bei Raumtemperatur unter dünnschichtchromatographischer Kontrolle. Mit 0,5 N NaOH wird gewaschen, getrocknet und das Lösungsmittel abdestilliert. Der Rückstand wird aus Methyl-tert.-butylether/n-Hexan umkristallisiert.
Ausbeute: 1,1 g (≙ 30 % der Theorie), Fp. 178°C

In Analogie zu Beispiel (I-1-c-1) und gemäß den allgemeinen Angaben zur Herstellung erhält man folgende Verbindungen der Formel (I-1-c)

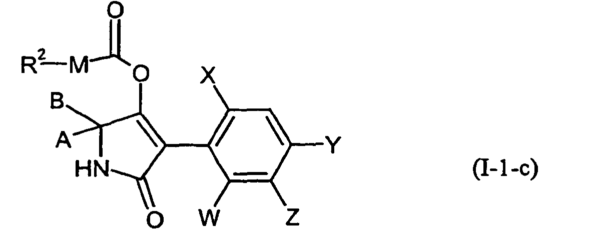

| Bsp.- Nr. | W | X | Y | Z | A | B | M | R² | Fp. °C | Isomer |
|---|---|---|---|---|---|---|---|---|---|---|
| I-1-c-2 | C₂H₅ | C₂H₅ | H | H | -(CH₂)₂-O-(CH₂)₂- | | O | C₂H₅ | 194 | β |
| I-1-c-3 | CH₃ | C₂H₅ | H | H | CH₃ | CH₃ | O | C₂H₅ | 119 | - |
| I-1-c-4 | CH₃ | CH₃ | -C≡CH | H | -(CH₂)₂-CHOCH₃-(CH₂)₂- | | O | C₂H₅ | 171 | β |
| I-1-c-5 | H | CH₃ | C₂H₅ | H | -(CH₂)₂-CHCH₃-(CH₂)₂- | | O | i-C₄H₉ | 155 | β |
| I-1-c-6 | H | C₂H₅ | CH₃ | H | -(CH₂)₂-O-(CH₂)₂- | | O | C₂H₅ | 215 | - |
| I-1-c-7 | H | CH₃ | CH₃ | C₂H₅ | -(CH₂)₂-CHOCH₃-(CH₂)₂ | | O | C₂H₅ | 137 | β |
| I-1-c-8 | H | CH₃ | CH₃ | C₂H₅ | -(CH₂)₂-CHOC₂H₅-(CH₂)₂ | | O | C₂H₅ | 168 | β |

### Beispiel II-1

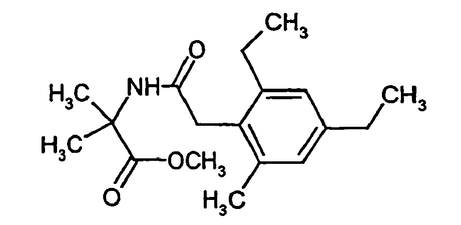

7,5 g 2,4-Diethyl-6-methyl-phenylessigsäure und 9,2 ml Thionylchlorid werden bei 80°C gerührt, bis die Gasentwicklung beendet ist. Das überschüssige Thionylchlorid wird abdestilliert und der Rückstand in 30 ml trockenem THF aufgenommen. Diese Lösung wird bei 0 bis 10°C zu 12,3 g 2-Amino-2-methylpropansäuremethylester in 320 ml trockenem THF, das mit 24,6 ml Triethylamin versetzt ist, getropft und 1 h bei Raumtemperatur gerührt. Diese Lösung wird dann einrotiert, in Methylenchlorid und 1 N HCl aufgenommen, extrahiert, getrocknet und einrotiert. Der Rückstand wird aus MTB-Ether/n-Hexan umkristallisiert.
Ausbeute: 8,07 g (≙ 66 % der Theorie), Fp. 120 bis 122°C

### Beispiel II-11

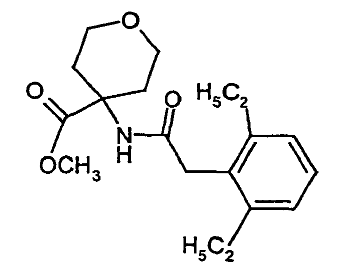

Zu 16,9 g konz. Schwefelsäure gibt man 10,3 g der Verbindung gemäß Beispiel XXVII-1 als Suspension in 110 ml Methylenchlorid bei einer Innentemperatur von 30 bis 40°C und rührt 2 h. 23 ml trockenes Methanol werden zugetropft und man rührt bei 40 bis 70°C 6 h. Die Lösung wird auf 0,18 kg Eis gegeben, mit Methylenchlorid extrahiert und mit einer NaHCO₃-Lösun gewaschen. Man trocknet, rotiert ein und kristallisiert den Rückstand aus MTB-Ether/n-Hexan um.
Ausbeute: 8,7 g (76 % der Theorie), Fp. 137°C

In Analogie zu Beispiel (II-1) und gemäß den allgemeinen Angaben zur Herstellung wurden folgende Verbindungen der Formel (II) erhalten

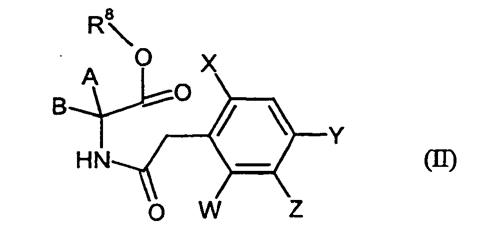

| Bsp.- Nr. | W | X | Y | Z | A | B | R⁸ | Fp.°C | Isomer |
|---|---|---|---|---|---|---|---|---|---|
| II-2 | CH₃ | C₂H₅ | C₂H₅ | H | -(CH₂)₂-CHCH₃-(CH₂)₂- | | CH₃ | 169 | β |
| II-3 | CH₃ | C₂H₅ | C₂H₅ | H | -(CH₂)₂-CHOCH₃-(CH₂)₂- | | CH₃ | 94 | β |
| II-4 | C₂H₅ | C₂H₅ | C₂H₅ | H | CH₃ | CH₃ | CH₃ | 109 | - |
| II-5 | C₂H₅ | C₂H₅ | C₂H₅ | H | -(CH₂)₂-CHCH₃-(CH₂)₂- | | CH₃ | 141 | β |
| II-6 | C₂H₅ | C₂H₅ | C₂H₅ | H | -(CH₂)₂-CHOCH₃-(CH₂)₂- | | CH₃ | 165 | β |
| II-7 | i-C₃H₇ | i-C₃H₇ | i-C₃H₇ | H | -(CH₂)₂-CHOCH₃-(CH₂)₂- | | CH₃ | 112 | β |
| II-8 | CH₃ | C₂H₅ | CH₃ | H | CH₃ | CH₃ | CH₃ | 126 | - |
| II-9 | CH₃ | C₂H₅ | CH₃ | H | -(CH₂)₂-CHCH₃-(CH₂)₂- | | CH₃ | 101 | β |
| II-10 | C₂H₅ | C₂H₅ | H | H | -(CH₂)₂-CHCH₃-(CH₂)₂- | | CH₃ | 108 | β |
| II-11 | C₂H₅ | C₂H₅ | H | H | -(CH₂)₂-O-(CH₂)₂- | | CH₃ | 137 | - |
| II-12 | CH₃ | C₂H₅ | H | H | CH₃ | CH₃ | CH₃ | 102 | - |
| II-13 | CH₃ | C₂H₅ | H | H | -(CH₂)₂-CHCH₃-(CH₂)₂- | | CH₃ | 169 | β |
| II-14 | H | CH₃ | H | i-C₄H₉ | CH₃ | CH₃ | CH₃ | 93 | - |
| II-15 | H | CH₃ | H | C₃H₇ | -(CH₂)₂-CHOCH₃-(CH₂)₂- | | CH₃ | 112 | β |
| II-16 | H | CH₃ | H | C₃H₇ | -(CH₂)₂-CHOC₂H₅-(CH₂)₂- | | CH₃ | 71 | β |
| II-17 | H | CH₃ | H | i-C₄H₉ | -(CH₂)₂-CHOCH₃-(CH₂)₂- | | CH₃ | 69 | β |
| II-18 | H | CH₃ | H | i-C₄H₉ | -(CH₂)₂-CHOC₂H₅-(CH₂)₂- | | CH₃ | 61 | β |
| II-19 | H | CH₃ | CH₃ | C₂H₅ | -(CH₂)₂-CHOCH₃-(CH₂)₂- | | CH₃ | 151 | β |
| II-20 | H | CH₃ | CH₃ | C₂H₅ | -(CH₂)₂-CHOC₂H₅-(CH₂)₂- | | CH₃ | 123 | β |
| II-21 | CH₃ | CH₃ | -C≡CH | H | -(CH₂)₂-CHOCH₃-(CH₂)₂- | | CH₃ | 141 | β |
| II-22 | H | CH₃ | C₂H₅ | H | -(CH₂)₂-CHCH₃-(CH₂)₂- | | CH₃ | 98 | β |
| II-23 | H | C₂H₅ | CH₃ | H | -(CH₂)₂-CHCH₃-(CH₂)₂- | | CH₃ | 149 | β |
| II-24 | H | C₂H₅ | CH₃ | H | -(CH₂)₂-O-(CH₂)₂- | | CH₃ | 164 | - |
| II-25 | H | C₂H₅ | CH₃ | H | CH₃ | CH₃ | CH₃ | 141 | - |
| II-26 | H | CH₃ | H | C₂H₅ | -(CH₂)₂-CHOC₂H₅-(CH₂)₂- | | CH₃ | 103 | β |
| II-27 | H | CH₃ | H | C₂H₅ | -(CH₂)₂-CHOCH₃-(CH₂)₂ | | CH₃ | Öl | β |
| II-28 | CH₃ | CH₃ | CH=CH₂ | H | -(CH₂)₂-CHOCH₃-(CH₂)₂ | | CH₃ | 234 | β |
| II-29 | CH3 | CH₃ | C₂H₅ | H | -(CH₂)₂-CHOCH₃-(CH₂)₂ | | CH₃ | 140 | β |

### Beispiel XXVII-1

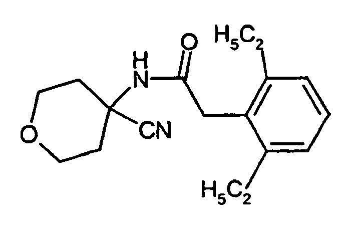

7,68 g 2,4-Diethyl-6-methyl-phenylessigsäure und 9,1 ml Thionylchlorid werden bei 80°C gerührt, bis die Gasentwicklung beendet ist. Das überschüssige Thionylchlorid wird abdestilliert und der Rückstand in 40 ml trockenem Toluol aufgenommen. Diese Lösung wird bei 0 bis 10°C zu 9 g 4-Amino-4-cyano-tetrahydropyran in 80 ml trockenem THF, das mit 6,2 ml Triethylamin versetzt ist, getropft und 1 h bei Raumtemperatur gerührt. Die Lösung wird dann einrotiert, in 1 N HCl in Methylenchlorid aufgenommen, getrocknet und einrotiert. Der Rückstand wird aus MTB-Ether/n-Hexan umkristallisiert.
Ausbeute: 10,3 g (≙ 85 % der Theorie), Fp. 155°C

In Analogie zu Beispiel XXVII-1 erhält man Beispiel XXVII-2 vom Fp. 142°C.

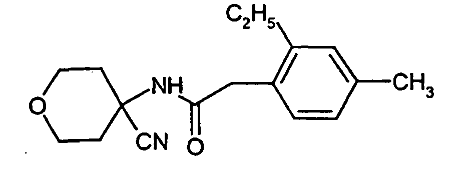

### Beispiel I-2-a-1

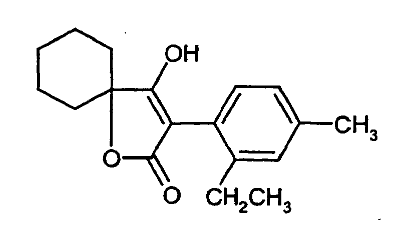

Zu 8,4 g Kalium-tert.-butylat in 50 ml wasserfreiem DMF tropft man bei 0 bis 10°C 16,6 g der Verbindung gemäß Beispiel III-1 gelöst in 50 ml wasserfreiem DMF und rührt 8 h bei Raumtemperatur. Nach Beendigung der Reaktion tropft man unter Eiskühlung 1000 ml 1 N HCl zu und rührt 30 min. Der Niederschlag wird abfiltriert, mit Wasser gewaschen und im Vakuum getrocknet.
Ausbeute: 11,5 g (≙ 80 % der Theorie), Fp. 135°C

In Analogie zu Beispiel (I-2-a-1) und gemäß den allgemeinen Angaben zur Herstellung erhält man folgende Verbindungen der Formel (I-2-a)

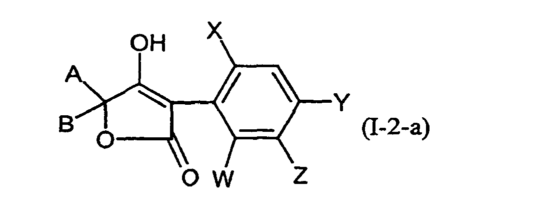

| Bsp.-Nr. | W | X | Y | Z | A | B | Fp.°C |
|---|---|---|---|---|---|---|---|
| I-2-a-2 | C₂H₅ | C₂H₅ | H | H | -(CH₂)₂-CHOCH₃-(CH₂)₂- | | Öl |
| I-2-a-3 | CH₃ | C₂H₅ | CH₃ | H | -(CH₂)₅- | | 223-225 |
| I-2-a-4 | CH₃ | C₂H₅ | CH₃ | H | -(CH₂)₂-CHOCH₃-(CH₂)₂- | | 175-178 |
| I-2-a-5 | CH₃ | C₂H₅ | C₂H₅ | H | -(CH₂)₂-CHOCH₃-(CH₂)₂- | | Öl |

### Beispiele I-2-b-1

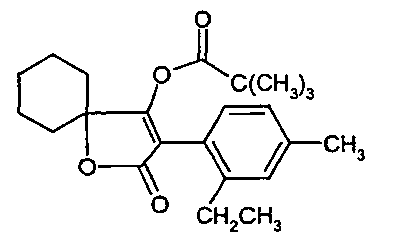

Zu 2,86 g der Verbindung gemäß Beispiel I-2-a-1 gelöst in 40 ml trockenem Methylenchlorid (CH₂Cl₂) gibt man 2,08 ml Triethylamin. Bei 0 - 10°C gibt man 1,5 g Pivaloylchlorid in 10 ml CH₂Cl₂ und rührt 20 h bei Raumtemperatur.

Die Reaktionslösung wird zunächst mit 10 %iger Citronensäure und dann mit 1 N NaOH gewaschen, getrocknet und einrotiert und der Rückstand mit Petrolether verrührt.
Ausbeute: 2,2 g (≙ 60 % d.Th.), Fp. 110-112°C.

### Beispiel I-2-b-2

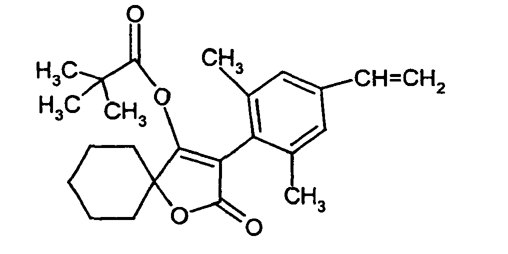

1,2 g (2,76 mmol) der Verbindung gemäß Beispiel I-2-b-2 aus WO 97/02243 werden in 20 ml Toluol vorgelegt, 3,5 g (11 mmol) Tributyl-vinylzinn, 133 mg (0,11 mmol) Pd(PPh₃)₄ und 2 Kristalle 2,6-Di-t-butyl-4-methylkresol zugesetzt, die Mischung über Nacht am Rückfluß gekocht und anschließend einrotiert.

Zur Aufreinigung wird das Rohgemisch über Kieselgel chromatographiert, wobei zuerst mit Cyclohexan überschüssige Zinnverbindungen eluiert werden und anschließend durch Wechsel des Laufmittels auf Cyclohexan/Essigester (2:1) das Produkt eluiert wird. Eine weitere Aufreinigung wurde durch Verreiben des Rohproduktes mit Petrolether erreicht.
Ausbeute: 0,46 g (44 % d.Th.) farblose Kristalle vom Fp. 152 - 155°C.

In Analogie zu den Beispielen (I-2-b-1) und (I-2-b-2) und gemäß den allgemeinen Angaben zur Herstellung erhält man folgende Verbindungen der Formel (I-2-b)

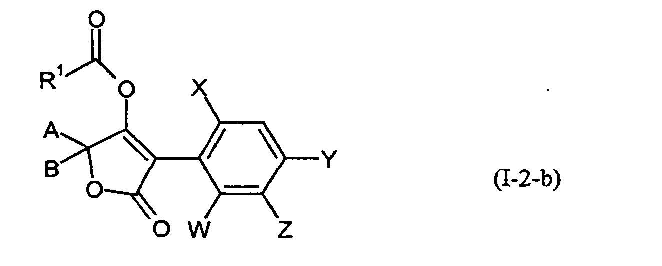

| Bsp.-Nr. | W | X | Y | Z | A B | R¹ | Fp.°C |
|---|---|---|---|---|---|---|---|
| I-2-b-3 | H | C₂H₅ | CH₃ | H | -(CH₂)₅- | t-C₄H₉-CH₂ | 162-164 |
| I-2-b-4 | C₂H₅ | C₂H₅ | H | H | -(CH₂)₂-CHOCH₃-(CH₂)₂- | t-C₄H₉ | Öl |
| I-2-b-5 | CH₃ | CH₃ | CH=CH₂ | H | -(CH₂)₂-CHOCH₃-(CH₂)₂- | t-C₄H₉ | 158-160 |
| I-2-b-6 | CH₃ | CH₃ | CH=CH₂ | H | -(CH₂)₂-CHCH₃-(CH₂)₂- | t-C₄H₉ | 143-145 |
| I-2-b-7 | CH₃ | CH₃ | CH=CH₂ | H | 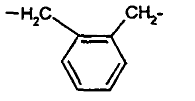 | t-C₄H₉ | 155-157 |
| I-2-b-8 | CH₃ | CH=CH₂ | CH₃ | H | -(CH₂)₂-CHOCH₃-(CH₂)₂- | t-C₄H₉ | 145 |
| I-2-b-9 | CH₃ | C₂H₅ | CH₃ | H | -(CH₂)₅- | t-C₄H₉ | 96-98 |
| I-2-b-10 | CH₃ | C₂H₅ | CH₃ | H | -(CH₂)₂-CHOCH₃-(CH₂)₂- | t-C₄H₉ | 90-93 |
| I-2-b-11 | C₂H₅ | C₂H₅ | C₂H₅ | H | -(CH₂)₂-CHOCH₃-(CH₂)₂- | H₅C₂-CHCH₃- | Öl |
| I-2-b-12 | H | CH₃ | CH=CH₂ | CH₃ | -(CH₂)₂-CHOCH₃-(CH₂)₂- | s-C₄H₉ | Öl |
| I-2-b-13 | H | CH=CH₂ | CH₃ | CH₃ | -(CH₂)₂-CHOCH₃-(CH₂)₂- | i-C₃H₇ | Öl |

### Beispiel III-1

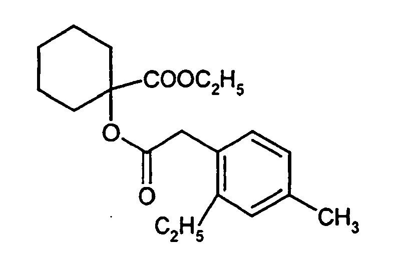

8,9 g 2-Ethyl-4-methyl-phenylessigsäure in 50 ml trockenem Toluol und 7,3 ml Thionylchlorid werden bei 80°C gerührt bis die Gasentwicklung beendet ist. Das überschüssige Thionylchlorid wird abdestilliert und der Rückstand in 30 ml trockenem Toluol aufgenommen. Diese Lösung wird bei 0-10°C zu 8,6 g 1-Hydroxycyclohexancarbonsäureethylester in 50 ml trockenem Toluol getropft und 8 h unter Rückfluß gerührt. Die Lösung wird dann einrotiert.
Ausbeute: 16,6 g (≙ 99 % der Theorie)

Der Rückstand wird ohne weitere Reinigung in die Kondensation zum Beispiel I-2-a-1 eingesetzt.

### Beispiel I-4-a-1

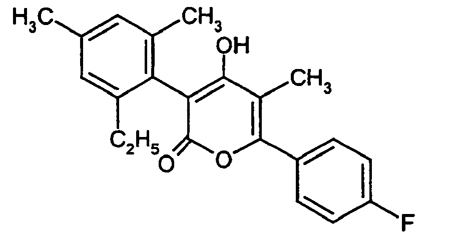

2,4 g 2-Ethyl-4,6-dimethyl-2-phenylchlorcarbonylketen werden in 30 ml abs. Xylol vorgelegt und 1,5 g 4-Fluorpropiophenon in 20 ml abs. Xylol zugetropft. Man erhitzt 8 Stunden unter Rückfluss. Man wäscht die Xylollösung mit Wasser, trocknet über Natriumsulfat und dampft im Vakuum ein. Die chromatographische Reinigung erfolgt an Kieselgel mit Toluol/Ethanol 20:1 als Elutionsmittel.
Ausbeute: 1 g (≙ 28 % der Theorie) vom Fp. 161-162°C.

### Beispiel I-6-a-1

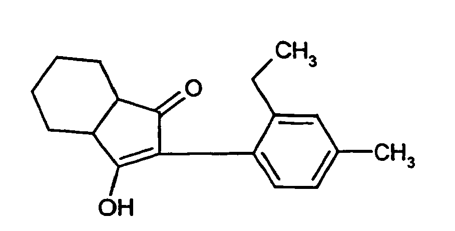

5,3 g der Verbindung gemäß Beispiel VIII-1 werden in 50 ml trockenem DMF vorgelegt, mit 2,95 g Kalium-tert.-Butylat versetzt und 1 h auf 60°C erwärmt.

Die Reaktionslösung wird mit 100 ml 1 N HCl versetzt, mit CH₂Cl₂ extrahiert, die organische Phase wird getrocknet und eingeengt. Der Rückstand wird säulenchromatographisch (Cyclohexan: Essigsäureethylester, 5:1) gereinigt.
Ausbeute: 2,35 g (49 % d.Th.), Fp. 148°C.

### Beispiel I-6-b-1

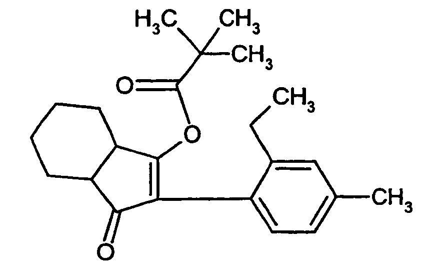

1 g der Verbindung gemäß Beispiel I-6-a-1 werden in 20 ml trockenem Methylenchlorid vorgelegt, mit 0,77 ml Triethylamin versetzt. 0,68 ml Pivaloylchlorid werden in 1 ml Methylenchlorid gelöst und unter Eiskühlung zugetropft und 2 h bei Raumtemperatur gerührt.

Die Reaktionslösung wird zweimal mit 10 % Citronensäurelösung extrahiert und die organische Phase zweimal mit 1 N NaOH gewaschen, getrocknet und eingeengt.
Ausbeute: 1,2 g (92 % d.Th.) Öl.
¹H-NMR (500 MHz, CDCl₃) : δ = 1,1 (s, 9H, -C(CH₃), 2,31 (s, 3H, Ar-CH₃); 2,45 (q, 2H, Ar-CH₂-CH₃) ppm

### Beispiel VIII-1

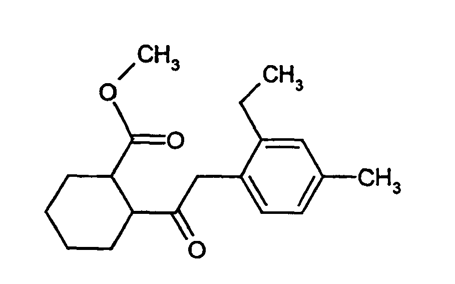

22,8 g Rohprodukt aus Beispiel XXXIV-1 werden in 200 ml trockenem Aceton vorgelegt mit 10,9 g Kaliumcarbonat versetzt und 33,6 g (14,75 ml) Methyliodid zugetropft. Man rührt 16 h unter Rückfluss.

Das Lösungsmittel wird abdestilliert und der Rückstand säulenchromatographisch gereinigt (Methylenchlorid/Petrolether: 8:1).
Ausbeute: 3,5 g (30 % d.Th.), Öl

Das Produkt wird direkt in die Cyclisierung zu Beispiel I-6-a-1 eingesetzt.

### Beispiel XXXIV-1

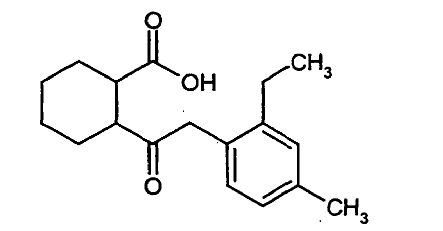

11,2 g Cyclohexandicarbonsäure-monomethylester werden mit 5,3 ml Thionylchlorid und einem Tropfen DMF in 50 ml trockenem Toluol auf 100°C erwärmt, bis die Gasentwicklung beendet ist. Das Lösungsmittel wird eingeengt.

Zu einer Lösung von 50 ml LDA-Lösung in 100 ml trockenem THF wird eine Lösung von 17,3 g 2-Ethyl-4-methyl-phenylessigsäure-methylester in 20 ml trockenem THF bei -15°C zugetropft und 30 min bei dieser Temperatur nachgerührt.

Dann wird bei -15°C eine Lösung des oben beschriebenen frisch hergestellten Säurechlorides in 15 ml trockenem THF zugetropft.

Der Ansatz wird eine Stunde bei Raumtemperatur gerührt, dann werden 150 ml Wasser und 40 g Ammoniumchlorid zugegeben. Das Zwischenprodukt wird mit Ether extrahiert, die Lösung eingeengt. Der Rückstand wird mit 100 g KOH und 330 ml Wasser zwei Tage unter Rückfluss gekocht.
Ausbeute: 23,70 g (91 % d.Th.), Öl

### Beispiel XXV-1

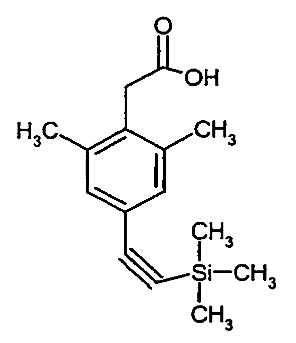

Zu 26 g der Verbindung gemäß Beispiel XXX-1 in 120 ml THF wird bei Raumtemperatur 4,7 g Lithiumhydroxid in 120 ml Wasser gelöst zugetropft und 8 h bei Raumtemperatur gerührt.

Die Reaktionslösung wird einrotiert mit Wasser versetzt und mit Methyl-tert.-butylether extrahiert.

Die wässrige Phase wird mit konzentrierter Salzsäure auf pH 2 gebracht und der Niederschlag abgesaugt und getrocknet.
Ausbeute: 14 g (59 % d.Th.), Fp.: 156,3°C

### Beispiel XXX-1

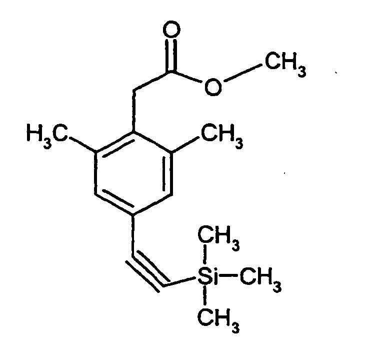

Zu einer Lösung von 7,32 g der Verbindung 2,6-Diemthyl-4-brom-phenylessigsäuremethylester (gemäß Beispiel XXVI-1 aus WO 97/02243) in 70 ml Triethylamin werden unter Rühren und Argonatmosphäre bei Raumtemperatur 0,27 g Kupfer(I)-iodid, 0,745 g Triphenylphosphin, 1 g Bis(triphenylphosphin)palladiumdichlorid zugegeben und anschließend 19,7 ml Trimethylsilyl-acetylen zugetropft.

Der Umsatz wird mittels Gaschromtographie verfolgt.

Die Reinigung erfolgt säulenchromatographisch an Kieselgel mit Petrolether/Essigsäureethylester, 20:1 als Elutionsmittel.
Ausbeute: 6 g (73 % d.Th.)

### Beispiel XXII-1

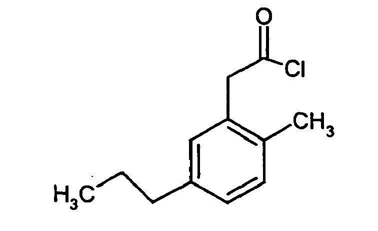

39 g der Verbindung gemäß Beispiel XXV-2 werden in 300 ml Thionylchlorid bei 50°C gerührt bis die Gasentwicklung beendet ist.

Das überschüssige Thionylchlorid wird abdestilliert, der Rückstand in 30 ml trockenem Toluol aufgenommen und abdestilliert.
Ausbeute: 37 g (87 % d.Th.), Kp.: 90-92°C (0,05 mbar)

### Beispiel XXV-2

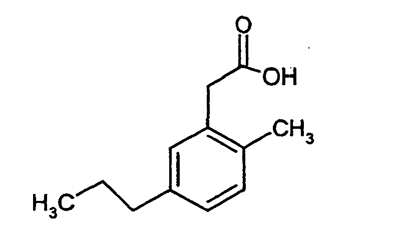

Zu 50 g der Verbindung gemäß Beispiel XXX-2 gibt man 60 ml Ethanol, 30 ml Wasser, 25 g Kaliumhydroxid und erhitzt 5 Stunden unter Rückfluss.

Nach beendeter Reaktion wird das Lösungsmittel abdestilliert, der Rückstand in Wasser gelöst und der pH-Wert mit konz. Salzsäure sauergestellt. Der Niederschlag wird abgesaugt, gewaschen und getrocknet.
Ausbeute: 41 g (93 % d.Th.)

### Beispiel XXX-2 (Verfahren (P))

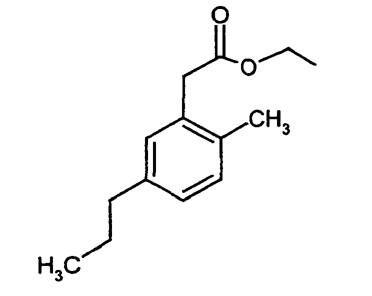

60 g der Verbindung gemäß Bsp. XLII-1 werden in 600 ml Ethanol gelöst, mit 50 ml konzentrierter Salzsäure versetzt und 5 g 10 % Pd/C zugegeben.

Bei 120°C und 150 bar wird Wasserstoff auf das Reaktionsgemisch gedrückt.

Nach beendeter Reaktion wird filtriert, das Lösungsmittel abdestilliert, der Rückstand in 300 ml Methylenchlorid gelöst und mit 300 ml Wasser gewaschen, getrocknet und eingeengt.
Ausbeute: 51 g (91 % d.Th.)

### Beispiel XLII-1 (Verfahren (P))

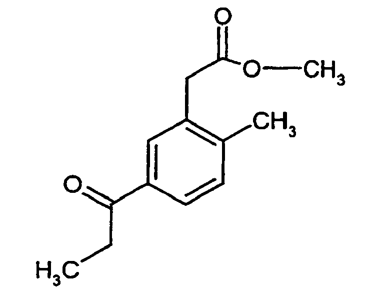

200 g ml Schwefelkohlenstoff und 86,7 g Aluminiumchlorid werden vorgelegt. Bei 0°C tropft man 50 g 2-Methylphenylessigsäure-methylester und 28,2 g Propionsäurechlorid zu. Die Lösung wird vier Stunden unter Rückfluss gerührt.

Die Lösung gibt man anschließend auf 1 kg Eiswasser und extrahiert mit 500 ml Methylenchlorid.

Die organische Phase wird mit 10 %iger Salzsäure und anschließend mit Sodalösung gewaschen, getrocknet und eingeengt.
Ausbeute: 60 g (91 % d.Th.)

### Beispiel XXV-3

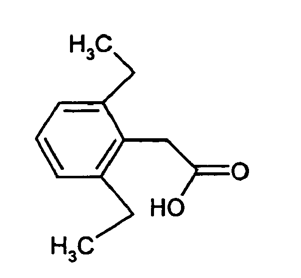

Zu 30 g der Verbindung gemäß Beispiel XXX-3 in 32 ml Methanol und 16 ml Wasser gibt man 12,2 g Kaliumhydroxid und rührt 5 h unter Rückfluss.

Die Lösung wird eingeengt und der Rückstand in Wasser aufgenommen, mit Essigsäureethylester gewaschen und die wässrige Phase mit konz. HCl auf pH 1 eingestellt. Der Niederschlag wird abgesaugt, gewaschen und getrocknet.
Ausbeute: 25 g (99 % d.Th.), Fp. 55-56°C

### Beispiel XXX-3 (Verfahren Q)

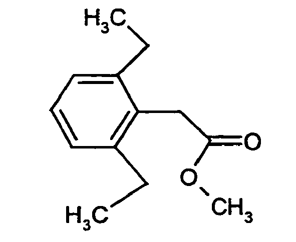

20,8 g der Verbindung 2,6-Diethyl-4-brom-phenylessigsäuremethylester (gemäß Beispiel XXVI-6 aus WO 97/02243) werden in 100 ml Methanol gelöst. Man gibt 7,2 g Natriumacetat und 2 g Palladiumhydroxid hinzu. Anschließend wird mit Wasserstoff unter Druck hydriert.

Nach beendeter Reaktion wird die Lösung filtriert und eingeengt. Der Rückstand wird in Methylenchlorid aufgenommen, mit Wasser gewaschen, getrocknet und eingeengt.
Ausbeute: 12 g (77 % d.Th.)

### Anwendungsbeispiele

### Beispiel A

| Myzus-Test | |
|---|---|
| Lösungsmittel: | 7 Gewichtsteile Dimethylformamid |
| Emulgator: | 1 Gewichtsteil Alkylarylpolyglykolether |

Zur Herstelung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen die Verbindungen der Herstellungsbeispiele I-2-a-4, I-2-b-5, I-2-b-6, I-1-a-6, I-1-c-4, I-4-a-1 bei einer beispielhaften Wirkstoffkonzentration von 1000 ppm eine Abtötung von 100 % nach 6 Tagen.

### Beispiel B

| Nephotettix-Test | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Blätter noch feucht sind.

Nach der gewünschten Zeit wird die Abtötung in % bestimmt. Dabei bedeutet 100 %, dass alle Zikaden abgetötet wurden; 0 % bedeutet, dass keine Zirkaden abgetötet wurden.

Bei diesem Test zeigen die Verbindungen der Herstellungsbeispiele I-2-b-9, I-2-a-4, I-1-a-9, I-1-a-8, I-1-a-2, I-1-a-1, I-1-a-3, I-4-a-1, bei einer beispielhaften Wirkstoffkonzentration von 1000 ppm eine Abtötung von 100 % nach 6 Tagen.

### Beispiel C

| Phaedon-Larven-Test | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerettichkäfers (Phaedon cochleariae) besetzt, solange die Blätter noch feucht sind.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden.

Bei diesem Test zeigen die Verbindungen der Herstellungsbeispiele I-2-a-3, I-2-b-8, I-2-b-6, I-1-a-2, I-1-a-3, I-1-a-21, I-4-a-1 bei einer beispielhaften Wirkstoffkonzentration von 1000 ppm eine Abtötung von 100 % nach 7 Tagen.

### Beispiel D

| Plutella-Test | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet werden.

Bei diesem Test zeigen die Verbindungen der Herstellungsbeispiele I-1-a-8, I-4-a-1 bei einer beispielhaften Wirkstoffkonzentration von 1000 ppm eine Abtötung von 100 % nach 7 Tagen.

### Beispiel E

| Spodoptera frugiperda-Test | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen die Verbindungen der Herstellungsbeispiele I-2-a-3 bei einer beispielhaften Wirkstoffkonzentration von 1000 ppm eine Abtötung von 100 % nach 7 Tagen.

### Beispiel F

| Tetranychus-Test (OP-resistent/Tauchbehandlung) | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen die Verbindungen der Herstellungsbeispiele I-2-a-4, I-2-b-10, I-2-b-11; I-2-b-8, I-2-b-2, I-1-a-3, I-1-a-21, I-1-c-4, I-4-a-1 bei einer beispielhaften Wirkstoffkonzentration von 1000 ppm eine Abtötung von 100 % nach 7 Tagen.

### Beispiel G

| Bemisia-Test | |
|---|---|
| Lösungsmittel: | 7,5 Gewichtsteile Dimethylformamid |
| Emulgator: | 2,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschten Konzentrationen.

Baumwollpflanzen (Gossypium hirsutum), die von Eiern, Larven und Puparien der Weißen Fliege Bemisia tabaci befallen sind, werden in eine Wirkstoffzubereitung der gewünschten Konzentration getaucht.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

Bei diesem Test zeigen die Verbindungen der Herstellungsbeispiele I-2-b-9, I-2-a-4, I-2-b-10 bei einer beispielhaften Wirkstoffkonzentration von 1000 ppm eine Abtötung von 100 % nach 10 Tagen.

### Beispiel H

| Post-emergence-Test | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, dass die jeweils gewünschten Wirkstoffmengen pro Flächeneinheit ausgebracht werden. Die Konzentrationen der Spritzbrühe wird so gewählt, dass in 10001 Wasser/ha die jeweils gewünschten Wirkstoffmenge ausgebracht werden.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

### Beispiel I

| Pre-emergence-Test | |
|---|---|
| Lösungsmittel: | 5 Gewichtsteile Aceton |
| Emulgator: | 1 Gewichtsteil Alkyarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Bodne ausgesät. Nach ca. 24 Stunden wird der Boden mit der Wirkstoffzubereitung bespritzt so, dass die jeweils gewünschten Wirkstoffmengen pro Flächeneinheit ausgebracht werden. Die Konzentration der Spritzbrühe wird so gewählt, dass in 10001 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrollen.

Es bedeuten:
0 % = keine Wirkung (wie unbehandelte Kontrolle)
100 % = totale Vernichtung

| Post-emergence | g ai/ha | Weizen | Baumwolle | Alopecurus | | Avena fatua | Lolium | | Sorghum |
|---|---|---|---|---|---|---|---|---|---|
| Bsp.I-2-a-4 | 60 | 0 | 0 | 95 | | 80 | 95 | | 80 |

| Post-emergence | g ai/ha | Zuckerrüben | Alopecurus | Avena fatua | Digitaria | Lolium | Setaria | Ipomoea | Polygonum |
|---|---|---|---|---|---|---|---|---|---|
| Bsp. I-1-a-21 | 125 | 0 | 100 | 90 | 100 | 95 | 99 | 70 | - |
| Bsp.I-1-c-4 | 60 | 10 | 90 | 90 | 95 | - | 100 | - | 70 |

| Post-emergence | g ai/ha | Soja | Digitaria | Lolium | Setaria | Sorghum | Galium |
|---|---|---|---|---|---|---|---|
| Bsp.I-1-a-5 | 125 | 10 | 90 | - | 100 | 95 | 80 |
| Bsp.I-1-a-3 | 125 | - | 80 | 70 | 90 | - | 80 |

| Post-emergence | g ai/ha | Zuckerrüben | Raps | Digitaria | Lolium | Setaria | Sorghum | Galium |
|---|---|---|---|---|---|---|---|---|
| Bsp.I-1-a-2 | 125 | 0 | 0 | 80 | 70 | 95 | 80 | 80 |

| Post-emergence | g ai/ha | Zuckerrüben | Baumwolle | Alopecurus | Avena fatua | Digitaria | Setaria |
|---|---|---|---|---|---|---|---|
| Bsp. I-1-a-9 | 15 | 10 | 0 | 100 | 95 | 95 | 100 |

| Post-emergence | g ai/ha | Zuckerrüben | Baumwolle | Digitaria | Echinochloa | Setaria | Sorghum |
|---|---|---|---|---|---|---|---|
| Bsp. I-1-a-8 | 60 | 10 | 0 | 95 | 100 | 100 | 95 |

| Post-emergence | g ai/ha | Digitaria | Lolium | Setaria | Sorghum |
|---|---|---|---|---|---|
| Bsp.I-1-a-6 | 125 | 95 | 80 | 95 | 100 |

| Post-emergence | g ai/ha | Weizen | Soja | Baumwolle | Digitaria | Lolium | Setaria |
|---|---|---|---|---|---|---|---|
| Bsp.I-2-b-12 | 125 | - | 0 | 0 | 95 | 90 | 95 |
| Bsp.I-2-b-13 | 125 | 10 | 0 | 0 | 80 | 70 | 95 |

| Post-emergence | g ai/ha | Weizen | Zuckerrüben | Baumwolle | Digitaria | Echinochloa | Setaria | Sorghum |
|---|---|---|---|---|---|---|---|---|
| Bsp.I-4-a-1 | 125 | 20 | 0 | 0 | 100 | 90 | 95 | 90 |

| Post-emergence | g ai/ha | Weizen | Lolium | Cassia | Solanum | Viola |
|---|---|---|---|---|---|---|
| Bsp. I-2-b-10 | 125 | 10 | 80 | 95 | 80 | 90 |

| Pre-emergence | g ai/ha | Weizen | Mais | Zuckerrüben | Soja | Alopecurus | Avena fatua | Bromus | Lolium | Setaria |
|---|---|---|---|---|---|---|---|---|---|---|
| Bsp.I-2-a-4 | 125 | - | 5 | 0 | 0 | 100 | 80 | 70 | 100 | 100 |
| Bsp.I-1-a-8 | 60 | 0 | 10 | 0 | 0 | 100 | 90 | 100 | 100 | 100 |

| Pre-emergence | g ai/ha | Alopecurus | Avena fatua | Digitaria | Echinochloa | Lolium | Setaria |
|---|---|---|---|---|---|---|---|
| Bsp. I-1-a-3 | 125 | 100 | 95 | 100 | 100 | 100 | 100 |

| Pre-emergence | g ai/ha | Alopecurus | Avena fatua | Echinochloa |
|---|---|---|---|---|
| Bsp.I-1-a-6 | 60 | 90 | 80 | 90 |

| Pre-emergence | g ai/ha | Zuckerrüben | Soja | Alopecurus | Digitaria | Lolium | Setaria | Veronica |
|---|---|---|---|---|---|---|---|---|
| Bsp. I-4-a-1 | 125 | 0 | 0 | 100 | 100 | 100 | 100 | 95 |
| Bsp. I-1-a-21 | 125 | 0 | 0 | 100 | 100 | 100 | 100 | 100 |
| Bsp.I-1-a-9 | 125 | 0 | 0 | 100 | 100 | 100 | 100 | 90 |

| Pre-emergence | g ai/ha | Weizen | Soja | Alopecurus | Echinochloa | Lolium | Setaria | Chenopodium |
|---|---|---|---|---|---|---|---|---|
| Bsp.I-1-a-5 | 60 | 0 | 0 | 100 | 100 | 100 | 100 | 70 |

| Pre-emergence | g ai/ha | Soja | Alopecurus | Bromus | Cyperus | Digitaria | Lolium | Setaria | Abutilo |
|---|---|---|---|---|---|---|---|---|---|
| Bsp.I-1-c-4 | 125 | 20 | 95 | 100 | 100 | 100 | 100 | 100 | 70 |

### Beispiel J

### Grenzkonzentrations-Test / Bodeninsekten - Behandlung transgener Pflanzen

| **Testinsekt:** | **Diabrotica balteata - Larven im Boden** |
|---|---|
| | |
| Lösungsmittel: | 7 Gewichtsteile |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 1 Töpfe und läßt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel K

### Heliothis virescens - Test - Behandlung transgener Pflanzen

| | |
|---|---|
| Lösungsmittel: | 7 Gewichtsteile |
| Emulgator : | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

## Patentansprüche

1. Verbindungen der Formel (I) 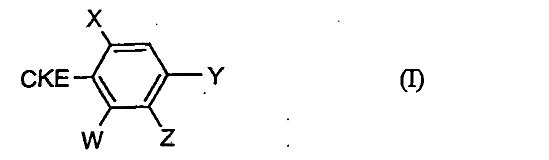 in welcher
W für Wasserstoff, Methyl, Ethyl, i-Propyl, Vinyl, oder Ethinyl steht,
X für Methyl, Ethyl, n-Propyl, i-Propyl, Vinyl, oder Ethinyl steht,
Y für Wasserstoff, Methyl, Ethyl, i-Propyl, Vinyl oder Ethinyl steht,
Z für Wasserstoff, Methyl, Ethyl, n-Propyl, i-Butyl, Vinyl oder Ethinyl steht,
mit der Maßgabe, dass mindestens einer der Reste W, X, Y oder Z für eine Kette mit mindestens zwei Kohlenstoffatomen steht, wobei maximal nur einer der Reste W, X, Y oder Z für Vinyl oder Ethinyl stehen darf,
CKE für eine der Gruppen 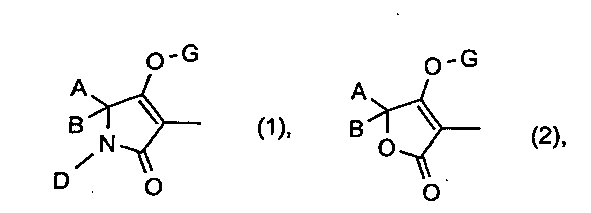 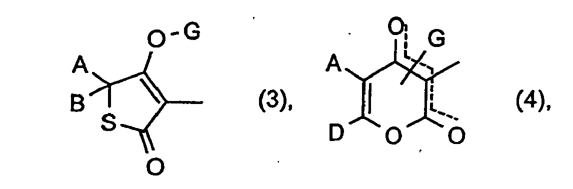 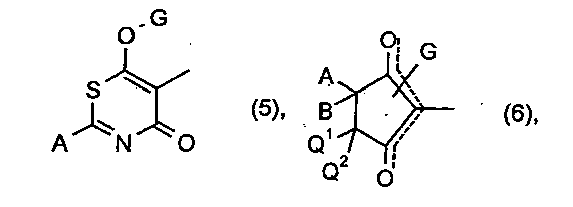 steht,
A für jeweils gegenbenenfalls durch Fluor substituiertes C₁-C₄-Alkyl oder C₁-C₂-Alkoxy-C₁-C₂-alkyl, Cyclopropyl, Cyclopentyl oder Cyclohexyl und nur im Fall der Verbindungen der Formel (I-5) für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl steht,
B für Wasserstoff, Methyl oder Ethyl steht, oder
A, B und das Kohlenstoffatom an das sie gebunden sind, für gesättigtes C₅-C₆-Cycloalkyl stehen, in welchem gegenbenefalls ein Ringglied durch Sauerstoff oder Schwefel ersetzt ist und welches gegenbenenfalls einfach durch Methyl, Ethyl, Propyl, Isopropyl, Trifluormethyl, Methoxy, Ethoxy, Propoxy oder Butoxy substituiert ist oder
A, B und das Kohlenstoffatom, an das sie gebunden sind, für C₆-Cycloalky stehenl, welches durch mit zwei nicht direkt benachbarten Sauerstoffatomen enthaltende Alkylendioxyl-Gruppe substituiert ist,
A, B und das Kohlenstoffatom, an das sie gebunden sind, für C₅-C₆-Cycloalkyl oder C₅-C₆-Cycloalkenyl stehen, worin zwei Substituenten gemeinsam mit den Kohlenstoffatomen, an die sie gebunden sind, für C₂-C₄-Alkandiyl oder C₂-C₄-Alkendiyl oder Butadiendiyl stehen,
D für Wasserstoff, für jeweils gegebenenfalls durch Fluor substituiertes C₁-C₄-Alkyl, C₃-C₄-Alkenyl, C₁-C₄-Alkoxy-C₂-C₃-alkyl, Cyclopropyl, Cyclopentyl oder Cyclohexyl oder (jedoch nicht im Fall der Verbindungen der Formeln (I-1)) für gegebenenfalls einfach durch Fluor, Chlor, Methyl, Ethyl, n-Propyl, iso-Propyl, Methoxy, Ethoxy oder Trifluormethyl substituiertes Phenyl oder Pyridyl steht,
A und Q¹ gemeinsam für gegebenenfalls einfach oder zweifach durch Methyl oder Methoxy substituiertes C₃-C₄-Alkandiyl stehen, oder
Q¹ für Wasserstoff steht,
Q² für Wasserstoff steht,
G für Wasserstoff (a) oder für eine der Gruppen 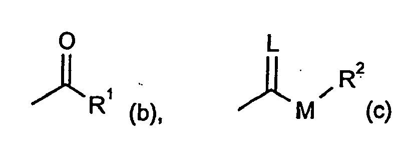
steht,
in welchen
L für Sauerstoff oder Schwefel steht und
M für Sauerstoff oder Schwefel steht,

2. Verfahren zur Herstellung von Verbindungen der Formel (I), **dadurch gekennzeichnet, dass** man zum Erhalt von
(A) 3-Phenylpyrrolidin-2,4-dione bzw. deren Enole der Formel (I-1-a) 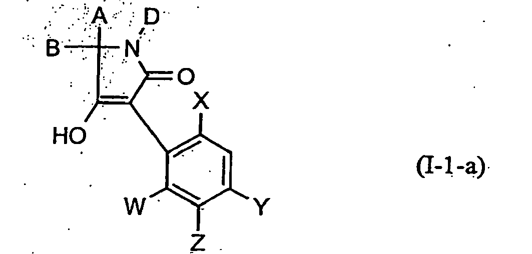 in welcher
A, B, D, W, X, Y und Z die oben angegebenen Bedeutungen haben,
N-Acylaminosaureester der Formel (II) 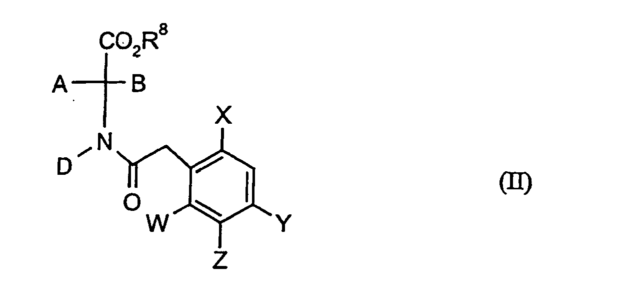
in welcher
A, B, D, W, X, Y und Z die oben angegebenen Bedeutungen haben,
und
R8 für Alkyl steht,
in Gegenwart eines Verdünnungsmittels und in Gegenwart einer Base intramolekular kondensiert,
(B) 3-Phenyl-4-hydroxy-Δ³-dihydrofuranon-Derivate der Formel (I-2-a) 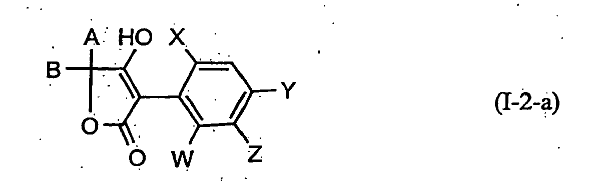 in welcher
A, B, W, X, Y und Z die oben angegebenen Bedeutungen haben,
Carbonsäureester der Förmel (III) 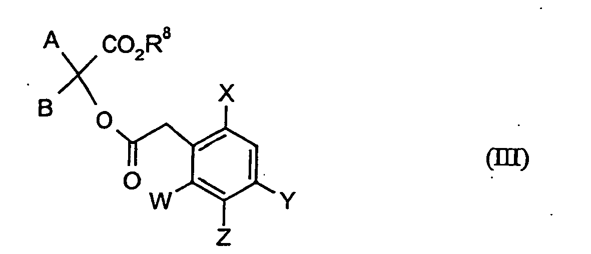 in welcher
A, B, W, X, Y, Z und R⁸ die oben angegebenen Bedeutungen haben,
in Gegenwart eines Verdünnungsmittels und in Gegenwart einer Base intramolekular kondensiert,
(C) 3-Phenyl-4-hydroky-Δ³-dihydrothiophenon-Derivate der Formel (I-3-a) 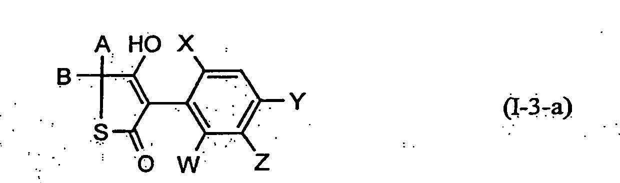 in welcher
A, B, W, X, Y und Z die oben angegebene Bedeutungen haben,
ß-Ketocarbonsäureester der Formel (IV) 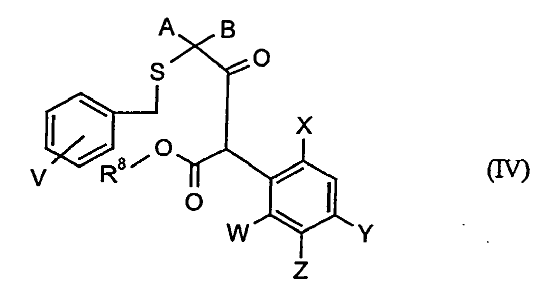 in welcher
A, B, W, X, Y, Z und R⁸ die oben angegebenen Bedeutungen haben und
V für Wasserstoff, Halogen, Alkyl öder Alkoxy steht,
gegebenenfalls in Gegenwart eines Verdünnungsmittels und in Gegenwart einer Säure intramolekular cyclisiert,
(D) 3-Phenylpyron-Derivate der Formel (I-4-a) 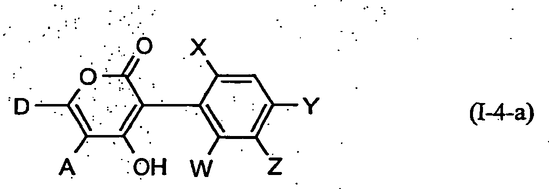 in welcher
A, D, W, X, Y und Z die oben angegebenen Bedeutungen haben,
Carbonylverbindungen der Formel (V) 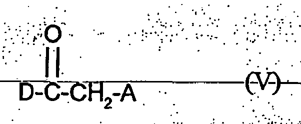 in welcher
A und D die oben angegebenen Bedeutungen haben,
oder deren Silylenolether der Formel (Va) 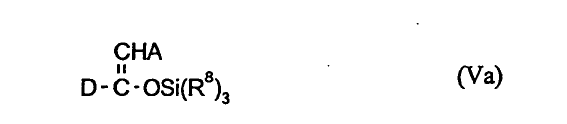 in welcher
A, D und R⁸ die oben angegebene Bedeutung haben,
mit Ketensäurehalogeniden der Formel (VI) 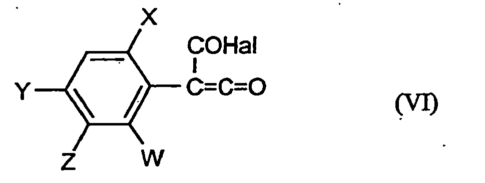 in welcher
W, X, Y und Z die oben angegebenen Bedeutungen haben und
Hal für Halogen steht,
gegebenenfalls in Gegenwart eines Verdünnungsmittels und gegebenenfalls in Gegenwart eines Säureakzeptors umsetzt,
(E) Phenyl-1,3-thiazin-Derivate der Formel (I-5-a) 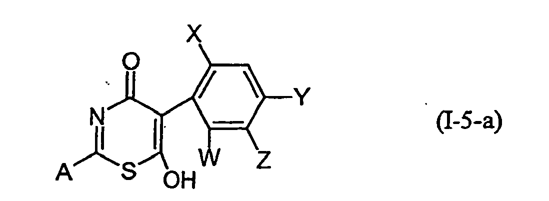 in welcher
A, W, X, Y und Z die oben angegebene Bedeutung haben,
Thioamide der Formel (VII) 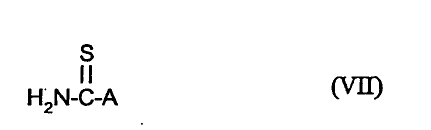 in welcher
A die oben angegebene Bedeutung hat,
mit Ketensäurehalogeniden der Formel (VI) 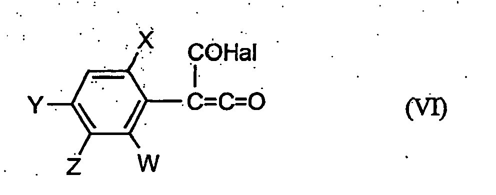 in welcher
Hal, W, X, Y und Z die oben angegebenen Bedeutungen haben,
gegebenenfalls in Gegenwart eines Verdünnungsmittels und gegebenenfalls in Gegenwart eines Säureakzeptors umsetzt,
(F) Verbindungen der Formel (I-6-a) 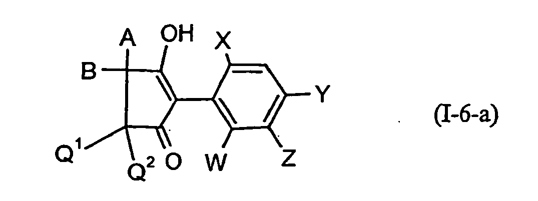 in welcher
A, B,Q¹, Q², W, X, Y und Z die oben angegebene Bedeutung haben,
Ketocarbonsäureester der Formel (VIII) 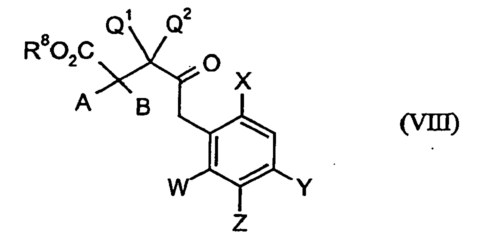 in welcher
A, B, Q¹, Q², W, X, Y und Z die oben angegebene Bedeutung haben, und
R⁸ für Alkyl steht,
gegebenenfalls in Gegenwart eines Verdünnungsmittels und in Gegenwart einer Base intramolekular cyclisiert,
(H) Verbindungen der oben gezeigten Formeln (I-1(a-g)) bis (I-6(a-g)), in welchen A, B, D, G, Q¹, Q², Q³, Q⁴, Q⁵, Q⁶, W, X, Y und Z die oben angegebene Bedeutung haben,
wobei ein, maximal zwei Reste W, X, Y oder Z für R²²-C≡C- oder 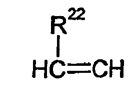 stehen, R²² steht für Wasserstoff oder C₁-C₄-Alkyl,
Verbindungen der Formel (I-1'(a-g)) bis (I-6'(a-g)), 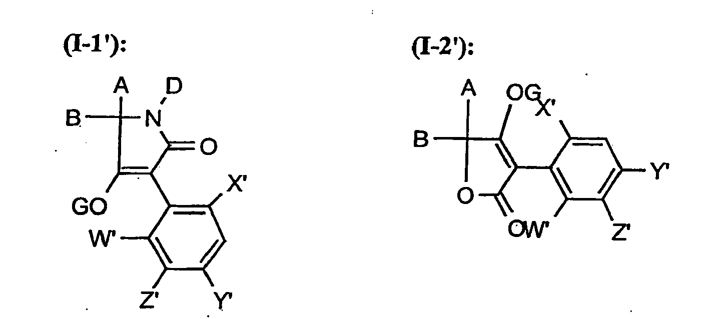 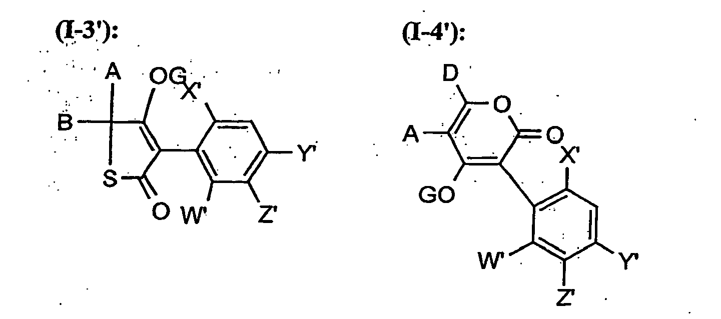 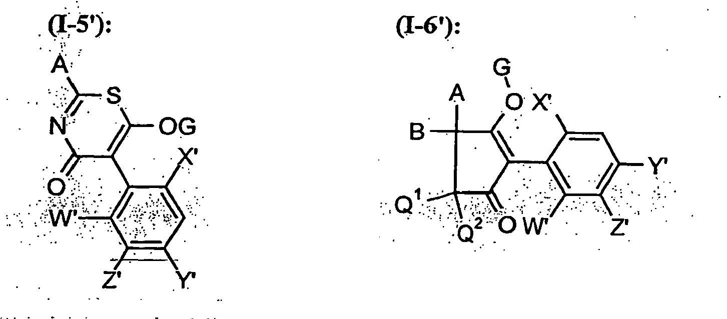 in welchen
A, B, D, G, Q¹, Q², W', X', Y' und Z' die oben angegebene Bedeutung haben und wobei der Hochstrich ' bedeutet, dass ein, maximal zwei Reste W, X, Y und Z bei diesem Verfahren für Chlor, Brom, Jod stehen, mit der Maßgabe, dass die anderen Reste W, X, Y oder Z nicht für Alkenyl oder Alkinyl stehen
mit Silylacetylenen der Formel (X-a) oder Vinylstananen der Formel (X-b) 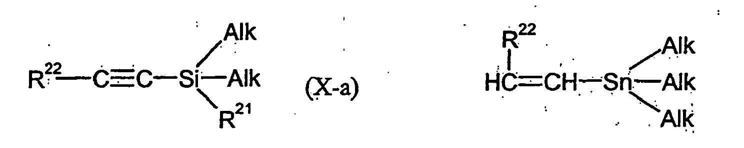 in welchen Alk für C₁-C₄-Alkyl und
R²¹ für C₁-C₄-Alkyl oder Phenyl steht,
R²² für Wasserstoff oder C₁-C₄-Alkyl steht,
in Gegenwart eines Lösungsmittels, gegebenenfalls in Gegenwart einer Base und eines Katalysators umsetzt,
(I) Verbindungen der oben gezeigten Formeln (I-1-b) bis (I-6-b), in welchen A, B, D, Q¹, Q², R¹, W, X, Y und Z die oben angebenen Bedeutungen haben, Verbindungen der oben gezeigten Formeln (I-1-a) bis (I-6-a), in welchen A, B, D, Q¹, Q², W, X, Y und Z die oben angegebenen Bedeutungen haben, jeweils
(α) mit Säurehalogeniden der Formel (XI) 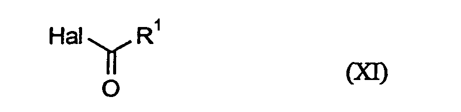 in welcher
R¹ die oben angegebene Bedeutung hat und
Hal für Halogen steht
oder
(β) mit Carbonsäureanhydriden der Formel (XII)
R¹-CO-O-CO-R¹ (XII)
in welcher
R¹ die oben angegebene Bedeutung hat,
gegebenenfalls in Gegenwart eines Verdünnungsmittels und gegebenenfalls in Gegenwart eines Säurebindemittels umsetzt;
(J) Verbindungen der oben gezeigten Formeln (I-1-c) bis (I-6-c), in welchen A, B, D, Q¹, Q², R², M, W, X, Y und Z die oben angegebenen Bedeutungen haben und L für Sauerstoff steht, Verbindungen der oben gezeigten Formeln (I-1-a) bis (I-6-a), in welchen A, B, D, Q¹, Q², W, X, Y und Z die oben angegebenen Bedeutungen haben, jeweils
mit Chlorameisensäureestern oder Chlorameisensäurethioestern der Formel (XIII)
R²-M-CO-Cl (XIII)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(K) Verbindungen der oben gezeigten Formeln (I-1-c) bis (I-6-c); in welchen A, B, D, Q¹, Q², R², M, W, X, Y und Z die oben angegebene Bedeutungen haben und L für Schwefel steht, Verbindungen der oben gezeigten Formeln (I-1-a) bis (I-6-a), in welchen A, B, D, Q1, Q², W, X, Y und Z die oben angegebenen Bedeutungen haben, jeweils
mit Chlormonothioameisensäureestern oder Chlordithioameisensäureestern der Formel (XIV) 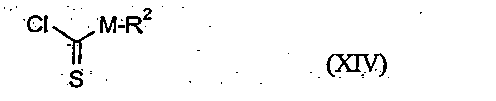 in welcher
M und R² die oben angegebenen Bedeutungen haben,
gegebenenfalls in Gegenwart eines Verdünnungsmittels und gegebenenfalls in Gegenwart eines Säurebindemittels umsetzt
und
(L) Verbindungen der oben gezeigten Formeln (I-1-d) bis (I-6-d), in welchen A, B, D, Q¹, Q², R³, W, X, Y und Z die oben angegebenen Bedeutungen haben, Verbindungen der oben gezeigten Formeln (I-1-a) bis (I-6-a), in welchen A, B, D, Q¹, Q², W, X, Y und Z die oben angegebenen Bedeutungen haben, jeweils
mit Sulfonsäurechloriden der Formel (XV)
R³-SO₂-Cl (XV)
in welcher
R³ die oben angegebene Bedeutung hat,
Gegebenenfalls in Gegenwart eines Verdünnungsmittels und gegebenenfalls in Gegenwart eines Säurebindemittels umsetzt,
(M) Verbindungen der oben gezeigten Formeln (I-1-e) bis (I-6-e), in welchen A, B, D, L, Q¹, Q², R⁴, R⁵, W, X, Y Y und Z die oben angegebenen Bedeutungen haben, Verbindungen der oben gezeigten Formeln (I-1-a) bis (I-6-a), in welchen A, B, D, Q¹, Q², W, X, Y und Z die oben angegebenen Bedeutungen haben, jeweils
mit Phosphorverbindungen der Formel (XVI) 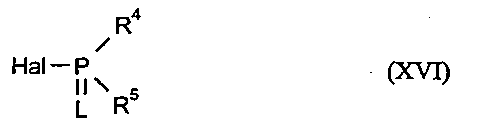 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(N) Verbindungen der oben gezeigten Formeln (I-1-f) bis (I-6-f), in welchen A, B, D, E, Q¹, Q², W, X, Y und Z die oben angegebenen Bedeutungen haben, Verbindungen der Formeln (I-1-a) bis (I-6-a), welchen A, B, D, Q¹, Q², W, X, Y und Z die oben angegebenen Bedeutungen haben, jeweils
mit Metallverbindungen oder Aminen der Formeln (XVII) oder (XVIII)
Me(OR¹⁰)ₜ (XVII) 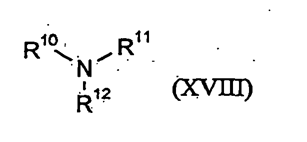
in welchen
Me für ein ein- oder zweiwertiges Metall,
t für die Zahl 1 oder 2 und
R¹⁰, R¹¹, R¹², unabhängig voneinander für Wasserstoff oder Alkyl stehen,
gegebenenfalls in Gegenwart eines Verdünnungsmittels umsetzt,
(O) Verbindungen der oben gezeigten Formeln (I-1-g) bis (I-6-g), in welchen A, B, D, L, Q¹, Q², R⁶, R⁷, W, X, Y und Z die oben angegebenen Bedeutungen haben, Verbindungen der oben gezeigten Formeln (I-1-a) bis (I-6-a), in welchen A, B, D, Q¹, Q², W, X, Y und Z die oben angegebenen Bedeutungen haben, jeweils
(α) mit Isocyanaten oder Isothiocyanaten der Formel (XIX)
R⁶-N=C=L (XIX)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
(β) mit Carbamidsäurechloriden oder Thiocarbamidsäurechloriden der Formel (XX) 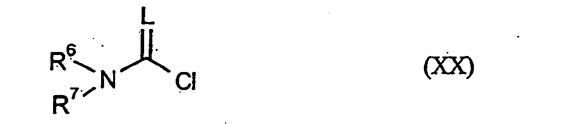
in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt.

3. Verbindung der Formel (I) gemäß Anspruch 1, welcher
W für Wasserstoff, Methyl, Ethyl oder i-Propyl steht,
X für Methyl, Ethyl, i-Propyl oder Vinyl steht,
Y für Wasserstoff, Methyl, Ethyl, i-Propyl, Vinyl oder Ethinyl steht,
Z für Wasserstoff, Methyl, Ethyl, n-Propyl oder i-Butyl steht,
mit der Maßgabe, dass mindestens einer der Reste W, X, Y oder Z für eine Kette mit mindestens zwei Kohlenstoffatomen steht, wobei maximal nur einer der Reste W, X, Y oder Z für Vinyl oder Ethinyl stehen darf,
CKE für eine der Gruppen 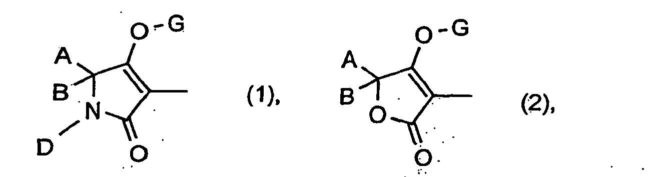 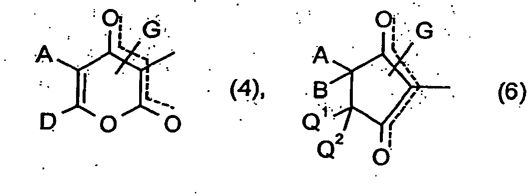 steht,
A für Methyl steht,
B für Methyl steht,
A, B und das Kohlenstoffatom, an das sie gebunden sind, für gesättigtes C₅-C₆-Cycloalkyl stehen, in welchem gegenbenenfalls ein Ringglied durch Sauerstoff ersetzt ist und welches gegenbenenfalls einfach durch Methyl, Ethyl, Methoxy, Ethoxy substituiert ist,
A und B gemeinsam für 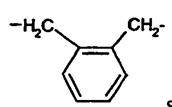 stehen,
D für Wasserstoff oder (jedoch nicht im Fall der Verbindung der Formel (I-1)) für durch Fluor substituiertes Phenyl steht,
G für Wasserstoff (a) oder für eine der Gruppen 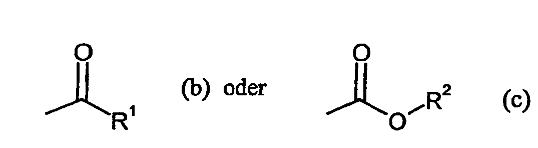 steht,
R¹ für C₁-C₆-Alkyl, C₁-C₂-Alkoxymethyl steht,
R² für C₁-C₄-Alkyl steht,
für CKE = (6) stehen
A und Q¹ gemeinsam für C₃-C₄-Alkyandiyl und
B und Q² jeweils für Wasserstoff.

4. Verbindungen der Formel (II) 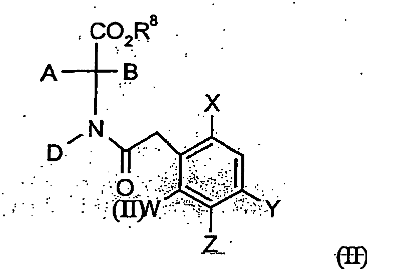 in welcher
A, B, D, W, X, Y, Z und R⁸ die oben angegebenen Bedeutungen haben.

5. Verbindungen der Formel (III) 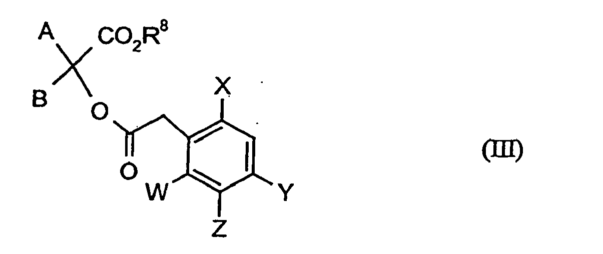 in welcher
A, B, W, X, Y, Z und R⁸ die oben angegebenen Bedeutungen haben.

6. Verbindungen der Formel (IV) 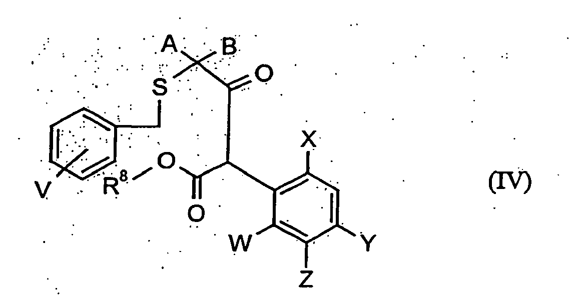 in welcher
A, B, W, X, Y, Z und R⁸ die oben angegebenen Bedeutungen haben,
V für Wasserstoff, Halogen, Alkyl oder Alkoxy steht.

7. Verbindungen der Formel (IV) 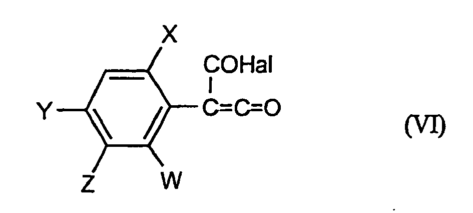 in welcher
W, X, Y und Z die oben angegebenen Bedeutungen haben und
Hal für Halogen steht.

8. Verbindungen der Formel (VIII) 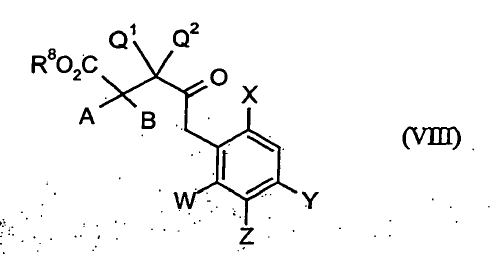 in welcher
A, B, Q¹, Q², W, X, Y und Z die oben angegebene Bedeutung haben, und
R⁸ für Alkyl steht.

9. Verbindungen der Formel (XXV) 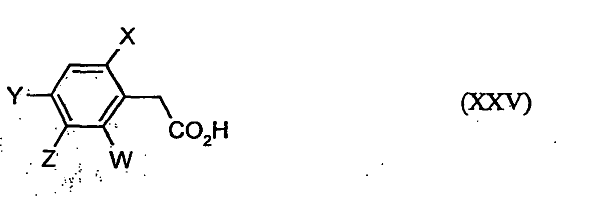 in welcher
W, X, Y und Z die oben angegebene Bedeutung haben und
Y außerdem für -C≡C-Si(CH₃)₃ stehen kann.

10. Verbindungen der Formel (XXII) 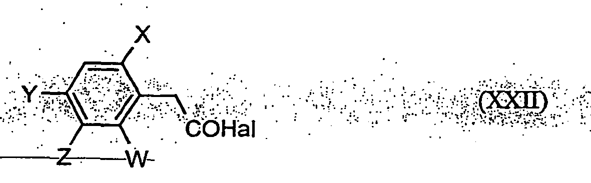 in welcher
W, X, Y und Z die oben angegebenen Bedeutungen haben und
Hal für Chlor oder Brom steht.

11. Verbindungen der Formel (XXX) 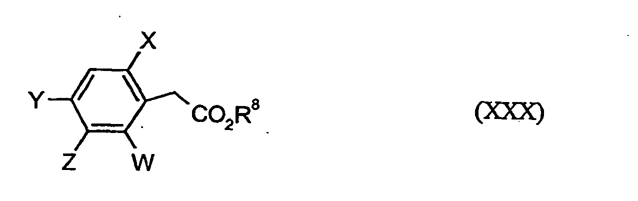 in welcher
W, X, Y, Z und R⁸ die oben angegebene Bedeutung haben und
Y außerdem für-C≡C-Si(CH₃)₃ stehen kann.

12. Verbindungen der Formel (XLII) 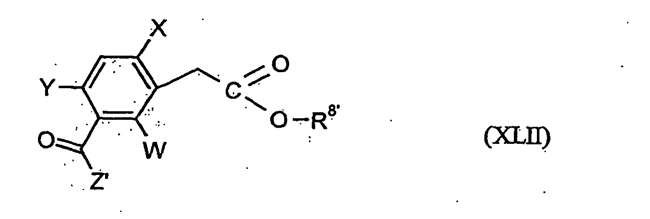 in welcher
W, X, Y und R^{8'} die oben angegebene Bedeutung haben und
Z' für Alkyl steht.

13. Schädlingsbekämpfungsmittel und Herbizide, **gekennzeichnet durch** einen Gehalt an mindestens einer Verbindung der Formel (I) gemäß Anspruch 1.

14. Verfahren zur Bekämpfung von tierischen Schädlingen und unerwünschten Pflanzenbewuchs, **dadurch gekennzeichnet, daß** man Verbindungen der Formel (I) gemäß Anspruch 1 auf Schädlinge und/oder ihren Lebensraum einwirken läßt, ausgenommen Verfahren am menschlichen oder tierischen körper.

15. Verwendung von Verbindungen der Formel (I) gemäß Anspruch 1 zur Bekämpfung von tierischen Schädlingen und unerwünschtem Pflanzenbewuchs, ausgenommen Verfahren am menschlichen oder tierischen Kkörper.

16. Verfahren zur Herstellung von Schädlingsbekämpfungsmitteln und Herbiziden, **dadurch gekennzeichnet, daß** man Verbindungen der Formel (I) gemäß Anspruch 1 mit Streckmitteln und/oder oberflächenaktiven Stoffen vermischt.

17. Verwendung von Verbindungen der Formel (I) gemäß Anspruch 1 zur Herstellung von Schädlingsbekämpfungsmitteln und Herbiziden.

## Claims

1. Compounds of the formula (I) 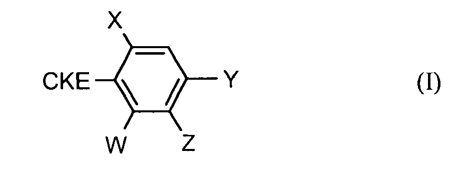 in which
W represents hydrogen, methyl, ethyl, i-propyl, vinyl or ethinyl,
X represents methyl, ethyl, n-propyl, i-propyl, vinyl or ethinyl,
Y represents hydrogen, methyl, ethyl, i-propyl, vinyl or ethinyl,
Z represents hydrogen, methyl, ethyl, n-propyl, i-butyl, vinyl or ethinyl,
with the proviso that at least one of the radicals W, X, Y or Z represents a chain having at least two carbon atoms where at most only one of the radicals W, X, Y or Z may represent vinyl or ethinyl,
CKE represents one of the groups 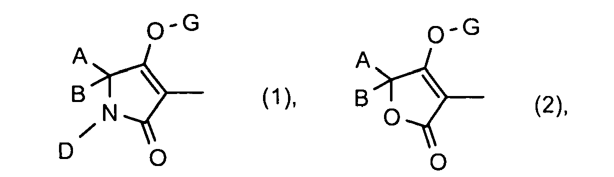 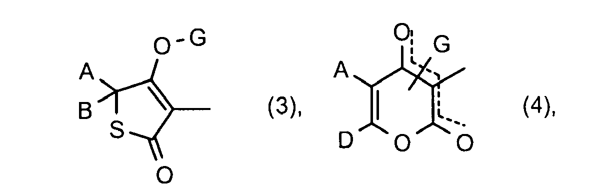 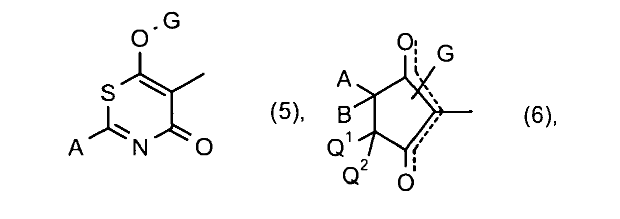
A represents in each case optionally fluorine-substituted C₁-C₄-alkyl or C₁-C₂-alkoxy-C₁-C₂-alkyl, cyclopropyl, cyclopentyl or cyclohexyl and, only in the case of the compounds of the formula (1-5), represents optionally fluorine-, chlorine-, bromine-, methyl-, ethyl-, n-propyl-, iso-propyl-, methoxy-, ethoxy-, trifluoromethyl-, trifluoromethoxy-, cyano- or nitro-substituted phenyl,
B represents hydrogen, methyl or ethyl, or
A, B and the carbon atom to which they are attached represent saturated C₅-C₆-cycloalkyl in which optionally one ring member is replaced by oxygen or sulphur and which is optionally monosubstituted by methyl, ethyl, propyl, isopropyl, trifluoromethyl, methoxy, ethoxy, propoxy or butoxy or
A, B and the carbon atom to which they are attached represent C₆-cycloalkyl which is substituted by an alkylenedioxyl group which contains two oxygen atoms which are not directly adjacent, or
A, B and the carbon atom to which they are attached represent C₅-C₆-cycloalkyl or C₅-C₆-cycloalkenyl in which two substituents together with the carbon atoms to which they are attached represent C₂-C₄-alkanediyl or C₂-C₄-alkenediyl or butadienediyl,
D represents hydrogen, represents in each case optionally fluorine-substituted C₁-C₄-alkyl, C₃-C₄-alkenyl, C₁-C₄-alkoxy-C₂-C₃-alkyl, cyclopropyl, cyclopentyl or cyclohexyl or (but not in the case of the compounds of the formula (I-1)) represents pyridyl or phenyl, each of which is optionally monosubstituted by fluorine, chlorine, methyl, ethyl, n-propyl, iso-propyl, methoxy, ethoxy or trifluoromethyl,
or
A and Q¹ together represent C₃-C₄-alkanediyl which is optionally mono- or disubstituted by methyl or methoxy, or
Q¹ represents hydrogen,
Q² represents hydrogen,
G represents hydrogen (a) or represents one of the groups 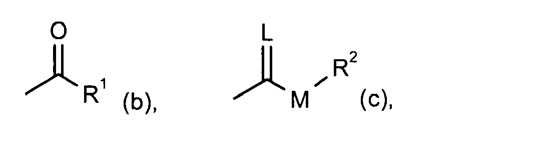 in which
L represents oxygen or sulphur and
M represents oxygen or sulphur,
R¹ represents C₁-C₆-alkyl, C₂-C₆-alkenyl, C₁-C₂-alkoxy-C₁-alkyl, C₁-C₂-alkylthio-C₁-alkyl, or represents cyclohexyl or cyclopropyl, each of which is optionally monosubstituted by fluorine, chlorine, methyl or methoxy,
represents phenyl which is optionally monosubstituted by fluorine, chlorine, bromine, cyano, nitro, methyl, methoxy, trifluoromethyl or trifluoromethoxy,
R² represents in each case optionally fluorine-substituted C₁-C₈-alkyl, C₂-C₆-alkenyl or C₁-C₄-alkoxy-C₂-C₃-alkyl, phenyl or benzyl.

2. Process for preparing compounds of the formula (I), **characterized in that**
(A) 3-phenylpyrrolidine-2,4-diones or their enols of the formula (I-1-a) 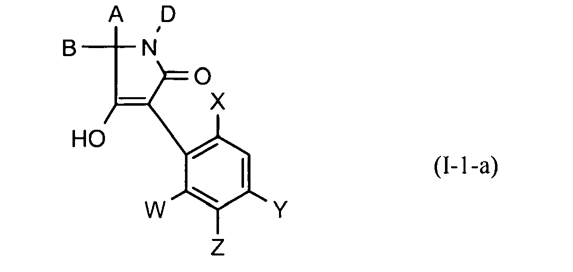 in which
A, B, D, W, X, Y and Z are each as defined above
are obtained by the intramolecular condensation of
N-acylamino acid esters of the formula (II) 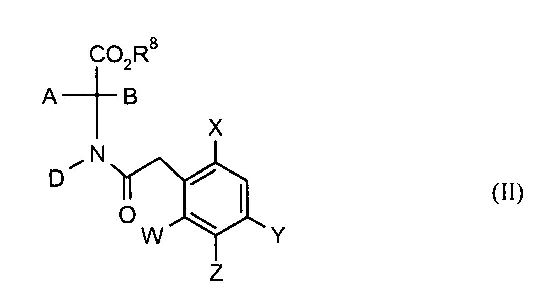 in which
A, B, D, W, X, Y and Z are each as defined above,
and
R⁸ represents alkyl,
in the presence of a diluent and in the presence of a base,
(B) 3-phenyl-4-hydroxy-Δ³-dihydrofuranone derivatives of the formula (I-2-a) 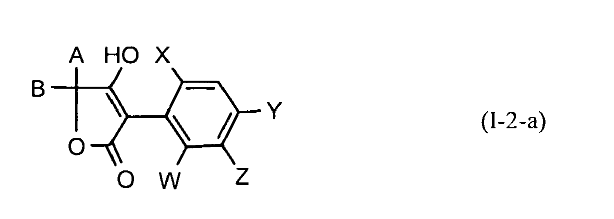 in which
A, B, W, X, Y and Z are each as defined above,
are obtained by the intramolecular condensation of
carboxylic esters of the formula (III) 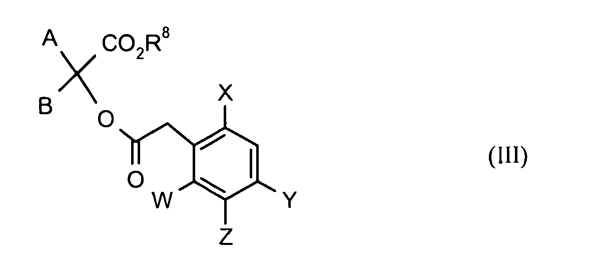 in which
A, B, W, X, Y, Z and R⁸ are each as defined above,
in the presence of a diluent and in the presence of a base,
(C) 3-phenyl-4-hydroxy-Δ³-dihydrothiophenone derivatives of the formula (1-3-a) 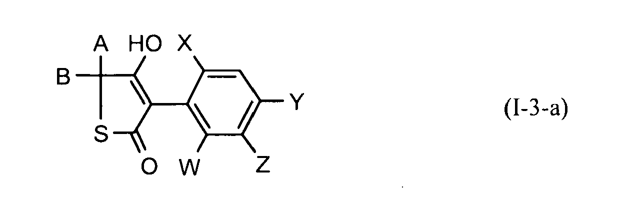 in which
A, B, W, X, Y and Z are each as defined above,
are obtained by the intramolecular cyclization of
β-ketocarboxylic esters of the formula (IV) 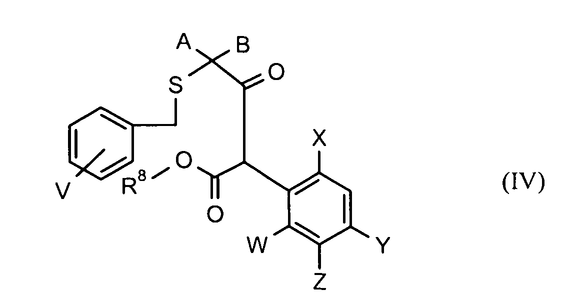 in which
A, B, W, X, Y, Z and R⁸ are each as defined above and
V represents hydrogen, halogen, alkyl or alkoxy,
if appropriate in the presence of a diluent and in the presence of an acid,
(D) 3-phenylpyrone derivatives of the formula (I-4-a) 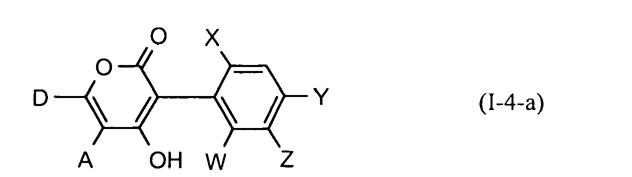 in which
A, D, W, X, Y and Z are each as defined above,
are obtained by reacting
carbonyl compounds of the formula (V) 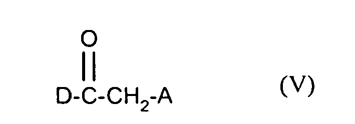 in which
A and D are as defined above
or their silyl enol ethers of the formula (Va) 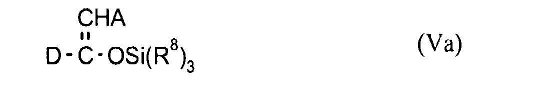 in which
A, D and R⁸ are each as defined above
with ketene acid halides of the formula (VI) 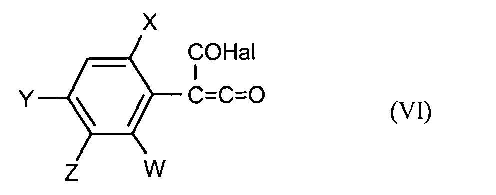 in which
W, X, Y and Z are each as defined above and
Hal represents halogen,
if appropriate in the presence of a diluent and if appropriate in the presence of an acid acceptor,
(E) phenyl-1,3-thiazine derivatives of the formula (I-5-a) 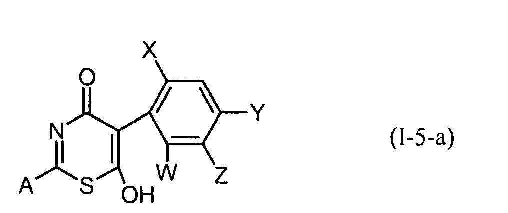 in which
A, W, X, Y and Z are each as defined above
are obtained by reacting
thioamides of the formula (VII) 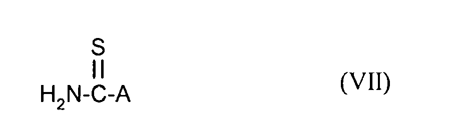 in which
A is as defined above,
with ketene acid halides of the formula (VI) 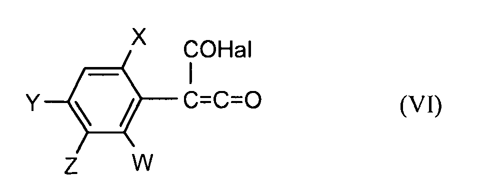 in which
Hal, W, X, Y and Z are each as defined above,
if appropriate in the presence of a diluent and if appropriate in the presence of an acid acceptor,
(F) compounds of the formula (I-6-a) 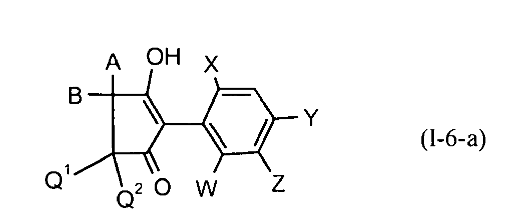 in which
A, B, Q¹, Q², W, X, Y and Z are each as defined above
are obtained by intramolecular cyclization of
ketocarboxylic esters of the formula (VIII) 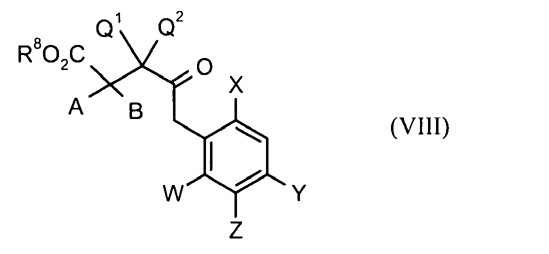 in which
A, B, Q¹, Q², W, X, Y and Z are each as defined above and
R⁸ represents alkyl,
if appropriate in the presence of a diluent and if appropriate in the presence of a base,
(H) compounds of the formulae (I-1(a-g)) to (I-6(a-g)) shown above, in which A, B, D, G, Q¹, Q², Q³, Q⁴, Q⁵, Q⁶, W, X, Y and Z are each as defined above,
where one, or at most two, radicals W, X, Y or Z represent R²²-C≡C- or 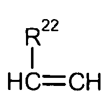 and R²² represents hydrogen or C₁-C₄-alkyl,
are obtained by reacting compounds of the formulae (I-1'(a-g)) to (I-6'(a-g)), 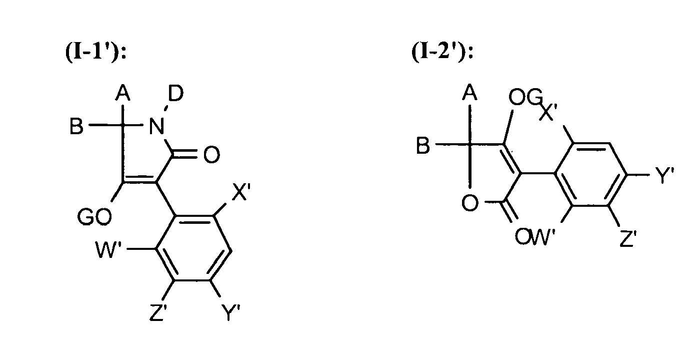 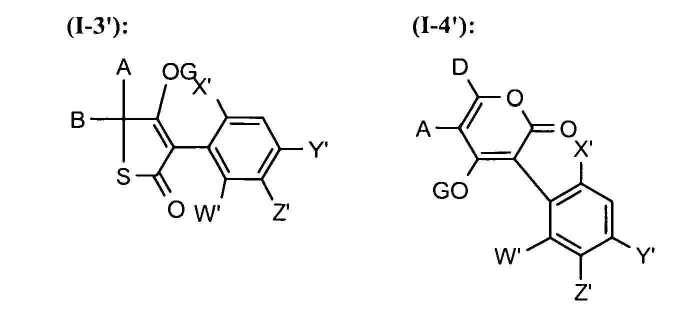 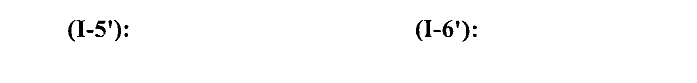 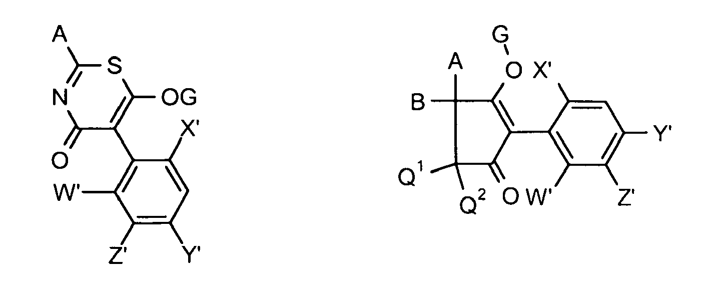 in which
A, B, D, G, Q¹, Q², W', X', Y' and Z' are each as defined above and where the apostrophe ' means that one, at most two, radicals W, X, Y and Z in this process represent chlorine, bromine or iodine, with the proviso that the other radicals W, X, Y and Z do not represent alkenyl or alkinyl
with silylacetylenes of the formula (X-a) or vinylstannanes of the formula (X-b) 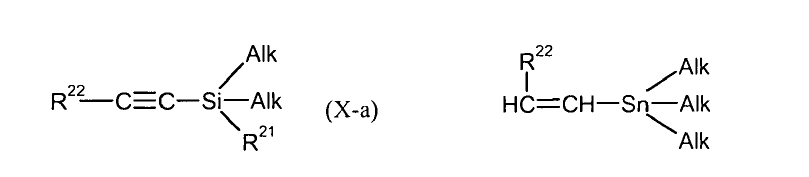 in which alk represents C₁-C₄-alkyl and
R²¹ represents C₁-C₄-alkyl or phenyl,
R²² represents hydrogen or C₁-C₄-alkyl,
in the presence of a solvent, if appropriate in the presence of a base and a catalyst,
(I) compounds of the formulae (I-1-b) to (1-6-b) shown above in which A, B, D, Q¹, Q², R¹, W, X, Y and Z are each as defined above are obtained by reacting compounds of the formulae (I-1-a) to (I-6-a) shown above in which A, B, D, Q¹, Q², W, X, Y and Z are each as defined above in each case
(α) with acid halides of the formula (XI) 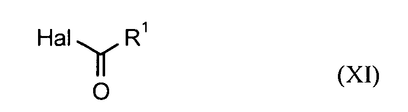 in which
R¹ is as defined above and
Hal represents halogen
or
(β) with carboxylic anhydrides of the formula (XII)
R¹-CO-O-CO-R¹ (XII)
in which
R¹ is as defined above,
if appropriate in the presence of a diluent and if appropriate in the presence of an acid binder,
(J) compounds of the formulae (I-1-c) to (I-6-c) shown above in which A, B, D, Q¹, Q², R², M, W, X, Y and Z are each as defined above and L represents oxygen are obtained by reacting compounds of the formulae (I-1-a) to (I-6-a) shown above in which A, B, D, Q¹, Q², W, X, Y and Z are each as defined above in each case
with chloroformic esters or chloroformic thioesters of the formula (XIII)
R²-M-CO-Cl (XIII)
in which
R² and M are each as defined above,
if appropriate in the presence of a diluent and if appropriate in the presence of an acid binder;
(K) compounds of the formulae (I-1-c) to (I-6-c) shown above in which A, B, D, Q¹, Q², R², M, W, X, Y and Z are each as defined above and L represents sulphur are obtained by reacting compounds of the formulae (I-1-a) to (I-6-a) shown above in which A, B, D, Q¹, Q², W, X, Y and Z are each as defined above in each case
with chloromonothioformic esters or chlorodithioformic esters of the formula (XIV) 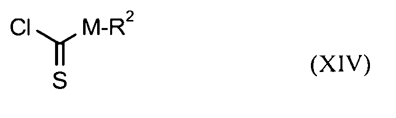 in which
M and R² are each as defined above,
if appropriate in the presence of a diluent and if appropriate in the presence of an acid binder,
and
(L) compounds of the formulae (I-1-d) to (I-6-d) shown above in which A, B, D, Q¹, Q², R³, W, X, Y and Z are each as defined above are obtained by reacting compounds of the formulae (I-1-a) to (I-6-a) shown above in which A, B, D, Q¹, Q², W, X, Y and Z are each as defined above in each case
with sulphonyl chlorides of the formula (XV)
R³-SO₂-Cl (XV)
in which
R³ is as defined above,
if appropriate in the presence of a diluent and if appropriate in the presence of an acid binder,
(M) compounds of the formulae (I-1-e) to (I-6-e) shown above in which A, B, D, L, Q¹, Q², R⁴, R⁵, W, X, Y and Z are each as defined above are obtained by reacting compounds of the formulae (I-1-a) to (I-6-a) shown above in which A, B, D, Q¹, Q², W, X, Y and Z are each as defined above in each case
with phosphorus compounds of the formula (XVI) 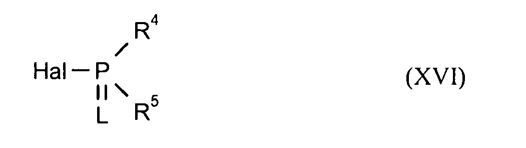 in which
L, R⁴ and R⁵ are each as defined above and
Hal represents halogen,
if appropriate in the presence of a diluent and if appropriate in the presence of an acid binder,
(N) compounds of the formulae (I-1-f) to (I-6-f) shown above in which A, B, D, E, Q¹, Q², W, X, Y and Z are each as defined above are obtained by reacting compounds of the formulae (I-1-a) to (I-6-a), in which A, B, D, Q¹, Q², W, X, Y and Z are each as defined above in each case
with metal compounds or amines of the formulae (XVII) or (XVIII)
Me(OR¹⁰)ₜ (XVII) 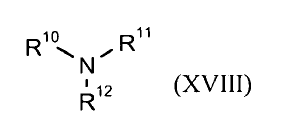
in which
Me represents a mono- or divalent metal,
t represents the number 1 or 2 and
R¹⁰, R¹¹, R¹² independently of one another each represents hydrogen or alkyl,
if appropriate in the presence of a diluent,
(O) compounds of the formulae (I-1-g) to (I-6-g) shown above in which A, B, D, L, Q¹, Q², R⁶, R⁷, W, X, Y and Z are each as defined above are obtained by reacting compounds of the formulae (I-1-a) to (I-6-a) shown above in which A, B, D, Q¹, Q², W, X, Y and Z are each as defined above in each case
(α) with isocyanates or isothiocyanates of the formula (XIX)
R⁶-N=C=L (XIX)
in which
R⁶ and L are each as defined above,
if appropriate in the presence of a diluent and if appropriate in the presence of a catalyst, or
(β) with carbamoyl chlorides or thiocarbamoyl chlorides of the formula (XX) 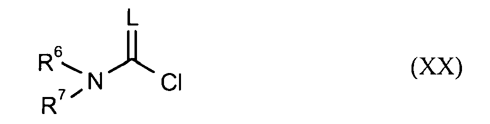
in which
L, R⁶ and R⁷ are each as defined above,
if appropriate in the presence of a diluent and if appropriate in the presence of an acid binder.

3. Compounds of the formula (I) according to Claim 1, in which
W represents hydrogen, methyl, ethyl or i-propyl,
X represents methyl, ethyl, i-propyl or vinyl,
Y represents hydrogen, methyl, ethyl, i-propyl, vinyl or ethinyl,
Z represents hydrogen, methyl, ethyl, n-propyl or i-butyl,
with the proviso that at least one of the radicals W, X, Y or Z represents a chain having at least two carbon atoms, where at most only one of the radicals W, X, Y or Z may represent vinyl or ethinyl,
CKE represents one of the groups 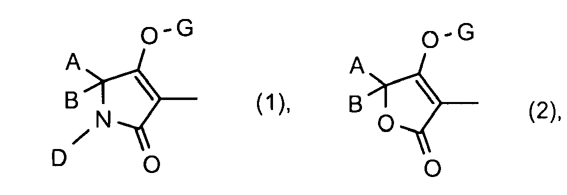 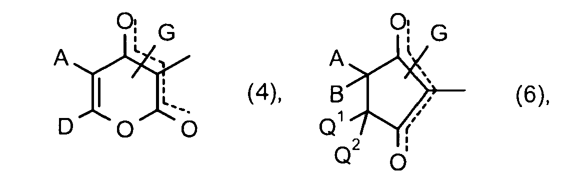
A represents methyl,
B represents methyl,
A, B and the carbon atoms to which they are attached represent saturated C₅-C₆-cycloalkyl in which optionally one ring member is replaced by oxygen and which is optionally monosubstituted by methyl, ethyl, methoxy, ethoxy,
A and B together represent 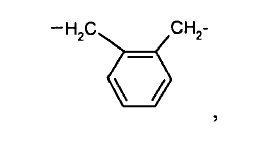
D represents hydrogen or (but not in the case of the compound of the formula (I-1)) represents fluorine-substituted phenyl,
G represents hydrogen (a) or represents one of the groups 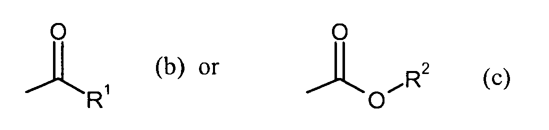
R¹ represents C₁-C₆-alkyl, C₁-C₂-alkoxymethyl,
R² represents C₁-C₄-alkyl,
for CKE = (6)
A and Q¹ together represent C₃-C₄-alkanediyl and
B and Q² each represent hydrogen.

4. Compounds of the formula (II) 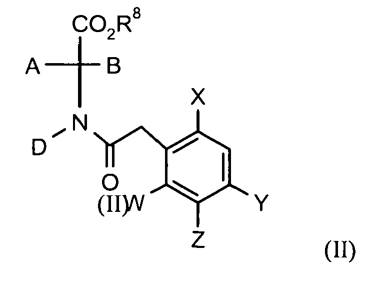 in which
A, B, D, W, X, Y, Z and R⁸ are each as defined above.

5. Compounds of the formula (III) 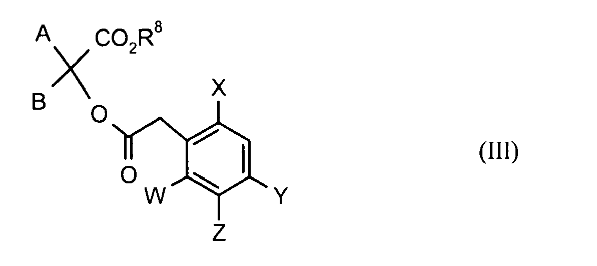 in which
A, B, W, X, Y, Z and R⁸ are each as defined above.

6. Compounds of the formula (IV) 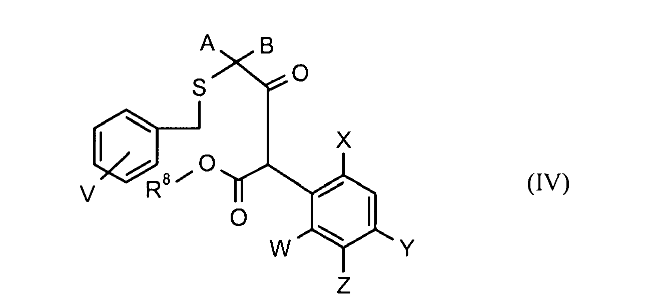 in which
A, B, W, X, Y, Z and R⁸ are each as defined above,
V represents hydrogen, halogen, alkyl or alkoxy.

7. Compounds of the formula (VI) 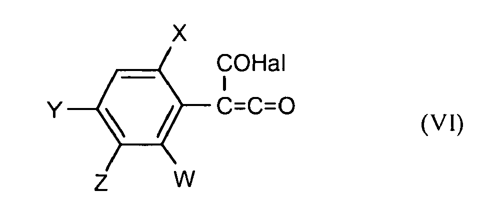 in which
W, X, Y and Z are each as defined above and
Hal represents halogen.

8. Compounds of the formula (VIII) 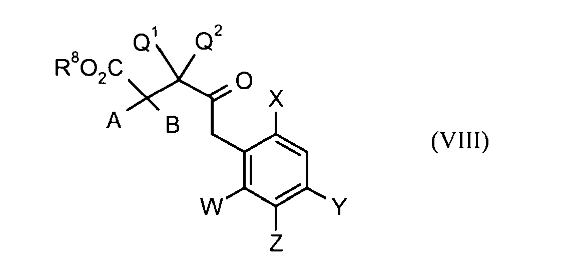 in which
A, B, Q¹, Q², W, X, Y and Z are each as defined above, and
R⁸ represents alkyl.

9. Compounds of the formula (XXV) 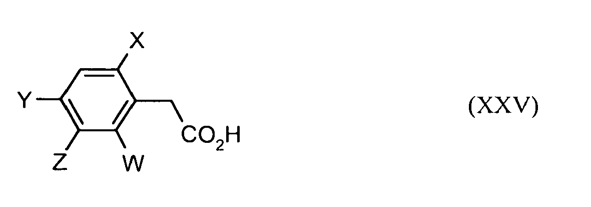 in which
W, X, Y and Z are each as defined above and
Y may furthermore represent -C≡C-Si(CH₃)_{3.}

10. Compounds of the formula (XXII) 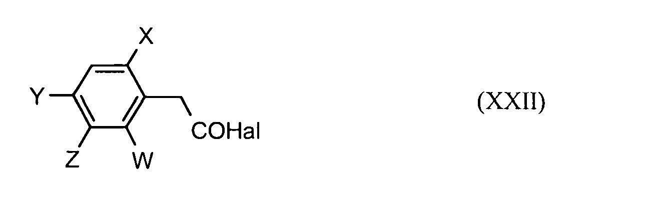 in which
W, X, Y and Z are each as defined above and
Hal represents chlorine or bromine.

11. Compounds of the formula (XXX) 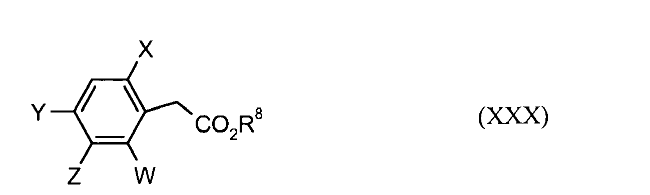 in which
W, X, Y, Z and R⁸ are each as defined above and
Y may furthermore represent -C≡C-Si(CH₃)₃.

12. Compounds of the formula (XLII) 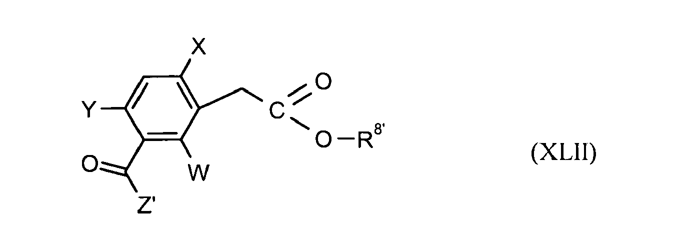 in which
W, X, Y and R^{8'} are each as defined above and
Z' represents alkyl.

13. Pesticides and herbicides, **characterized in that** they comprise at least one compound of the formula (I) according to Claim 1.

14. Method for controlling animal pests and undesirable vegetation, **characterized in that** compounds of the formula (I) according to Claim 1 are allowed to act on pests and/or their habitat, with the exception of methods performed on the human or animal body.

15. Use of compounds of the formula (I) according to Claim 1 for controlling animal pests and undesirable vegetation, with the exception of methods performed on the human or animal body.

16. Process for preparing pesticides and herbicides, **characterized in that** compounds of the formula (I) according to Claim 1 are mixed with extenders and/or surfactants.

17. Use of compounds of the formula (I) according to Claim 1 for preparing pesticides and herbicides.

## Revendications

1. Composés de formule (I) 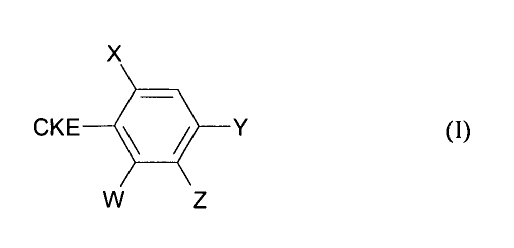 dans laquelle
W est un hydrogène ou un groupe méthyle, éthyle, isopropyle, vinyle ou éthynyle,
X est un groupe méthyle, éthyle, n-propyle, isopropyle, vinyle ou éthynyle,
Y est un hydrogène ou un groupe méthyle, éthyle, isopropyle, vinyle ou éthynyle,
Z est un hydrogène ou un groupe méthyle, éthyle, n-propyle, isobutyle, vinyle ou éthynyle,
à la condition que l'un au moins des restes W, X, Y ou Z soit une chaîne comprenant au moins deux atomes de carbone, au maximum un seul des restes W, X, Y ou Z pouvant être un groupe vinyle ou éthynyle,
CKE représente un des groupes 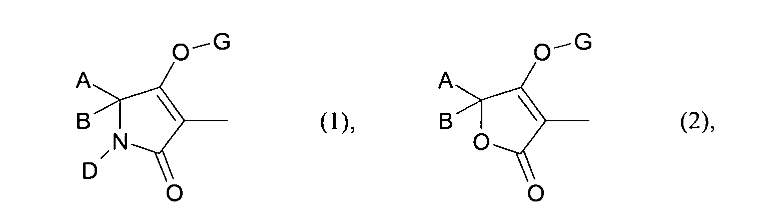 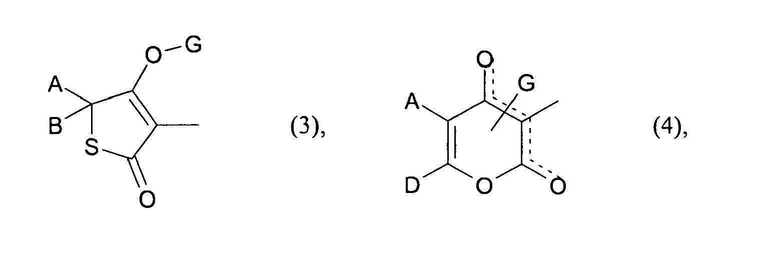 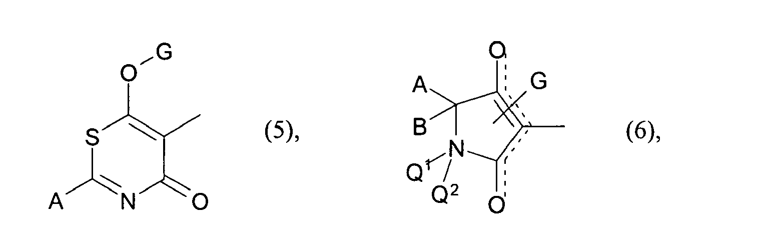 dans lesquels
A est un groupe alkyle en C₁-C₄ ou alcoxy en C₁-C₂-alkyle en C₁-C₂, cyclopropyle, cyclopentyle ou cyclohexyle facultativement substitués chacun par un fluor et, uniquement dans le cas des composés de formule (1-5), un groupe phényle facultativement substitué par un groupe fluoro, chloro, bromo, méthyle, éthyle, n-propyle, isopropyle, méthoxy, éthoxy, trifluorométhyle, trifluorométhoxy, cyano ou nitro,
B est un hydrogène ou un groupe méthyle ou éthyle, ou bien
A, B et l'atome de carbone auquel ils sont rattachés forment un cycloalkyle en C₅-C₆ saturé dont un terme du noyau est facultativement remplacé par un atome d'oxygène ou de soufre et qui est facultativement monosubstitué par un groupe méthyle, éthyle, propyle, isopropyle, trifluorométhyle, méthoxy, éthoxy, propoxy ou butoxy, ou
A, B et l'atome de carbone auquel ils sont rattachés forment un groupe cycloalkyle en C₆ substitué par un groupe alkylènedioxy contenant deux atomes d'oxygène non directement voisins,
A, B et l'atome de carbone auquel ils sont rattachés forment un groupe cycloalkyle en C₅-C₆ ou cycloalcényle en C₅-C₆, deux substituants pris ensemble avec l'atome de carbone auquel ils sont rattachés formant un groupe alcanediyle en C₂-C₄, alcènediyle en C₂-C₄ ou butadiènediyle,
D est un hydrogène, un groupe alkyle en C₁-C₄, alcényle en C₃-C₄, alcoxy en C₁-C₄-alkyle en C₂-C₃, cyclopropyle, cyclopentyle ou cyclohexyle substitués chacun facultativement par un fluor, ou bien (mais pas dans le cas des composés des formules (I-1)) un groupe phényle ou pyridyle facultativement monosubstitué par un groupe fluoro, chloro, méthyle, éthyle, n-propyle, isopropyle, méthoxy, éthoxy ou trifluorométhyle,
A et Q¹ pris ensemble forment un groupe alcanediyle en C₃-C₄ facultativement mono- ou disubstitué par un groupe méthyle ou méthoxy, ou
Q¹ est un hydrogène,
Q² est un hydrogène;
G est un hydrogène (a) ou un des groupes 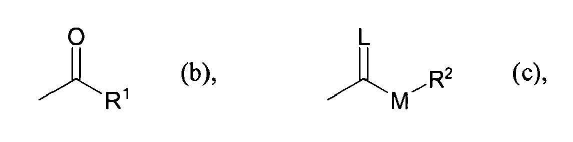 dans lesquels
L est un atome d'oxygène ou de soufre, et
M est un atome d'oxygène ou de soufre,
R¹ est un groupe alkyle en C₁-C₆, alcényle en C₂-C₆, alcoxy en C₁-C₂-alkyle en C₁, alkylthio en C₁-C₂-alkyle en C₁ ou un groupe cyclopropyle ou cyclohexyle facultativement monosubstitué par un groupe fluoro, chloro, méthyle ou méthoxy,
un groupe phényle facultativement monosubstitué par un groupe fluoro, chloro, bromo, cyano, nitro, méthyle, méthoxy, trifluorométhyle ou trifluorométhoxy,
R² est un groupe alkyle en C₁-C₈, alcényle en C₂-C₆ ou alcoxy en C₁-C₄-alkyle en C₂-C₃, phényle ou benzyle facultativement substitués chacun par un fluor.

2. Procédé de préparation de composés de formule (I), **caractérisé en ce que** :
(A) Pour obtenir des 3-phénylpyrrolidine-2,4-diones ou leurs énols de formule (I-1-a) 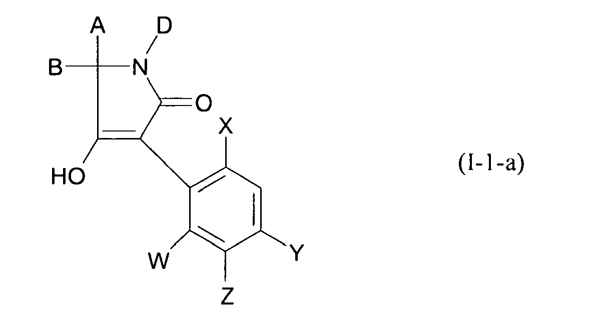 dans laquelle A, B, D, W, X, Y et Z ont les significations indiquées plus haut, on soumet des esters de N-acylaminoacides de formule (II) 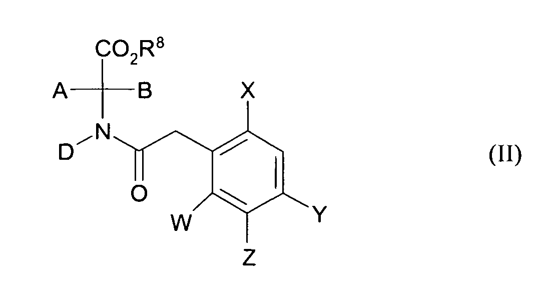 dans laquelle A, B, D, W, X, Y et Z ont les significations indiquées plus haut, et
R⁸ est un groupe alkyle,
à une condensation intramoléculaire en présence d'un diluant et en présence d'une base ;
(B) Pour obtenir des dérivés de 3-phényl-4-hydroxy-Δ³-dihydrofuranone de formule (1-2-a) 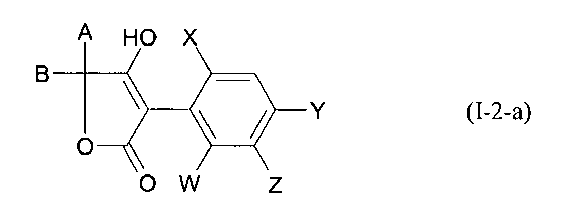 dans laquelle A, B, W, X, Y et Z ont les significations indiquées plus haut, on soumet des esters d'acides carboxyliques de formule (III) 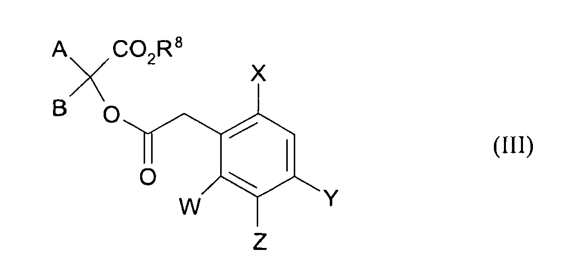 dans laquelle A, B, W, X, Y, Z et R⁸ ont les significations indiquées plus haut, à une condensation intramoléculaire en présence d'un diluant et en présence d'une base ;
(C) Pour obtenir des dérivés de 3-phényl-4-hydroxy-Δ³-dihydrothiophénone de formule (I-3-a) 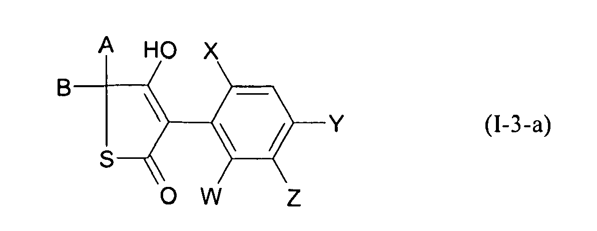 dans laquelle A, B, W, X, Y et Z ont les significations indiquées plus haut, on soumet des esters d'acides β-cétocarboxyliques de formule (IV) 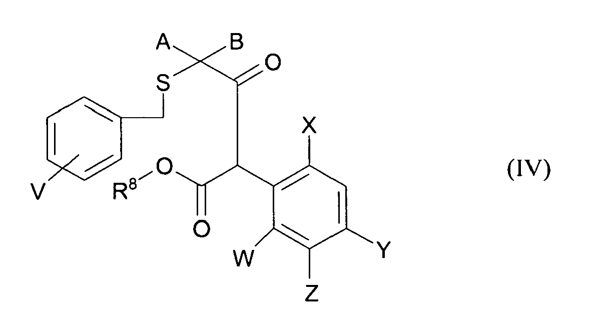 dans laquelle
A, B, W, X, Y, Z et R⁸ ont les significations indiquées plus haut et
V est un hydrogène, un halogène, un alkyle ou un alcoxy,
à une cyclisation intramoléculaire, facultativement en présence d'un diluant et en présence d'un acide ;
(D) Pour obtenir des dérivés de 3-phénylpyrone de formule (I-4-a) 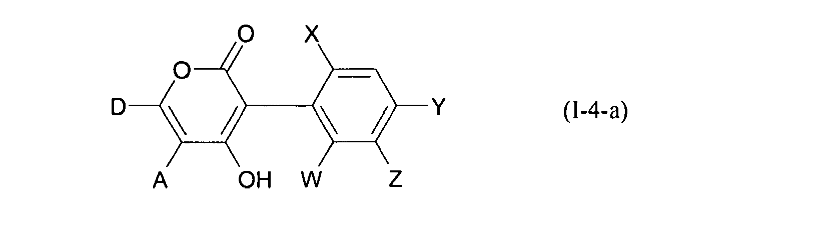 dans laquelle A, D, W, X, Y et Z ont les significations indiquées plus haut, on fait réagir des composés de carbonyle de formule (V) 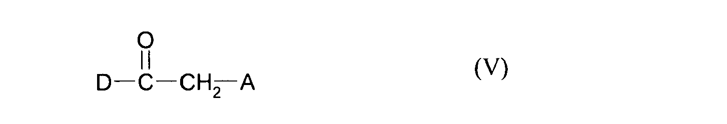 dans laquelle A et D ont les significations indiquées plus haut, ou leurs éthers de silylénol de formule (Va) 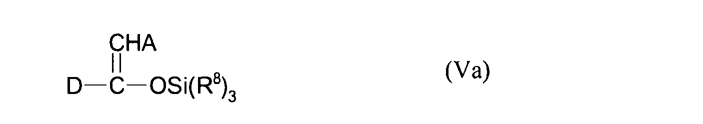 dans laquelle
A, D et R⁸ ont les significations indiquées plus haut, avec des halogénures d'acide céténoïque de formule (VI) 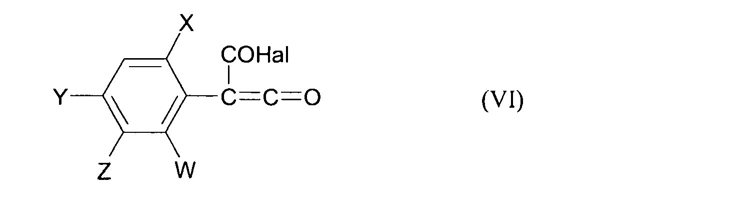
dans laquelle
W, X, Y et Z ont les significations indiquées plus haut et Hal est un halogène, facultativement en présence d'un diluant et facultativement en présence d'un accepteur d'acide ;
(E) Pour obtenir des dérivés de phényl-1,3-thiazine de formule (I-5-a) 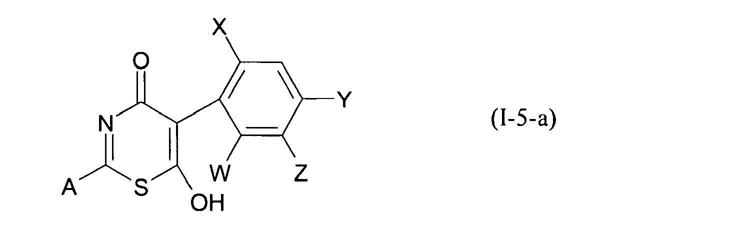 dans laquelle
A, W, X, Y et Z ont les significations indiquées plus haut, on fait réagir des thioamides de formule (VII) 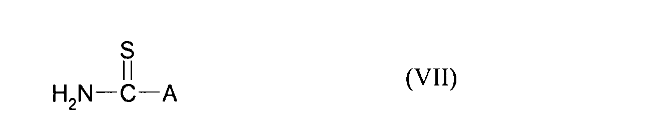
dans laquelle
A a la signification indiquée plus haut, avec des halogénures d'acide céténoïque de formule (VI) 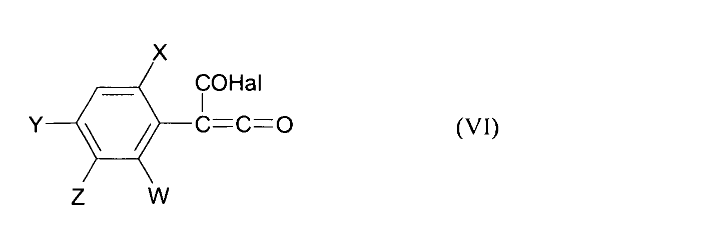
dans laquelle Hal, W, X, Y et Z ont les significations indiquées plus haut, facultativement en présence d'un diluant et facultativement en présence d'un accepteur d'acide ;
(F) Pour obtenir des composés de formule (I-6-a) 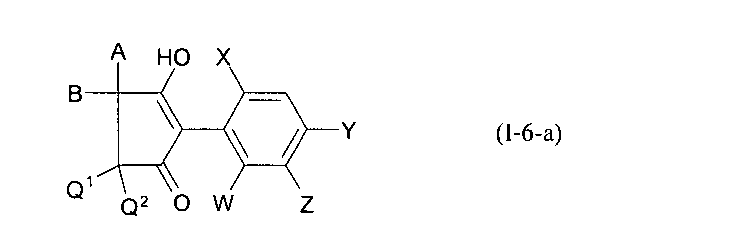 dans laquelle
A, B, Q¹, Q², W, X, Y et Z ont les significations indiquées plus haut, on soumet des esters d'acides cétocarboxyliques de formule (VIII) 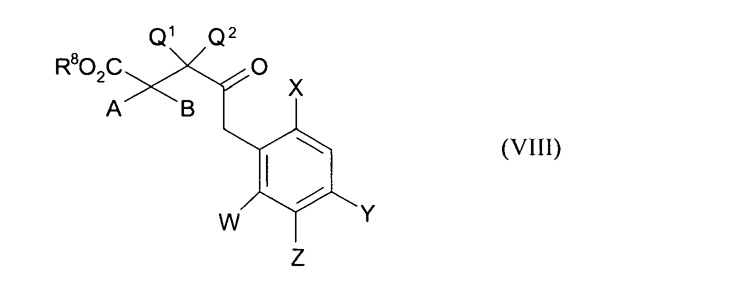
dans laquelle
A, B, Q¹, Q², W, X, Y et Z ont les significations indiquées plus haut et
R⁸ est un groupe alkyle, à une cyclisation intramoléculaire, facultativement en présence d'un diluant et en présence d'une base ;
(H) Pour obtenir des composés des formules (I-1(a-g)) à (I-6(a-g)) ci-dessus, dans lesquelles A, B, D, G, Q¹, Q², Q³, Q⁴, Q⁵, Q⁶, W, X, Y et Z ont les significations indiquées plus haut et un, au maximum deux restes W, X, Y ou Z sont R²²-C≡C- ou 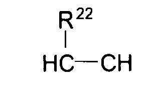 où R²² est un hydrogène ou un alkyle en C₁-C₄, on fait réagir des composés des formules (I-1'(a-g)) à (I-6'(a-g)), 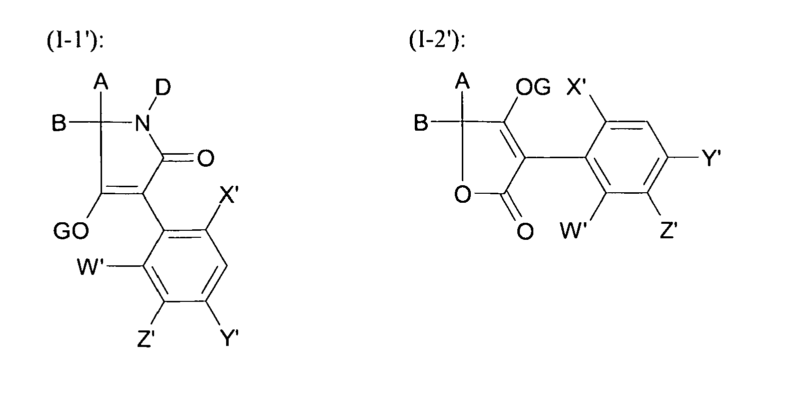 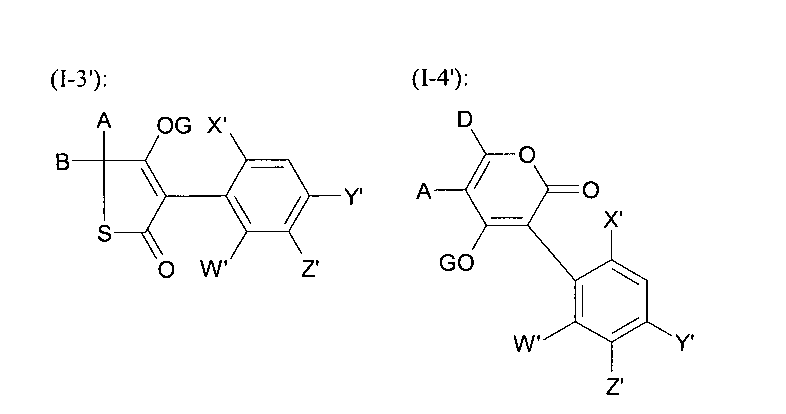 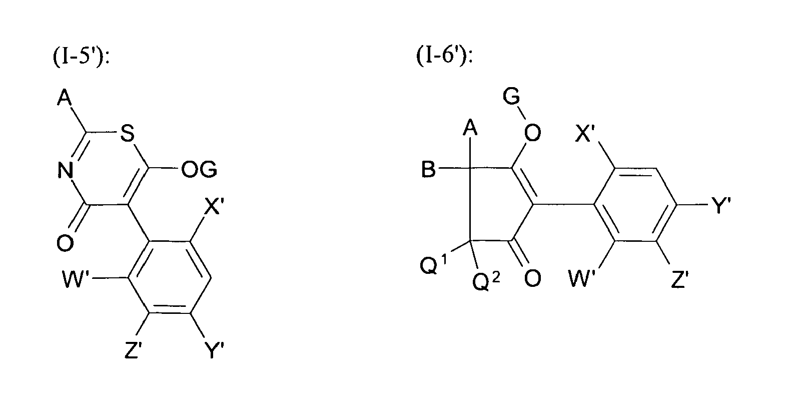 dans lesquelles A, B, D, G, Q¹, Q², W', X', Y' et Z' ont les significations indiquées plus haut et l'apostrophe ' signifie que, dans ce procédé, un, au maximum deux restes W, X, Y et Z représentent un atome de chlore, de brome ou d'iode, à la condition que les autres restes W, X, Y ou Z ne soient pas un groupe alcényle ou alcynyle,
avec des silylacétylènes de formule (X-a) ou des vinylstannanes de formule (X-b) 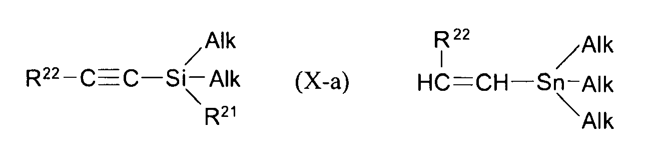 dans lesquelles Alk est un groupe alkyle en C₁-C₄,
R²¹ est un groupe alkyle en C₁-C₄ ou un phényle et
R²² est un hydrogène ou un groupe alkyle en C₁-C₄, en présence d'un solvant, facultativement en présence d'une base et d'un catalyseur ;
(I) Pour obtenir des composés des formules (I-1-b) à (1-6-b) ci-dessus, dans lesquelles A, B, D, Q¹, Q², R¹, W, X, Y et Z ont les significations indiquées plus haut, on fait réagir des composés des formules (I-1-a) à (I-6-a) ci-dessus, dans lesquelles A, B, D, Q¹, Q², W, X, Y et Z ont les significations indiquées plus haut, respectivement
(α) avec des halogénures d'acides de formule (XI) 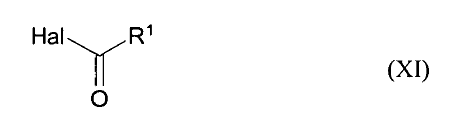
dans laquelle
R¹ a la signification indiquée plus haut et
Hal est un halogène
ou
(β) avec des anhydrides carboxyliques de formule (XII)
R¹-CO-O-CO-R¹ (XII)
dans laquelle
R¹ a la signification indiquée plus haut, facultativement en présence d'un diluant et facultativement en présence d'un liant d'acide ;
(J) Pour obtenir des composés des formules (I-1-c) à (1-6-c) ci-dessus, dans lesquels A, B, D, Q¹, Q², R², M, W, X, Y et Z ont les significations indiquées plus haut et L est un atome d'oxygène, on fait réagir des composés des formules (I-1-a) à (I-6-a) ci-dessus, dans lesquelles A, B, D, Q¹, Q², W, X, Y et Z ont les significations indiquées plus haut, respectivement avec des esters ou des thioesters d'acide chloroformique de formule (XIII)
R²-M-CO-CI (XIII)
dans laquelle
R² et M ont les significations indiquées plus haut, facultativement en présence d'un diluant et facultativement en présence d'un liant d'acide ;
(K) Pour obtenir des composés des formules (I-1-c) à (1-6-c) ci-dessus, dans lesquelles A, B, D, Q¹, Q², R², M, W, X, Y et Z ont les significations indiquées plus haut et L est un atome de soufre, on fait réagir des composés des formules (I-1-a) à (I-6-a) ci-dessus, dans lesquelles A, B, D, Q¹, Q², W, X, Y et Z ont les significations indiquées plus haut, respectivement
avec des esters d'acide chloromonothioformique ou chlorodithioformique de formule (XIV) 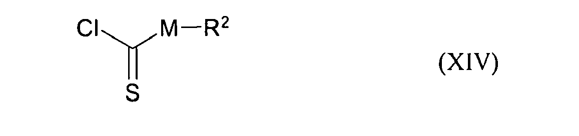 dans laquelle
M et R² ont les significations indiquées plus haut, facultativement en présence d'un diluant et facultativement en présence d'un liant d'acide ;
Et
(L) Pour obtenir des composés des formules (I-1-d) à (1-6-d) ci-dessus, dans lesquelles A, B, D, Q¹, Q², R³, W, X, Y et Z ont les significations indiquées plus haut, on fait réagir des composés des formules (I-1-a) à (I-6-a) ci-dessus, dans lesquelles A, B, D, Q¹, Q², W, X, Y et Z ont les significations indiquées plus haut, respectivement
avec des chlorures d'acide sulfonique de formule (XV)
R³-SO₂-Cl (XV)
dans laquelle
R³ a la signification indiquée plus haut, facultativement en présence d'un diluant et facultativement en présence d'un liant d'acide ;
(M) Pour obtenir des composés des formules (I-1-e) à (1-6-e) ci-dessus, dans lesquelles A, B, D, L, Q¹, Q², R⁴, R⁵, W, X, Y et Z ont les significations indiquées plus haut, on fait réagir des composés des formules (I-1-a) à (I-6-a) ci-dessus, dans lesquelles A, B, D, Q¹, Q², W, X, Y et Z ont les significations indiquées plus haut, respectivement avec des composés de phosphore de formule (XVI) 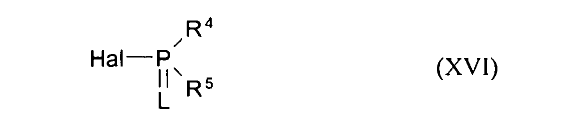 dans laquelle
L, R⁴ et R⁵ ont les significations indiquées plus haut et Hal est un halogène,
facultativement en présence d'un diluant et facultativement en présence d'un liant d'acide ;
(N) Pour obtenir des composés des (I-1-f) à (I-6-f) ci-dessus, dans lesquelles A, B, D, E, Q¹, Q², W, X, Y et Z ont les significations indiquées plus haut, on fait réagir des composés des formules (I-1-a) à (I-6-a), dans lesquelles A B, D, Q¹, Q², W, X, Y et Z ont les significations indiquées plus haut, respectivement avec des composés métalliques ou des amines de formules (XVII) ou (XVIII)
Me(OR¹⁰)ₜ (XVII) 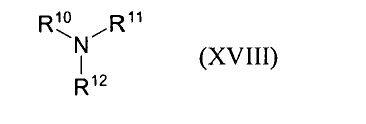
dans lesquelles Me est un métal mono- ou divalent, t est un nombre égal à 1 ou 2, et
R¹⁰, R¹¹, R¹² sont indépendamment les uns des autres un hydrogène ou un alkyle, facultativement en présence d'un diluant ;
(O) Pour obtenir des composés des formules (I-1-g) à (1-6-g) ci-dessus, dans lesquelles A, B, D, L, Q¹, Q², R⁶, R⁷, W, X, Y et Z ont les significations indiquées plus haut, on fait réagir des composés des formules (I-1-a) à (I-6-a) ci-dessus, dans lesquelles A, B, D, Q¹, Q², W, X, Y et Z ont les significations indiquées plus haut, respectivement
(α) avec des isocyanates ou isothiocyanates de formule (XIX) 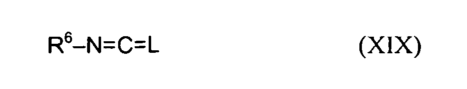 dans laquelle
R⁶ et L ont les significations indiquées plus haut, facultativement en présence d'un diluant et facultativement en présence d'un catalyseur, ou
(β) avec des chlorures d'acide carbamique ou thiocarbamique de formule (XX) 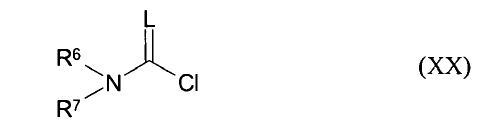 dans laquelle L, R⁶ et R⁷ ont les significations indiquées plus haut, facultativement en présence d'un diluant et facultativement en présence d'un liant d'acide.

3. Composé de formule (I) selon la revendication 1, dans laquelle
W est un hydrogène ou un groupe méthyle, éthyle ou isopropyle,
X est un groupe méthyle, éthyle, isopropyle ou vinyle,
Y est un hydrogène ou un groupe méthyle, éthyle, isopropyle, vinyle ou éthynyle,
Z est un hydrogène ou un groupe méthyle, éthyle, n-propyle ou isobutyle,
à la condition que l'un au moins des restes W, X, Y ou Z soit une chaîne comprenant au moins deux atomes de carbone, au maximum un seul des restes W, X, Y ou Z pouvant être un groupe vinyle ou éthynyle,
CKE est un des groupes 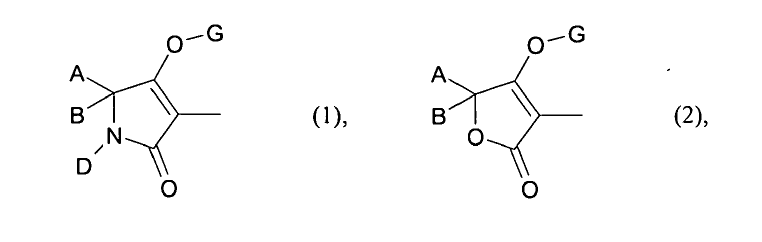 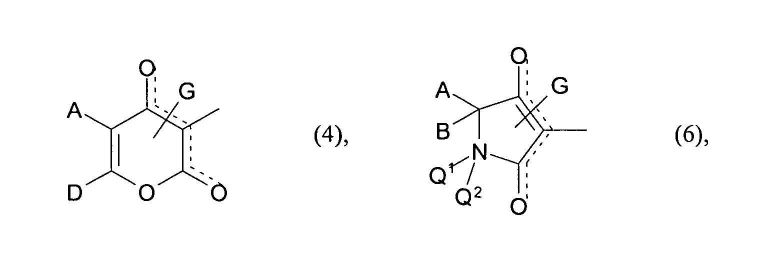 dans lesquels
A est un groupe méthyle,
B est un groupe méthyle,
A, B et l'atome de carbone auquel ils sont rattachés forment un groupe cycloalkyle en C₅-C₆ saturé, dans lequel un terme du noyau est facultativement remplacé par un oxygène et qui est facultativement monosubstitué par un groupe méthyle, éthyle, méthoxy, éthoxy,
A et B pris ensemble sont un groupe 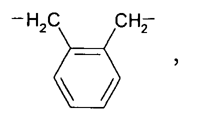
D est un hydrogène ou (mais pas dans le cas du composé de formule (I-1)) un groupe par phényle substitué par un atome de fluor,
G est un hydrogène (a) ou représente un des groupes 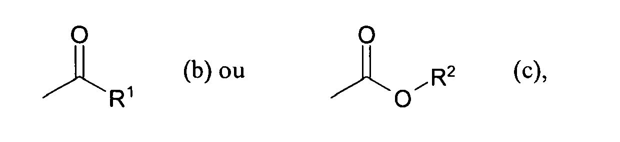
R¹ est un groupe alkyle en C₁-C₆, alcoxyméthyle en C₁-C₂,
R² est un groupe alkyle en C₁-C₄,
et, lorsque CKE = (6),
A et Q¹ pris ensemble forment un alcanediyle en C₃-C₄ et
B et Q² sont chacun un hydrogène.

4. Composés de formule (II) 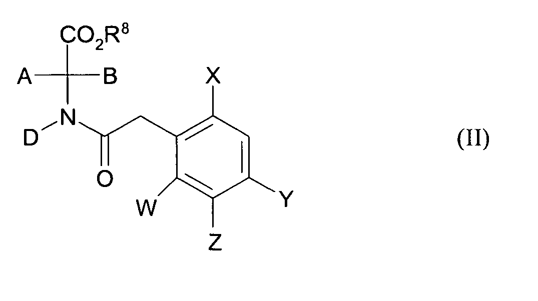 dans laquelle
A, B, D, W, X, Y, Z et R⁸ ont les significations indiquées plus haut.

5. Composés de formule (III) 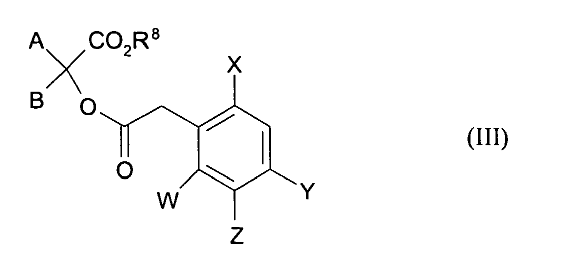 dans laquelle A, B, W, X, Y, Z et R⁸ ont les significations indiquées plus haut.

6. Composés de formule (IV) 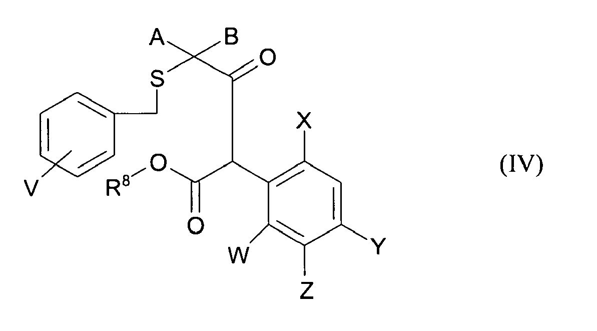 dans laquelle
A, B, W, X, Y, Z et R⁸ ont les significations indiquées plus haut,
V est un hydrogène, un halogène, un alkyle ou un alcoxy.

7. Composés de formule (VI) 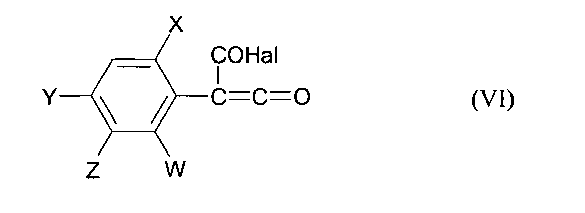 dans laquelle
W, X, Y et Z ont les significations indiquées plus haut et
Hal est un halogène.

8. Composés de formule (VIII) 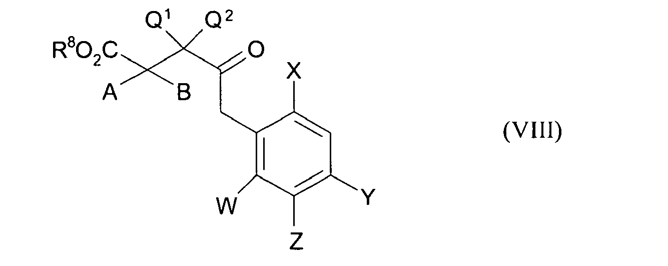 dans laquelle
A, B, Q¹, Q², W, X, Y et Z ont les significations indiquées plus haut et
R⁸ est un groupe alkyle.

9. Composés de formule (XXV) 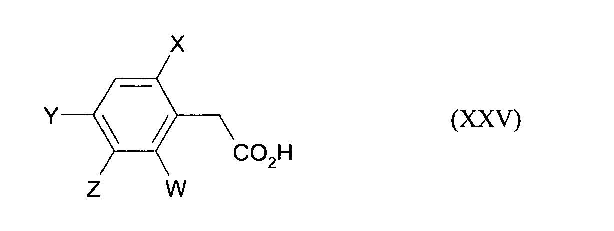 dans laquelle
W, X, Y et Z ont les significations indiquées plus haut et
Y peut en outre être un groupe -C≡C-Si(CH₃)₃.

10. Composés de formule (XXII) 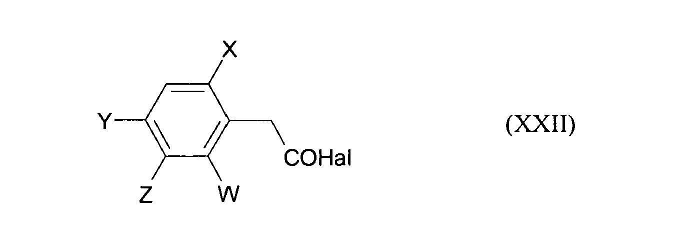 dans laquelle
W, X, Y et Z ont les significations indiquées plus haut et
Hal est un atome de chlore ou de brome.

11. Composés de formule (XXX) 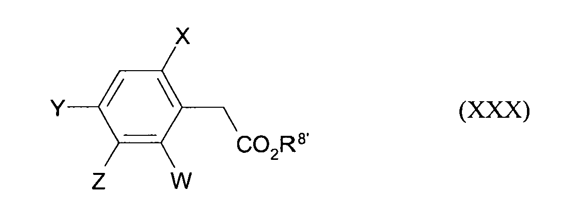 dans laquelle
W, X, Y, Z et R^{8'} ont les significations indiquées plus haut et
Y peut en outre être un groupe-C≡C-Si(CH₃)₃.

12. Composés de formule (XLII) 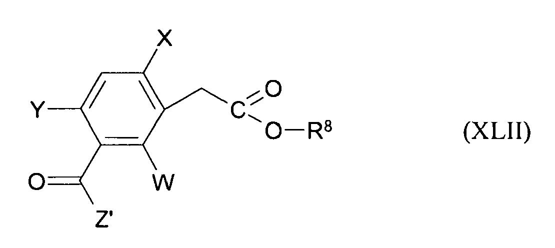 dans laquelle W, Y et R⁸ ont les significations indiquées plus haut et
Z' est un groupe alkyle.

13. Pesticides et herbicides, **caractérisés par** une teneur en au moins un composé de formule (I) selon la revendication 1.

14. Procédé de lutte contre des animaux nuisibles et des végétaux indésirables, **caractérisé en ce que** l'on fait agir des composés de formule (I) selon la revendication 1 sur des nuisibles et/ou sur leur biotope, à l'exception de procédés mis en oeuvre sur l'organisme humain ou animal.

15. Utilisation de composés de formule (I) selon la revendication 1 pour lutter contre des animaux nuisibles et des végétaux indésirables, à l'exception de procédés mis en oeuvre sur l'organisme humain ou animal.

16. Procédé de fabrication de pesticides et herbicides, **caractérisé en ce que** l'on mélange des composés de formule (I) selon la revendication 1 avec des extendeurs et/ou des substances tensio-actives.

17. Utilisation de composés de formule (I) selon la revendication 1 pour fabriquer des pesticides et des herbicides.
